# EUROPEAN PATENT APPLICATION

(11) **EP 4 183 784 A1**
(43) Date of publication of application: **24.05.2023**
(21) Application number: 21861915.3
(22) Date of filing: 04.08.2021
(51) Int. Cl.: C07D 407/12, C07D 307/91, C07D 409/12, C07D 333/76, C07D 263/32, C07D 413/12, C07D 417/12, C07D 277/22, H01L 51/00

(54) **TERTIARY AMINE DERIVATIVE AND ORGANIC ELECTROLUMINESCENT DEVICE COMPRISING SAME**

(30) Priority: 27.08.2020 KR 20200108635
(71) Applicant: Lapto Co., Ltd., Gyeonggi-do 13215 (KR)
(72) Inventor: SEOK, Moon-ki, Seongnam-si, Gyeonggi-do 13215 (KR); GO, Byung-soo, Seongnam-si, Gyeonggi-do 13215 (KR); KIM, Hye-jeong, Seongnam-si, Gyeonggi-do 13215 (KR); KWON, Young-jae, Seongnam-si, Gyeonggi-do 13215 (KR); PARK, Yong-pil, Seongnam-si, Gyeonggi-do 13215 (KR); YOON, Jeoung-hun, Seongnam-si, Gyeonggi-do 13215 (KR); KIM, Gyu-ri, Seongnam-si, Gyeonggi-do 13215 (KR); HAN, Kap-jong, Seongnam-si, Gyeonggi-do 13215 (KR); OH, Eu-gene, Seongnam-si, Gyeonggi-do 13215 (KR)
(74) Representative: Isarpatent
(86) International application number: PCT/KR2021/010195
(87) International publication number: WO 2022/045618

(57) **Abstract**

Provided is a tertiary amine derivative represented by Formula 1. The tertiary amine derivative effectively absorbs light in the UV region from high-energy external light sources to minimize damage to organic materials in organic electroluminescent devices, contributing to a substantial improvement in the lifetime of the organic electroluminescent devices. An organic electroluminescent device of the present invention includes a first electrode, a second electrode, an organic layer arranged between the first and second electrodes, and a capping layer wherein the capping layer includes the tertiary amine derivative represented by Formula 1.

## Description

### Technical Field

The present invention relates to a tertiary amine derivative and an organic electroluminescent device including the tertiary amine derivative, particularly an organic electroluminescent device including a capping layer in which the tertiary amine derivative is used to achieve both high refractive index and excellent UV absorption properties.

### Background Art

With the recent trend toward large-sized displays, there is an increasing demand in the display industry for flat display devices that take up small spaces. Liquid crystal displays (LCDs) have a limited viewing angle and require additional light sources because they are not self-luminous. For these reasons, self-luminous organic light emitting diodes (OLEDs) have attracted attention as promising displays.

In 1963, Pope *et al.* have conducted the first research on carrier injection electroluminescence (EL) utilizing a single crystal of an anthracene aromatic hydrocarbon in the field of OLEDs. Based on this research, basic mechanisms such as charge injection, recombination, exciton formation, and light emission in organic materials, and luminescent properties have been understood and studied.

Particularly, various approaches aimed at increasing the luminescent efficiency of devices have been proposed, for example, in connection with changes in the structure of devices and the development of device materials (Sun, S., Forrest, S. R., Appl. Phys. Lett. 91, 263503 (2007)/Ken-Tsung Wong, Org. Lett., 7, 2005, 5361-5364).

A typical OLED display basically has a multilayer structure consisting of an anode, a hole injection layer (HIL), a hole transporting layer (HTL), an emission layer (EML), an electron transporting layer (ETL), and a cathode. That is, the OLED display has a structure in which an organic multilayer film is sandwiched between both electrodes.

In general, organic luminescence refers to a phenomenon in which organic materials are used to convert electrical energy into light energy. An organic light emitting device using organic luminescence usually has a structure including an anode, a cathode, and an organic layer interposed therebetween. The organic layer often consists of one or more layers composed of different materials to increase the efficiency and stability of the organic light emitting device. For example, the organic layer may include a hole injection layer, a hole transporting layer, an emission layer, an electron transporting layer, and an electron injection layer.

When a voltage is applied between the two electrodes of the organic light emitting device, holes and electrons are injected into the organic layer from the anode and the cathode, respectively. The holes recombine with the electrons in the organic layer to form excitons. The excitons fall to the ground state to emit light. Such organic light emitting devices are known to have many advantages, including self-luminescence, high luminance, high efficiency, low driving voltage, wide viewing angle, high contrast, and fast response.

Materials for organic layers of organic light emitting devices can be classified into light emitting materials and charge transporting materials, for example, hole injection materials, hole transporting materials, electron transporting materials, and electron injection materials, by their functions.

Light emitting materials include blue, green, and red light emitting materials that emit different colors of light and yellow and orange light emitting materials that are necessary to obtain more natural colors. Other light emitting materials are host/dopant systems that can be used to achieve high color purity and luminescent efficiency through energy transfer. The principle is that when a small amount of a dopant, whose energy band gap is smaller and whose luminescent efficiency is higher than those of a host as a main material for an emission layer, is introduced into the emission layer, excitons generated from the host are transported to the dopant to emit light with high efficiency. At this time, since the wavelength of the host is shifted to the wavelength band of the dopant, light of a desired wavelength can be obtained depending on the type of the dopant.

Materials for organic layers of organic light emitting devices, for example, hole injection materials, hole transporting materials, light emitting materials, electron transporting materials, and electron injection materials, have been developed to achieve excellent characteristics of the devices. Organic light emitting devices using commercialized materials for organic layers have been recognized for their performance.

However, since the commercialization of organic light emitting devices, characteristics other than the luminescent properties of organic light emitting devices have been taken into consideration.

Most organic light emitting devices are exposed to external light sources for a long time and thus remain exposed to high-energy UV light, which continuously affects organic materials constituting the organic light emitting devices. This problem can be solved by applying UV-absorbing capping layers to organic light emitting devices to prevent their exposure to high-energy light sources.

A typical organic light emitting device is known to have a wide viewing angle but undergoes a large variation in terms of light source's spectrum depending on the viewing angle. This is due to a deviation between the total refractive index of constituent elements and materials (e.g., including a glass substrate, organic materials, and electrode materials) of the organic light emitting device and an optimal refractive index depending on the emission wavelength of the organic light emitting device.

Generally, a high refractive index is required for blue light emission and a lower refractive index is required for a longer wavelength. There is thus a need to develop a material for a capping layer that simultaneously meets the aforementioned requirements in terms of UV absorption characteristics and optimal refractive index.

The efficiency of an organic light emitting device can be generally divided into internal luminescent efficiency and external luminescent efficiency. The internal luminescent efficiency is related to the formation efficiency of excitons in the organic layer for photoconversion.

The external luminescent efficiency refers to the emission efficiency of light from the organic layer outside the organic light emitting device.

Not only high internal luminescent efficiency but also high external luminescent efficiency is required to raise the overall efficiency. Thus, there is a need to develop a compound for a capping layer (CPL) that has new functions to achieve high external luminescent efficiency and prevent many problems caused by long-term exposure to daylight. Particularly, there is a need to develop a material for a capping layer (CPL) that has an outstanding ability to absorb light in a UV wavelength band.

Unlike a bottom-emitting device having a non-resonant structure, a top-emitting device having a resonant structure is constructed such that the produced light is reflected from the anode as a reflective film and is directed toward the cathode, resulting in a significant loss of optical energy due to surface plasmon polaritons (SPPs).

Accordingly, one important approach to improve the shape and efficiency of the EL spectrum is to form a capping layer on the top cathode.

As for SPPs, four metals, including Al, Pt, Ag, and Au, are mainly used for electron emission and surface plasmons are generated on the surfaces of the metal electrodes. For example, when Ag is used for the cathode, emitted light is quenched by SPPs (light energy is lost by Ag), resulting in efficiency reduction.

In contrast, the use of a capping layer causes the generation of SPPs at the interface between a MgAg electrode and an organic material. When the organic material has a high refractive index (for example, n > 1.69 at 620 nm), transverse electric (TE) polarized light is dissipated on the surface of the capping layer in the vertical direction by evanescent waves, the wavelength of transverse magnetic (TM) polarized light traveling along the cathode and the capping layer is amplified by surface plasmon resonance, and as a result, the intensity of the peak increases, achieving high efficiency and enabling effective control over color purity.

However, there remains a need to develop materials and structures of organic light emitting devices that are necessary to improve various characteristics of the devices in a balanced manner while achieving improved efficiency and color purity of the devices.

### Detailed Description of the Invention

### Problems to be Solved by the Invention

One object of the present invention is to provide a material for a capping layer of an organic light emitting device that can be used to improve the luminescent efficiency and lifetime of the device while achieving improved viewing angle characteristics.

A further object of the present invention is to provide a high-efficiency, long-lasting organic electroluminescent device including a capping layer with high refractive index and good heat resistance, particularly to achieve improved light extraction efficiency.

### Means for Solving the Problems

The present invention provides an organic electroluminescent device including a first electrode, an organic layer arranged on the first electrode, a second electrode arranged on the organic layer, and a capping layer arranged on the second electrode wherein the organic layer or the capping layer includes a tertiary amine derivative represented by Formula 1: 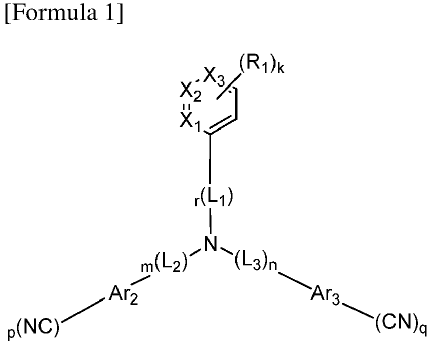 wherein X₁, X₂, and X₃ are each independently CH or N, n, m, and r are each independently 0 to 2, p and q are each independently 0 to 5, k is 0 to 2, Ri is selected from hydrogen, deuterium, fluoro, cyano, methyl, t-butyl, trimethylsilyl, and trifluoromethyl, L₁, L₂, and L₃ are each independently selected from phenylene, pyridylene, naphthalenyl, dibenzofuran, and dibenzothiophene groups, and Ar₂ and Ar₃ are the same as or different from each other and are each independently selected from substituted or unsubstituted phenyl, substituted or unsubstituted naphthyl, substituted or unsubstituted pyridine, substituted or unsubstituted dibenzofuran, substituted or unsubstituted dibenzothiophene, substituted or unsubstituted benzoxazole, substituted or unsubstituted benzothiazole groups, and substituted or unsubstituted benzimidazole groups.

### Effects of the Invention

The compound of the present invention can be used as a material for an organic layer or a capping layer of an organic light emitting device.

Due to its excellent UV absorption properties, the compound of the present invention can be used to minimize damage to organic materials in an organic light emitting device caused by an external light source, improve the efficiency of the device, lower the driving voltage of the device, and/or increase the life characteristics of the device.

The use of the compound according to the present invention as a material for a capping layer of an organic light emitting device can lead to an improvement in the luminous efficiency of the device and a reduction in the full width at half maximum of the emission spectrum, resulting in a significant improvement in color purity.

The compound of the present invention is featured in that one or more cyano groups are introduced to achieve a high refractive index. Due to this feature, the compound of the present invention can be used as a material for a capping layer to improve the viewing angle and optical efficiency of light extracted into the air.

### Brief Description of the Drawings

Fig. 1 shows an exemplary structure of an organic light emitting device according to one embodiment of the present invention in which a first electrode 110, a hole injection layer 210, a hole transporting layer 215, an emission layer 220, an electron transporting layer 230, an electron injection layer 235, a second electrode 120, and a capping layer 300 are stacked in this order on a substrate 100.
Fig. 2 shows the refraction and absorption properties of light when tertiary amine derivatives according to exemplary embodiments of the present invention were used.

### Best Mode for Carrying out the Invention

As the present invention allows for various changes and numerous embodiments, particular embodiments will be illustrated in drawings and described in detail in the written description. However, this is not intended to limit the present invention to particular modes of practice, and it is to be appreciated that all changes, equivalents, and substitutes that do not depart from the spirit and technical scope of the present invention are encompassed in the present invention.

As used herein, the term "substituted" in the definition of "substituted or unsubstituted" refers to substitution with at least one substituent selected from the group consisting of deuterium and halogen atoms and cyano, nitro, amino, hydroxyl, silyl, boron, phosphine oxide, phosphine sulfide, alkyl, haloalkyl, alkoxy, alkenyl, aryl, heteroaryl, and heterocyclic groups. The term "unsubstituted" in the same definition indicates having no substituent. Each of the substituents exemplified above may be optionally further substituted. For example, a biphenyl group may be interpreted as an aryl group or a phenyl group substituted with a phenyl group.

Examples of the halogen atoms include fluorine, chlorine, bromine, and iodine atoms.

The alkyl groups may be linear, branched or cyclic. The number of carbon atoms in each of the alkyl groups is 1 to 50, preferably 1 to 6. Examples of the alkyl groups include, but are not limited to, methyl, ethyl, n-propyl, isopropyl, n-butyl, s-butyl, t-butyl, i-butyl, 2-ethylbutyl, 3,3-dimethylbutyl, n-pentyl, i-pentyl, neopentyl, t-pentyl, cyclopentyl, 1-methylpentyl, 3-methylpentyl, 2-ethylpentyl, 4-methyl-2-pentyl, n-hexyl, 1-methylhexyl, 2-ethylhexyl, 2-butylhexyl, cyclohexyl, 4-methylcyclohexyl, 4-t-butylcyclohexyl, n-heptyl, 1-methylheptyl, 2,2-dimethylheptyl, 2-ethylheptyl, 2-butylheptyl, n-octyl, t-octyl, 2-ethyloctyl, 2-butyloctyl, 2-hexyloctyl, 3,7-dimethyloctyl, cyclooctyl, n-nonyl, n-decyl, adamantyl, 2-ethyldecyl, 2-butyldecyl, 2-hexyldecyl, 2-octyldecyl, n-undecyl, n-dodecyl, 2-ethyldodecyl, 2-butyldodecyl, 2-hexyldodecyl, 2-octyldodecyl, n-tridecyl, n-tetradecyl, n-pentadecyl, n-hexadecyl, 2-ethylhexadecyl, 2-butylhexadecyl, 2-hexylhexadecyl, 2-octylhexadecyl, n-heptadecyl, n-octadecyl, n-nonadecyl, n-eicosyl, 2-ethyleicosyl, 2-butyleicosyl, 2-hexyleicosyl, 2-octyleicosyl, n-henicosyl, n-docosyl, n-tricosyl, n-tetracosyl, n-pentacosyl, n-hexacosyl, n-heptacosyl, n-octacosyl, n-nonacosyl, and n-triacontyl groups.

As used herein, the term "cyclic hydrocarbon group" refers to any functional group or substituent derived from an alicyclic hydrocarbon. The cyclic hydrocarbon group may be a saturated cyclic hydrocarbon group having 5 to 20 ring carbon atoms.

As used herein, the term "aryl group" refers to any functional group or substituent derived from an aromatic cyclic hydrocarbon ring. The aryl group may be monocyclic or polycyclic. The number of ring carbon atoms in the aryl group may be 6 to 30, preferably 6 to 15. Examples of such aryl groups include, but are not limited to, phenyl, naphthyl, fluorenyl, anthracenyl, phenanthryl, biphenyl, terphenyl, quaterphenyl, quinquephenyl, sexiphenyl, triphenylenyl, pyrenyl, perylenyl, naphthacenyl, benzofluoranthenyl, and chrysenyl groups.

The fluorenyl group may be substituted. In this case, two substituents may be bonded to each other to form a spiro structure.

The heteroaryl group may be interrupted by one or more heteroatoms selected from O, N, P, Si, and S. The N and S atoms may be optionally oxidized and the N atom(s) may be optionally quaternized. The number of ring carbon atoms in the heteroaryl group is 2 to 30 or 2 to 20. The heteroaryl group may be monocyclic or polycyclic. For example, the polycyclic heteroaryl group may have a bicyclic or tricyclic structure.

Examples of such heteroaryl groups include, but are not limited to, thiophenyl, furanyl, pyrrolyl, imidazolyl, pyrazolyl, thiazolyl, oxazolyl, oxadiazolyl, triazolyl, pyridinyl, bipyridinyl, pyrimidinyl, triazinyl, tetrazinyl, triazolyl, tetrazolyl, acridyl, pyridazinyl, pyrazinyl, quinolinyl, quinazolinyl, quinoxalinyl, phenoxazinyl, phthalazinyl, pyridopyrimidinyl, pyridopyrazinopyrazinyl, isoquinolinyl, cinnolinyl, indolyl, isoindolyl, indazolyl, carbazolyl, N-arylcarbazolyl, N-heteroarylcarbazolyl, N-alkylcarbazolyl, benzoxazolyl, benzoimidazolyl, benzothiazolyl, benzocarbazolyl, benzothiophenyl, benzoisothiazolyl, benzoisoxazolyl, dibenzothiophenyl, benzofuranyl, phenanthrolinyl, phenanthridinyl, thiazolyl, isoxazolyl, oxadiazolyl, thiadiazolyl, isothiazolyl, phenothiazinyl, benzodioxolyl, dibenzosilolyl, dibenzofuranyl, and isobenzofuranyl groups. An N-oxide aryl group may correspond to the monocyclic or polycyclic heteroaryl group. The N-oxide aryl group may be, for example, a quaternary salt such as a pyridyl N-oxide or quinolyl N-oxide group but is not limited thereto.

As used herein, the term "silyl group" is intended to include alkylsilyl and arylsilyl groups. Examples of such silyl groups include, but are not limited to, trimethylsilyl, triethylsilyl, t-butyldimethylsilyl, vinyldimethylsilyl, propyldimethylsilyl, triphenylsilyl, diphenylsilyl, and phenylsilyl groups.

As used herein, the term "boron group" is intended to include alkyl boron and aryl boron groups. Examples of such boron groups include, but are not limited to, trimethyl boron, triethyl boron, t-butyl dimethyl boron, triphenyl boron, diphenyl boron, and phenyl boron groups.

The alkenyl groups may be linear or branched. The number of carbon atoms in each of the alkeyl groups is 2 to 30, preferably 2 to 10, but is not particularly limited thereto. Examples of the alkenyl groups include, but are not limited to, vinyl, 1-butenyl, 1-pentenyl, 1,3-butadienyl, allyl, styrenyl, and styrylvinyl groups.

As used herein, the term "adjacent group" may mean a substituent on an atom directly attached to an atom substituted with the corresponding substituent, another substituent on an atom substituted with the corresponding substituent or a substituent disposed sterically closest to the corresponding substituent. For example, the two methyl groups in 1,2-dimethylbenzene can be interpreted as "adjacent groups" and the two ethyl groups in 1,1-diethylcyclopentene can be interpreted as "adjacent groups".

The present invention provides a tertiary amine derivative that is used to form an organic layer and/or a capping layer.

According to one embodiment of the present invention, the tertiary amine derivative is represented by Formula 1: 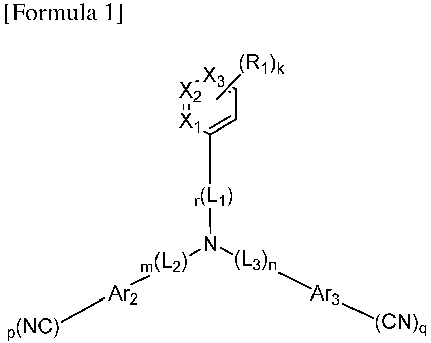 wherein X₁, X₂, and X₃ are each independently CH or N, n, m, and r are each independently 0 to 2, p and q are each independently 0 to 5, k is 0 to 2, Ri is selected from hydrogen, deuterium, fluoro, cyano, methyl, t-butyl, trimethylsilyl, and trifluoromethyl, L₁, L₂, and L₃ are each independently selected from phenylene, pyridylene, naphthalenyl, dibenzofuran, and dibenzothiophene groups, and Ar₂ and Ar₃ are the same as or different from each other and are each independently selected from substituted or unsubstituted phenyl, substituted or unsubstituted naphthyl, substituted or unsubstituted pyridine, substituted or unsubstituted dibenzofuran, substituted or unsubstituted dibenzothiophene, substituted or unsubstituted benzoxazole, substituted or unsubstituted benzothiazole groups, and substituted or unsubstituted benzimidazole groups.

According to one embodiment of the present invention, the tertiary amine derivative represented by Formula 1 may be selected from the compounds represented by Formulae 2:

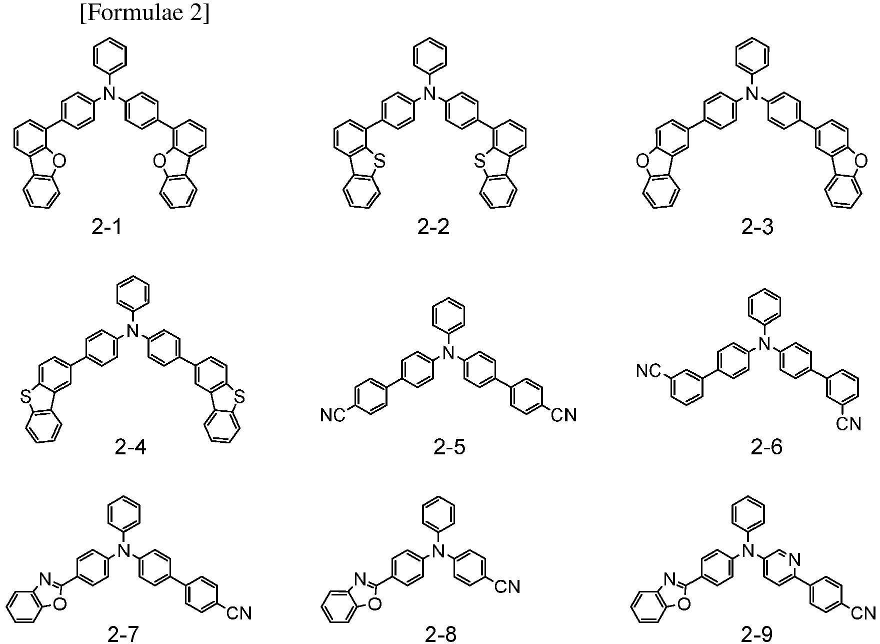

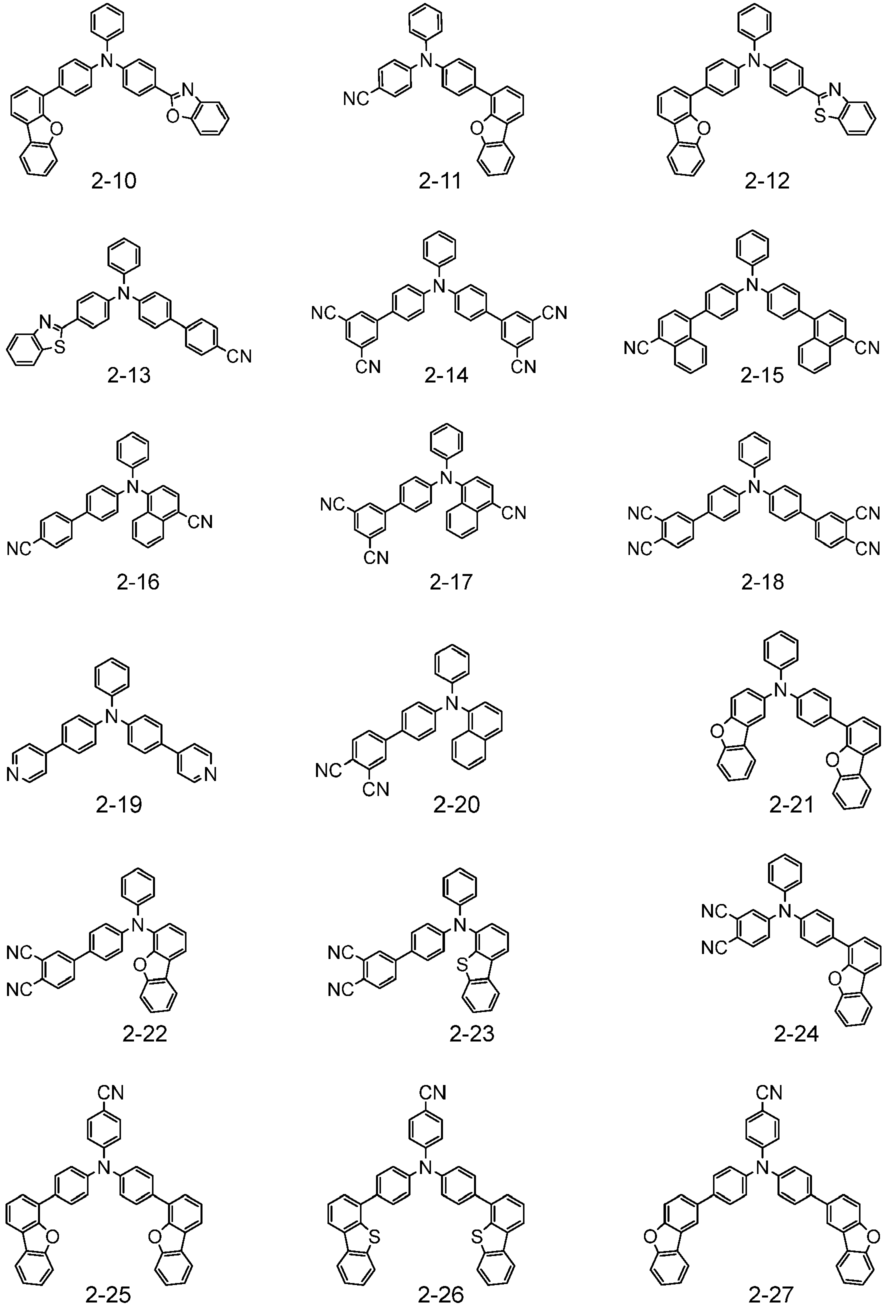

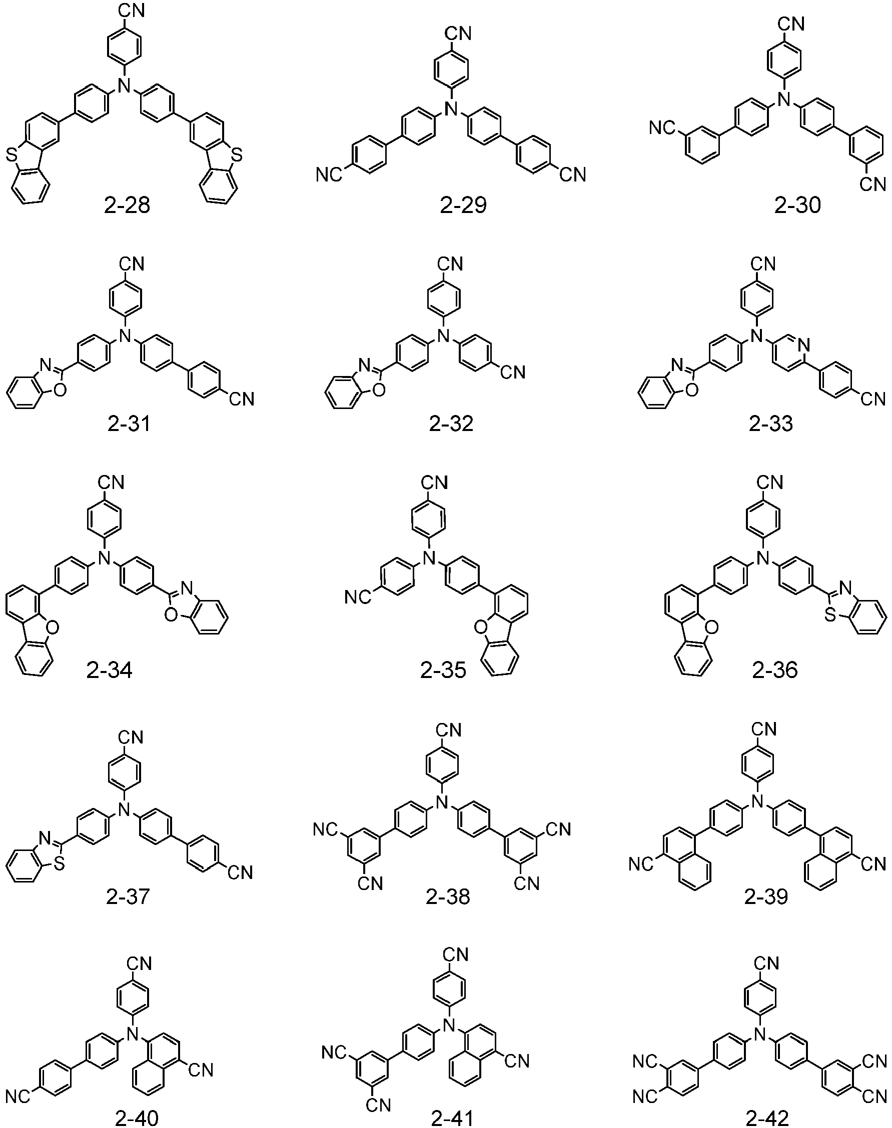

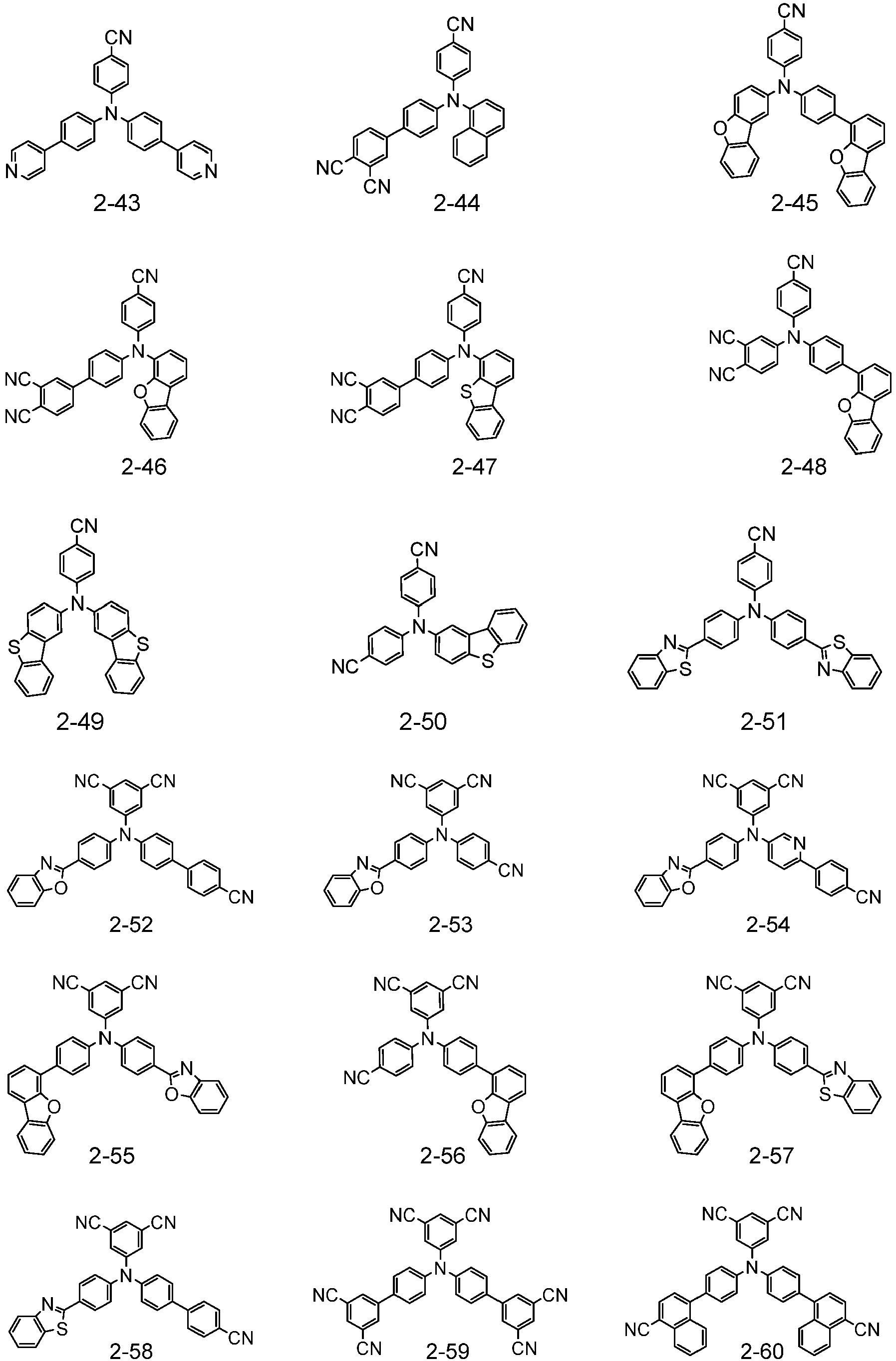

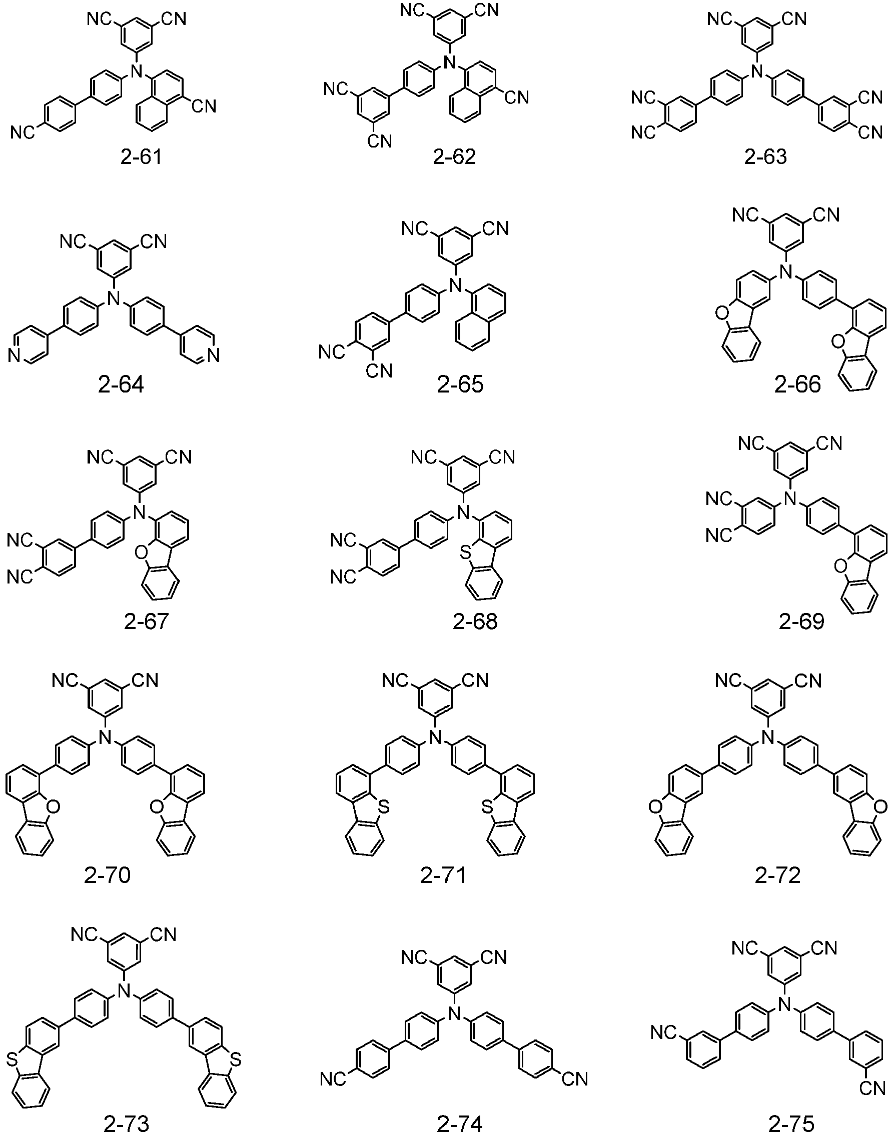

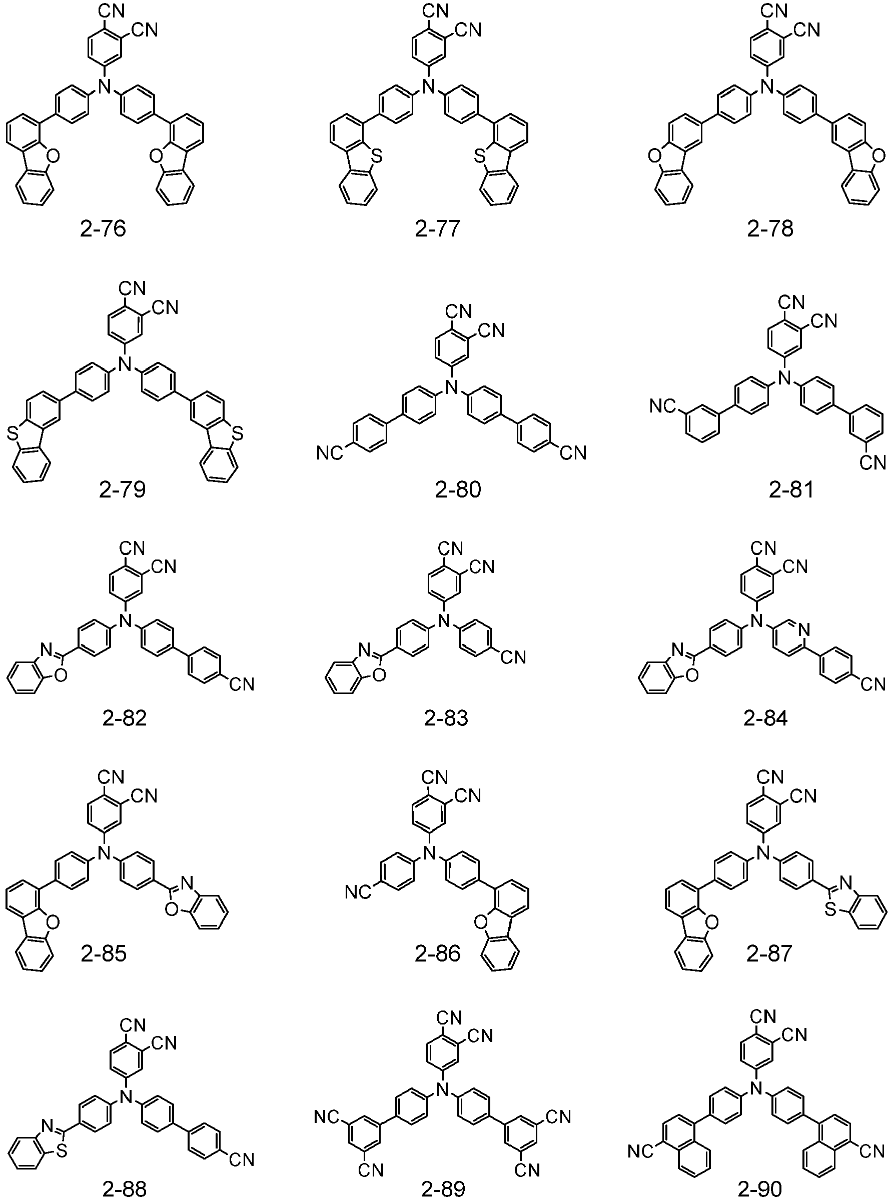

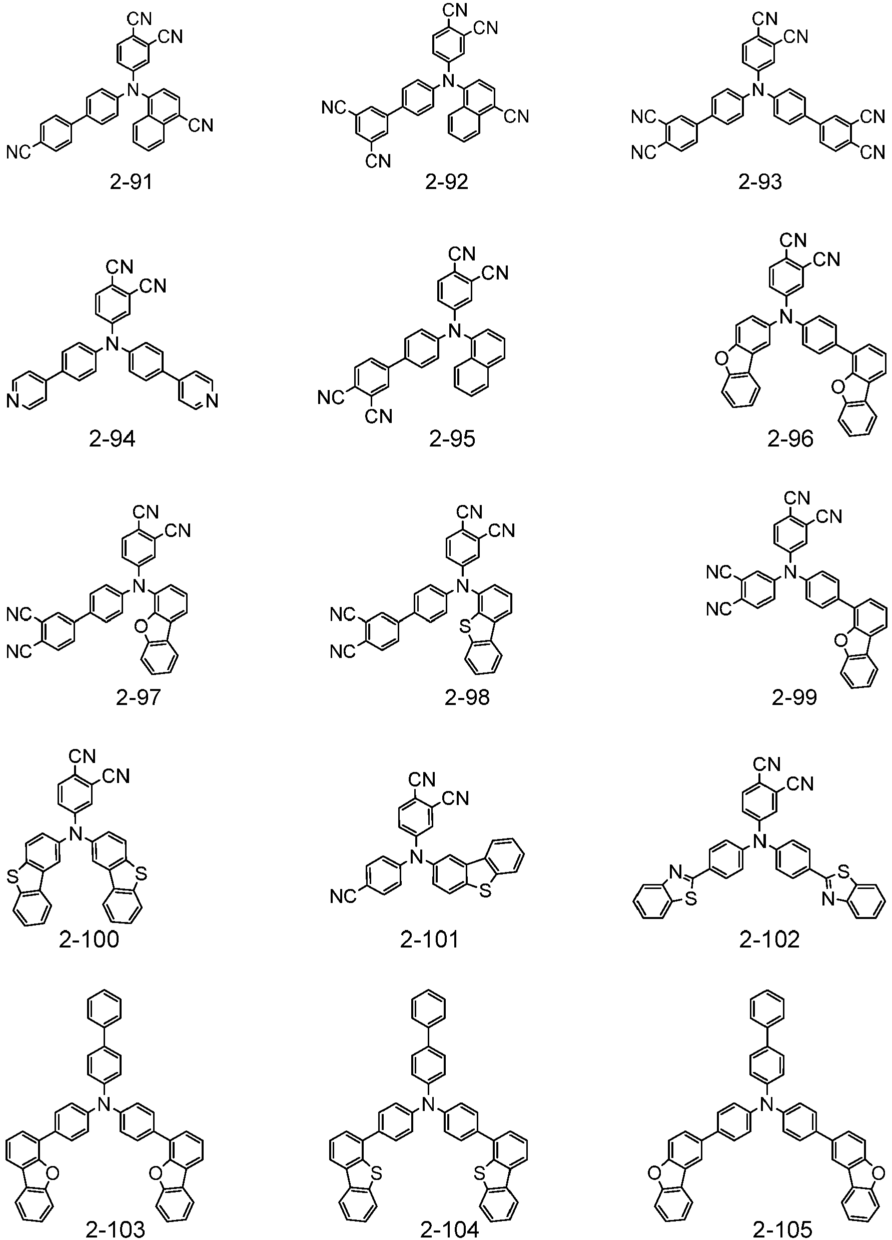

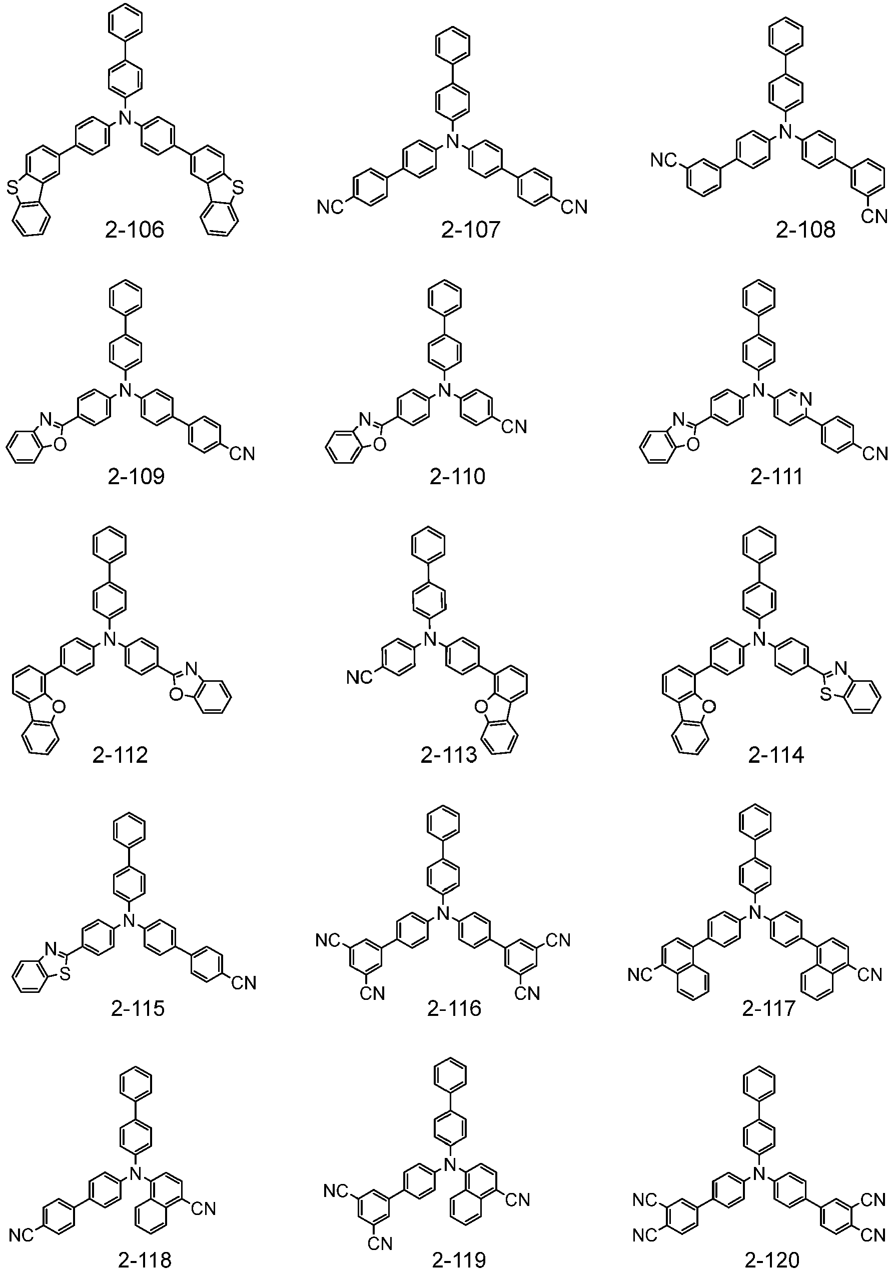

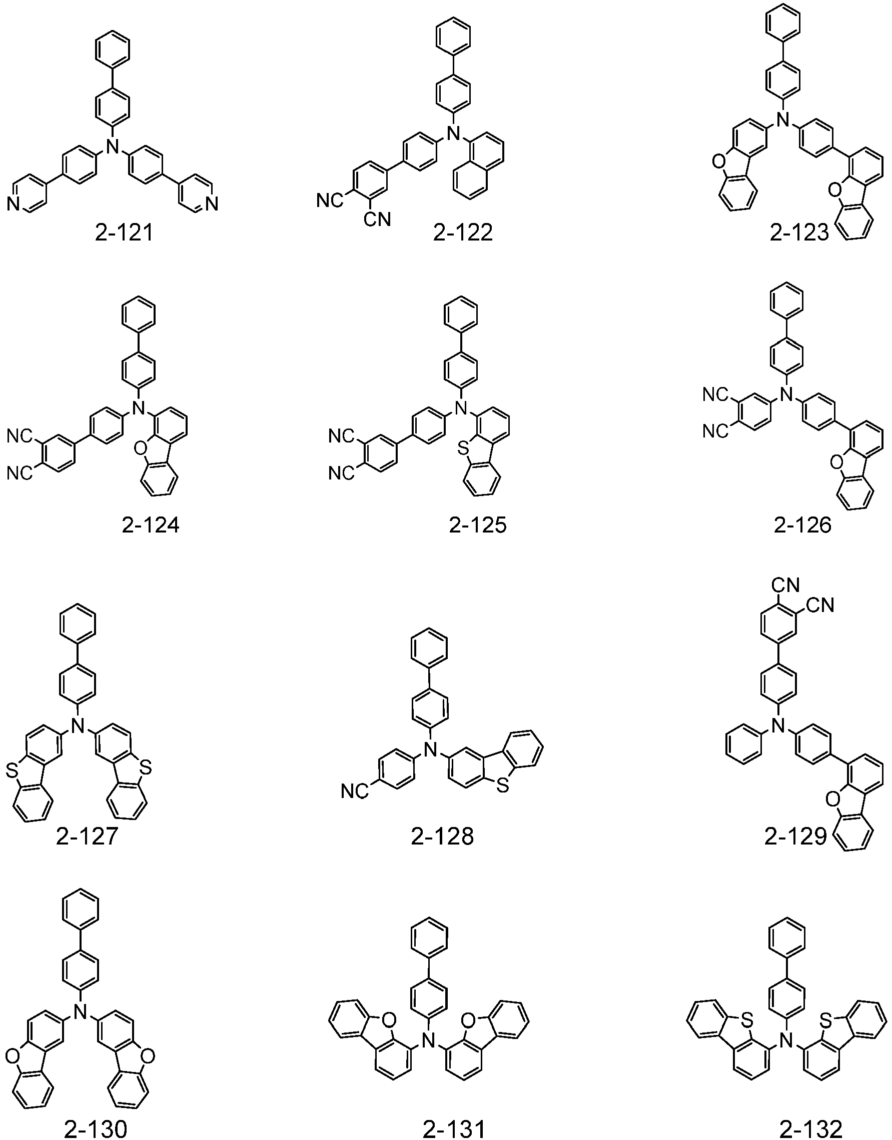

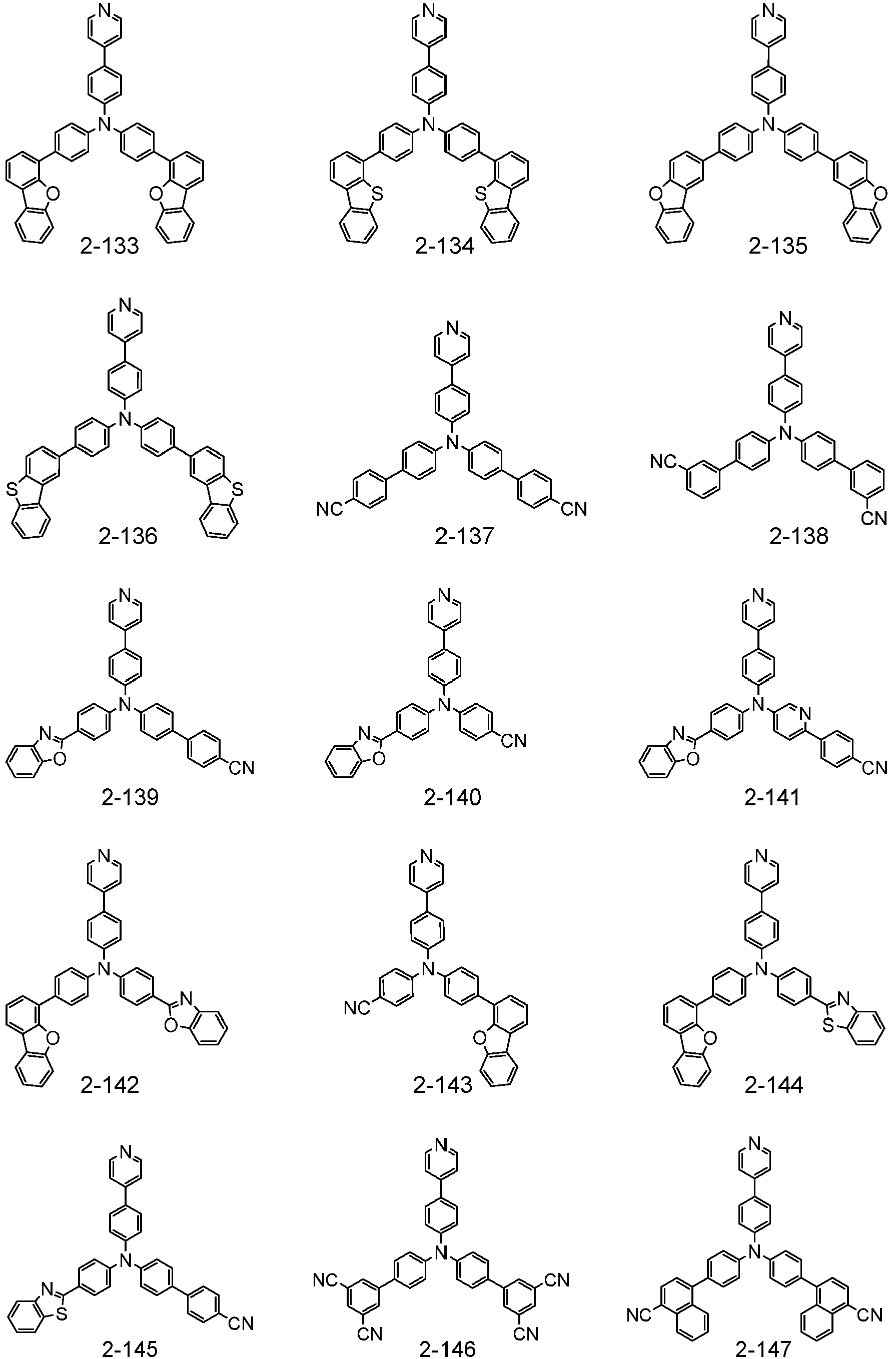

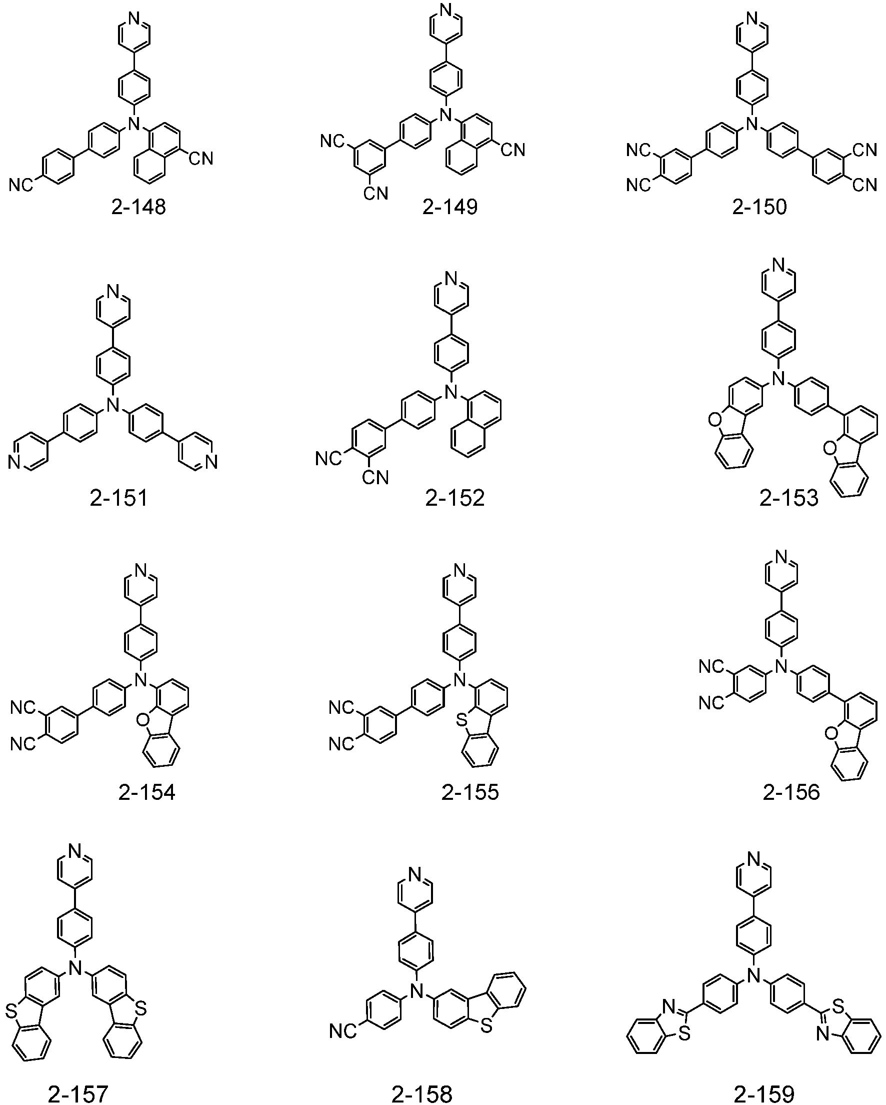

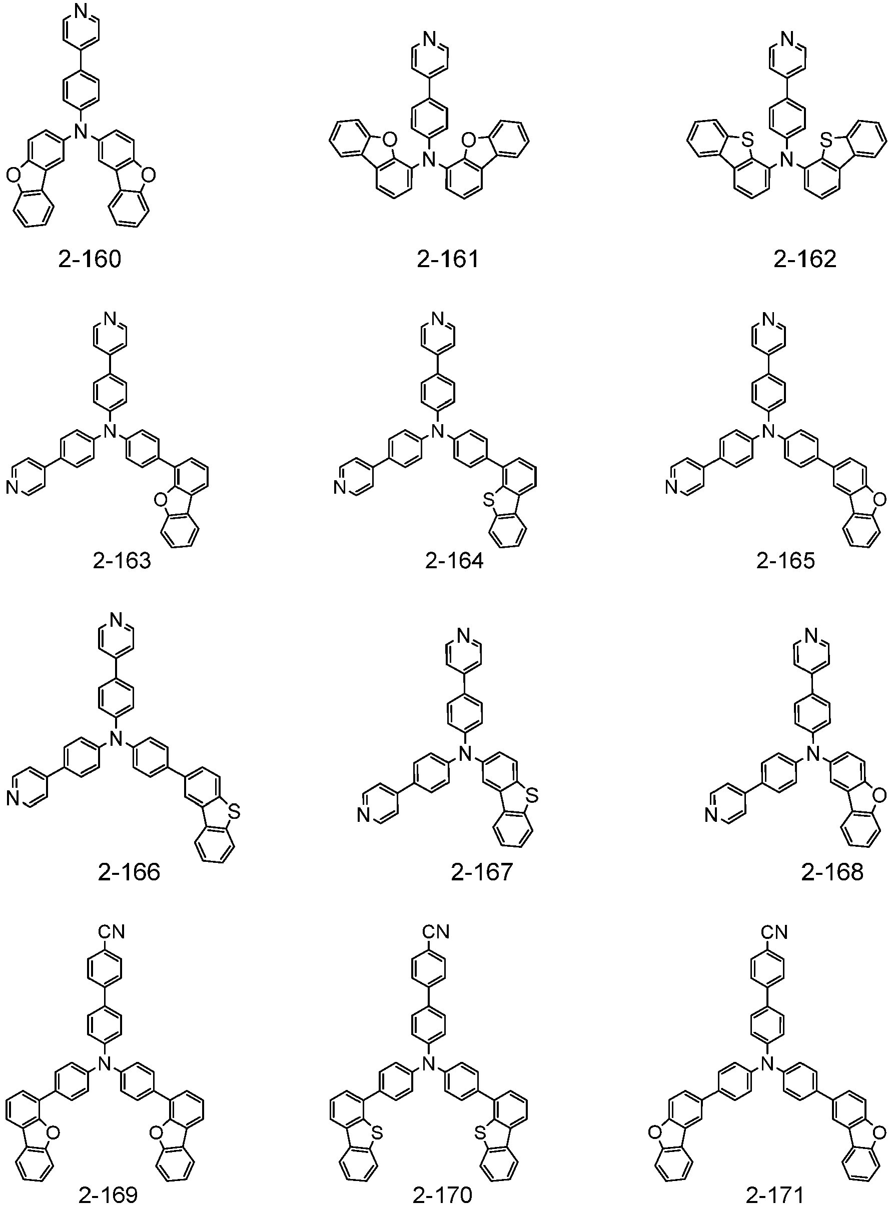

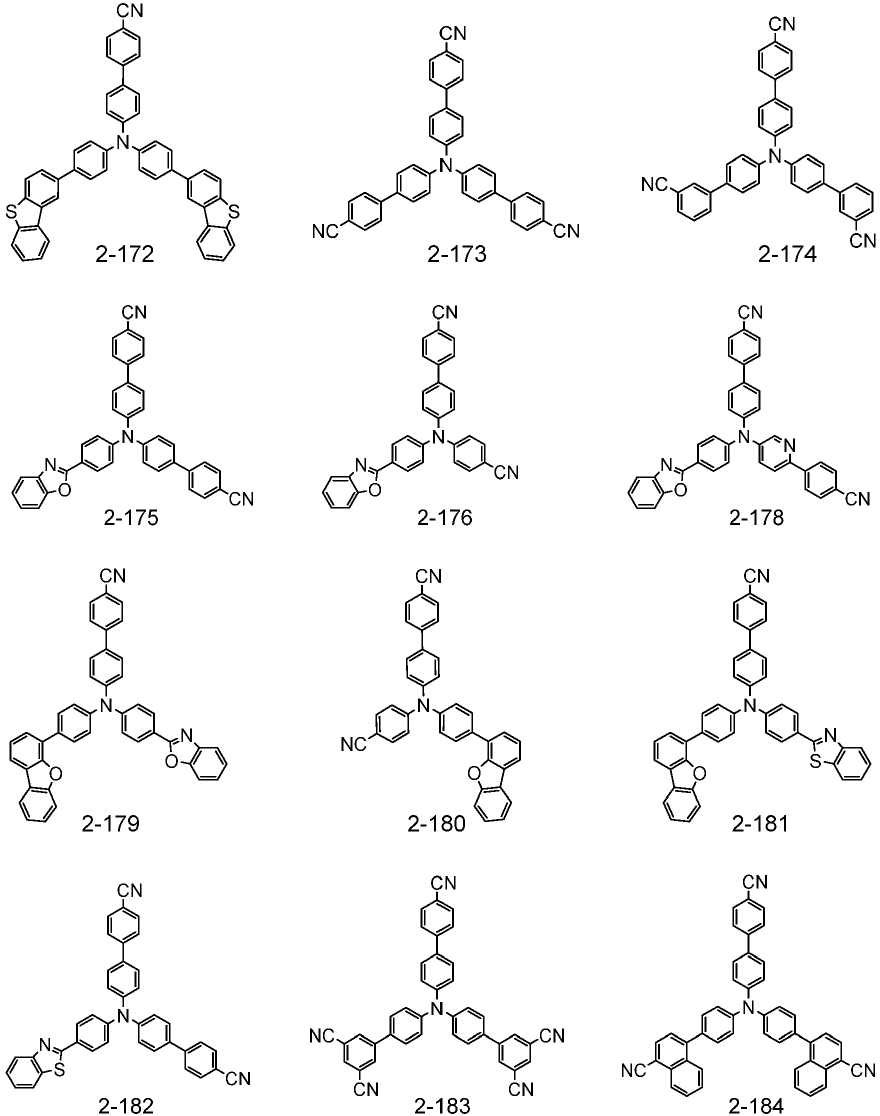

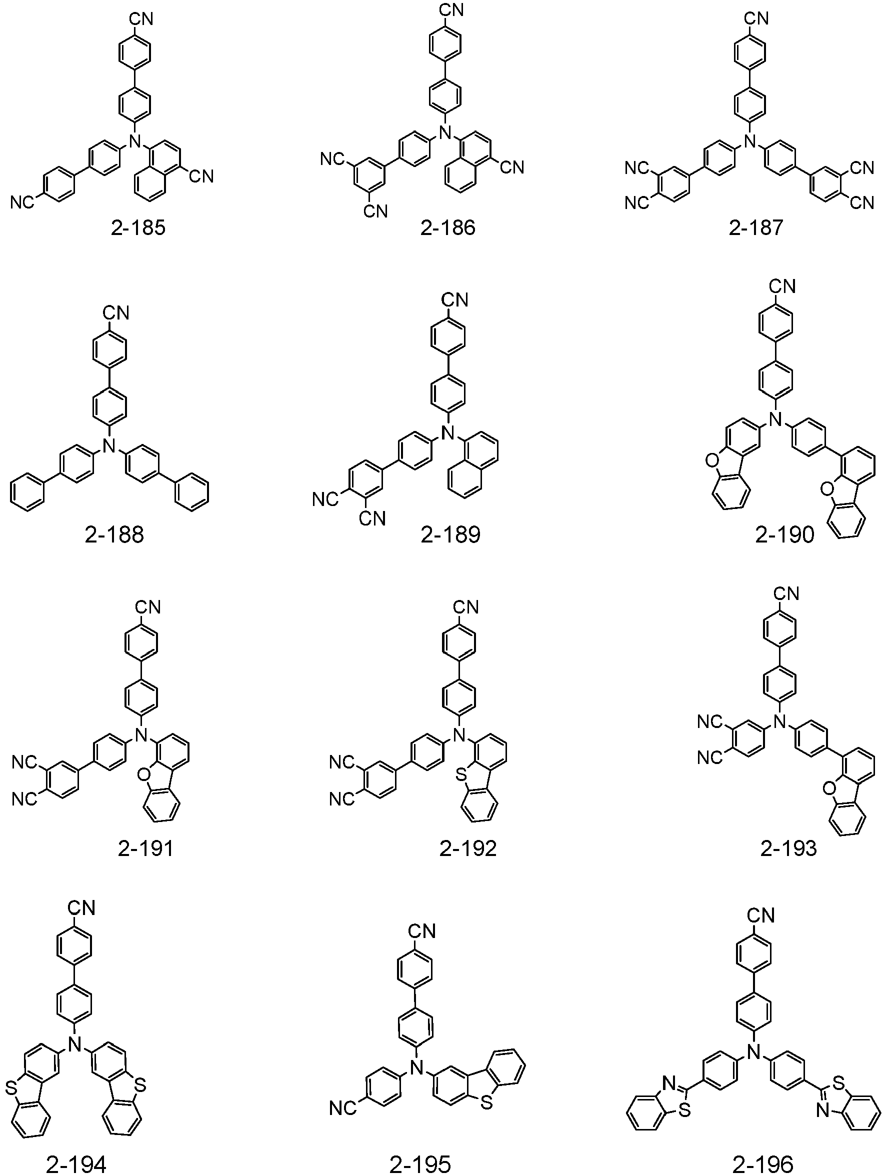

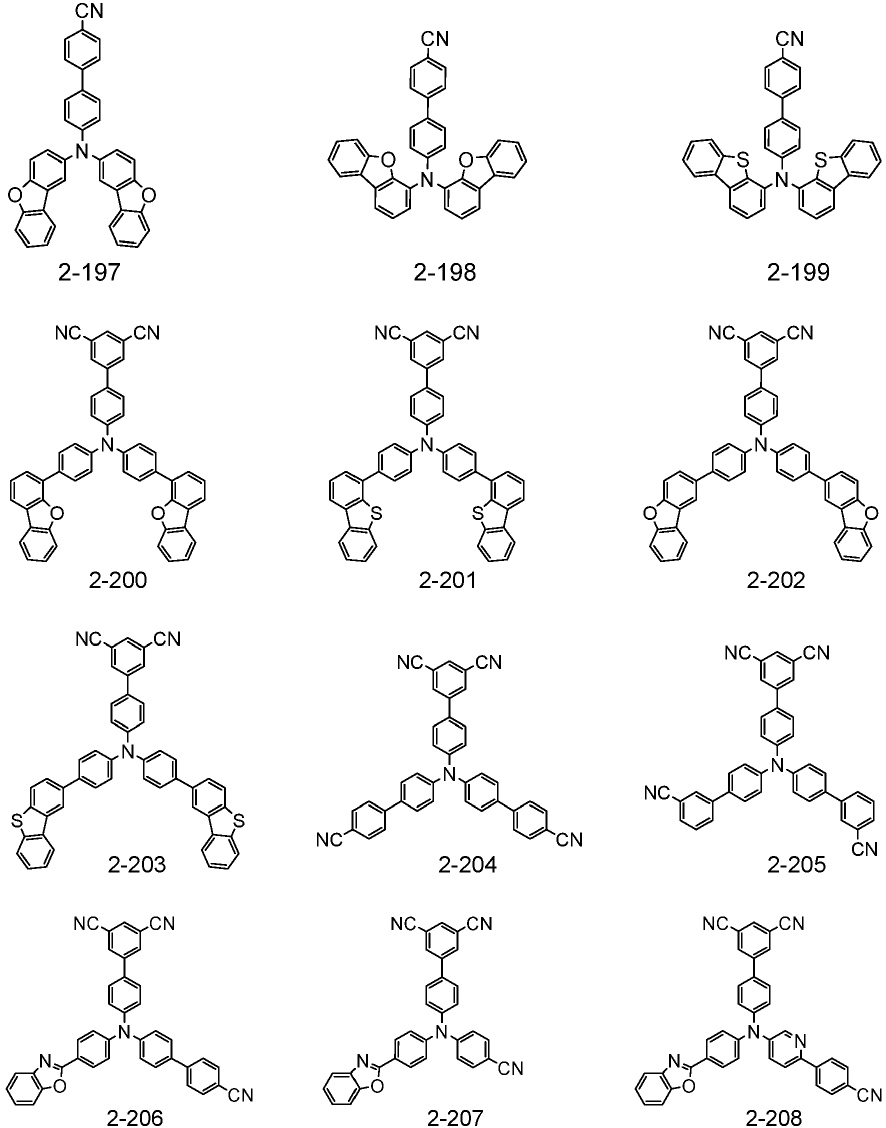

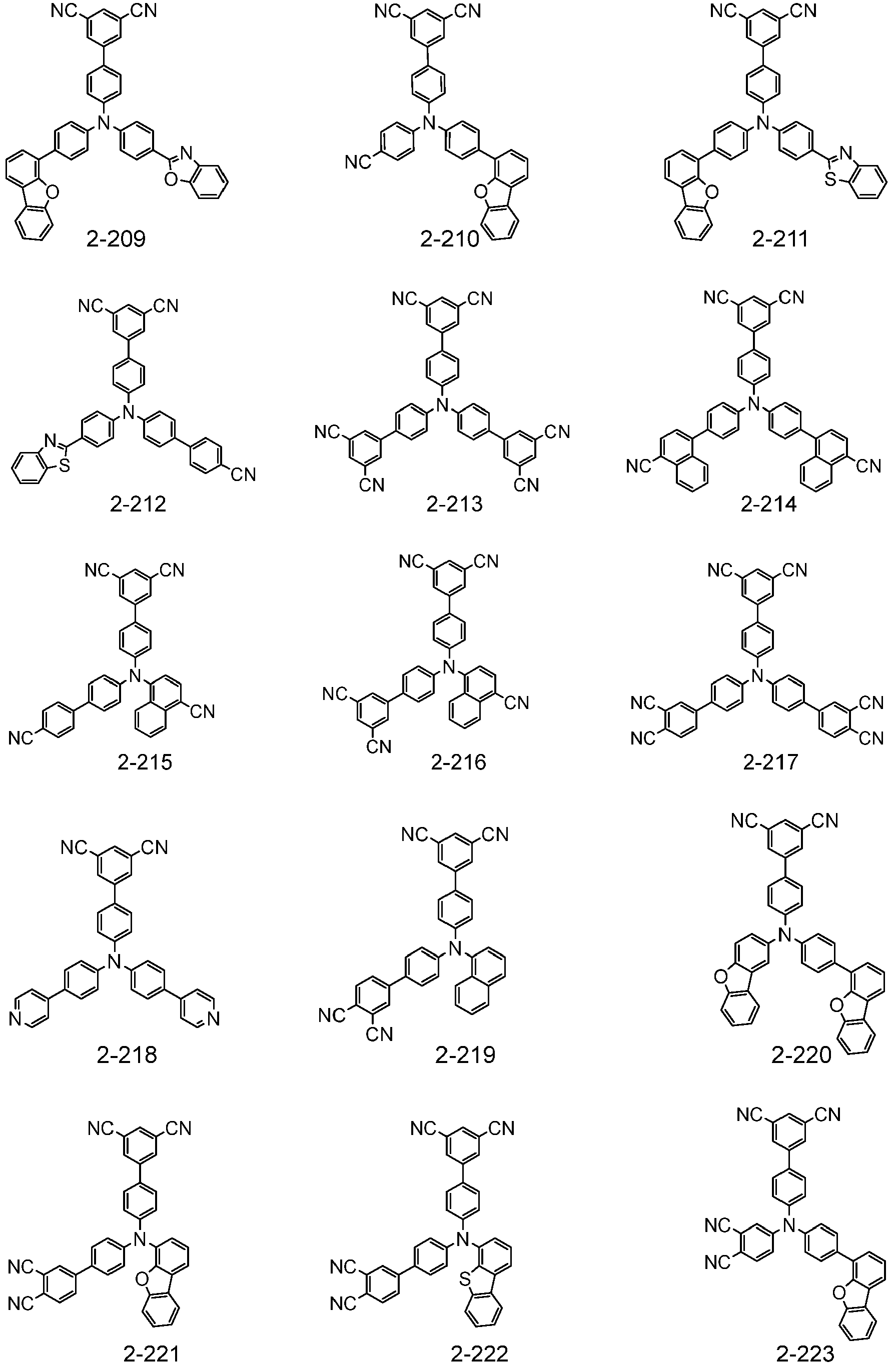

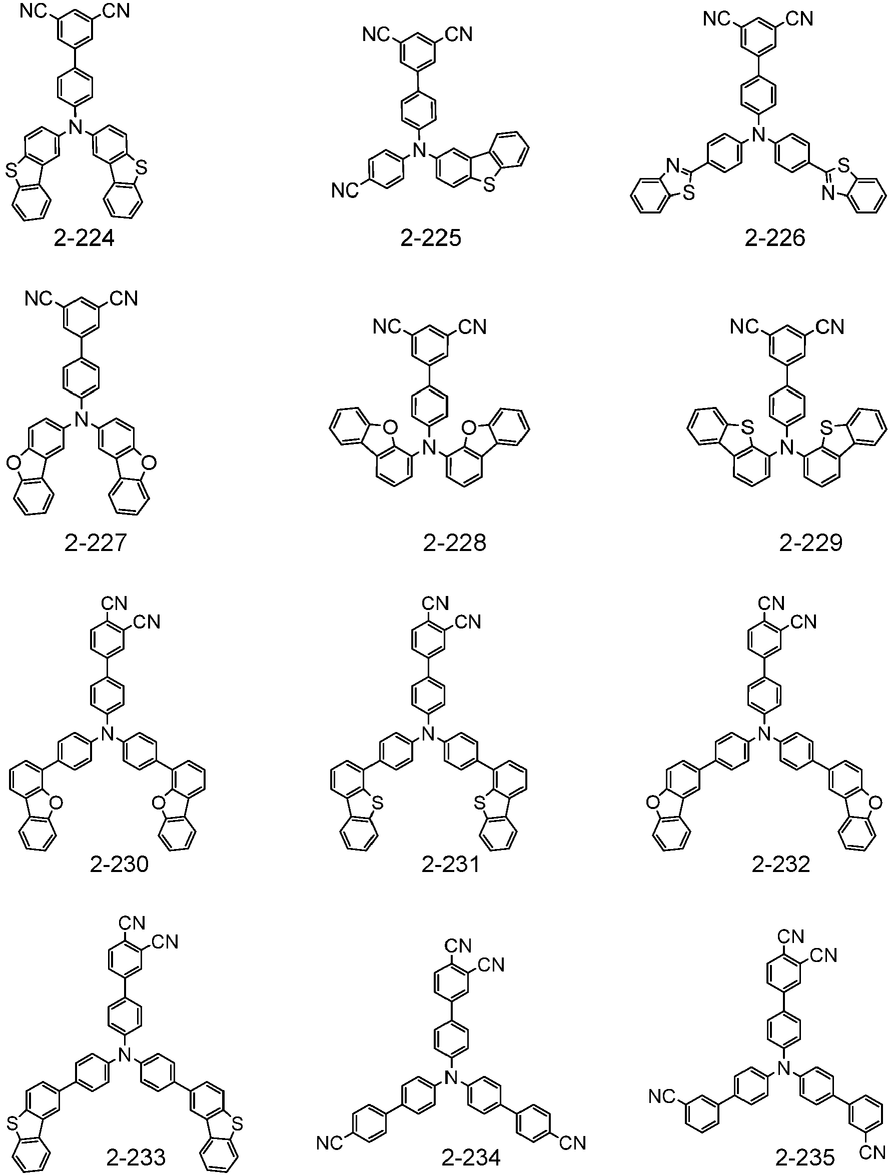

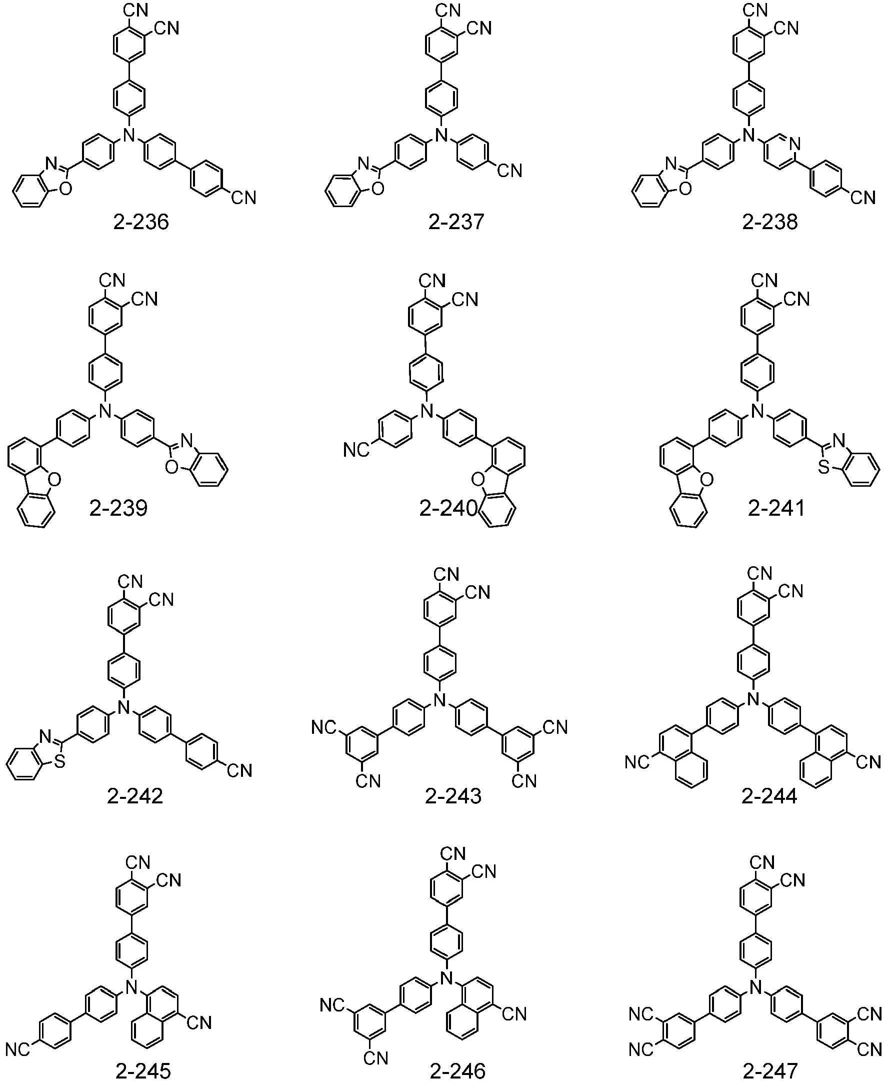

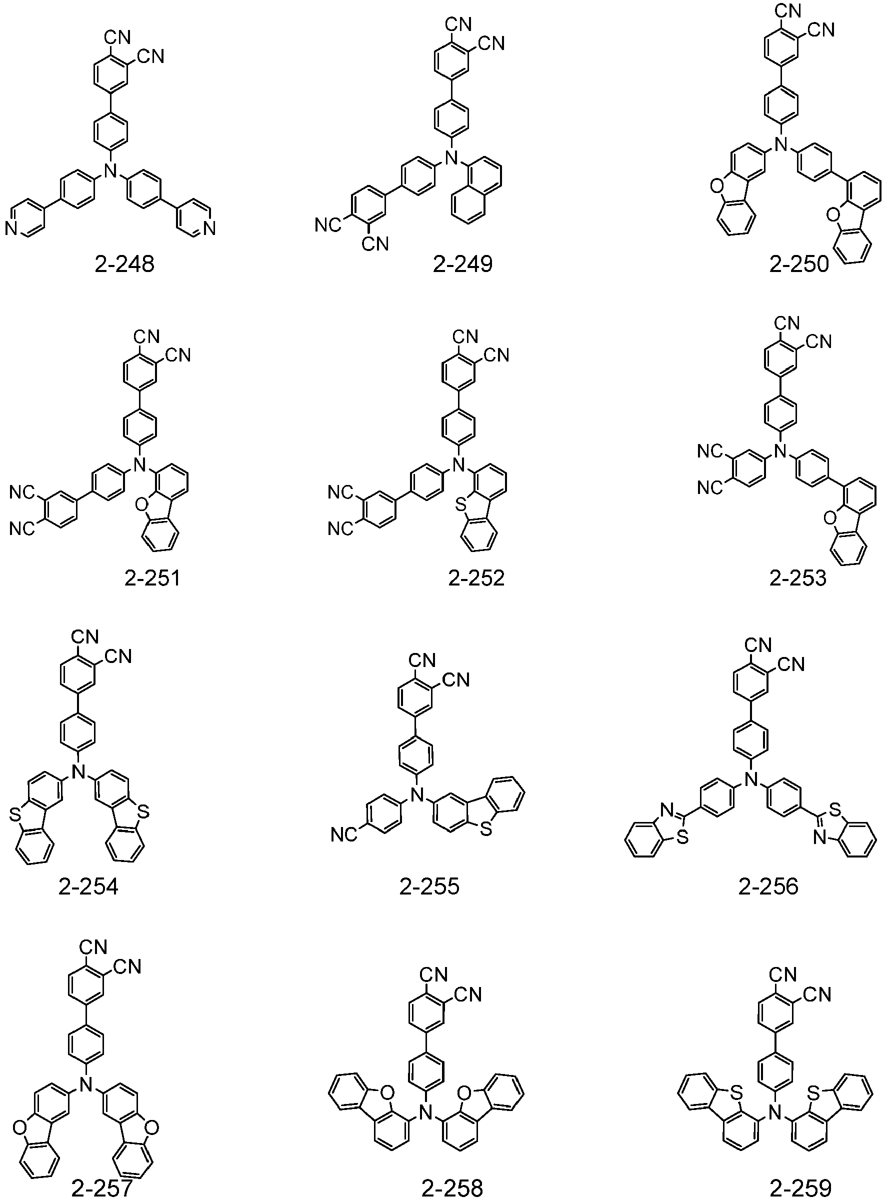

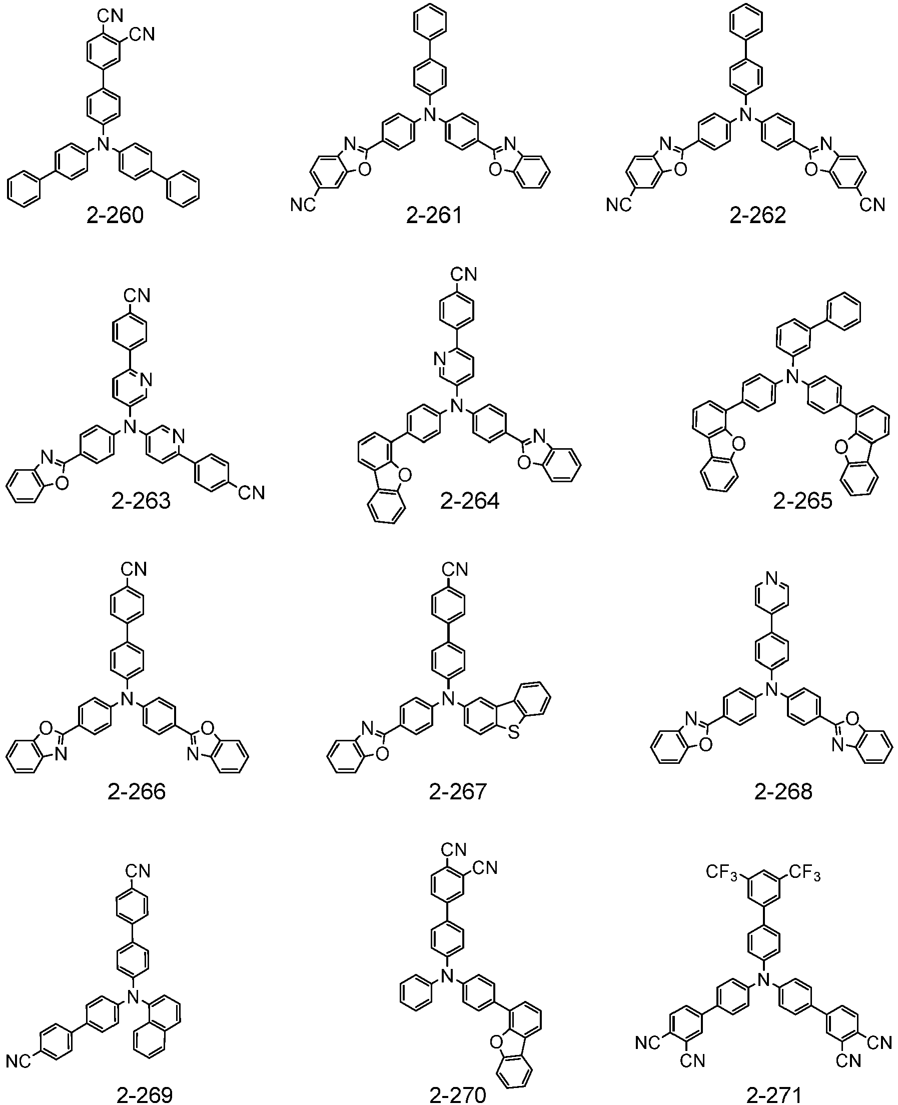

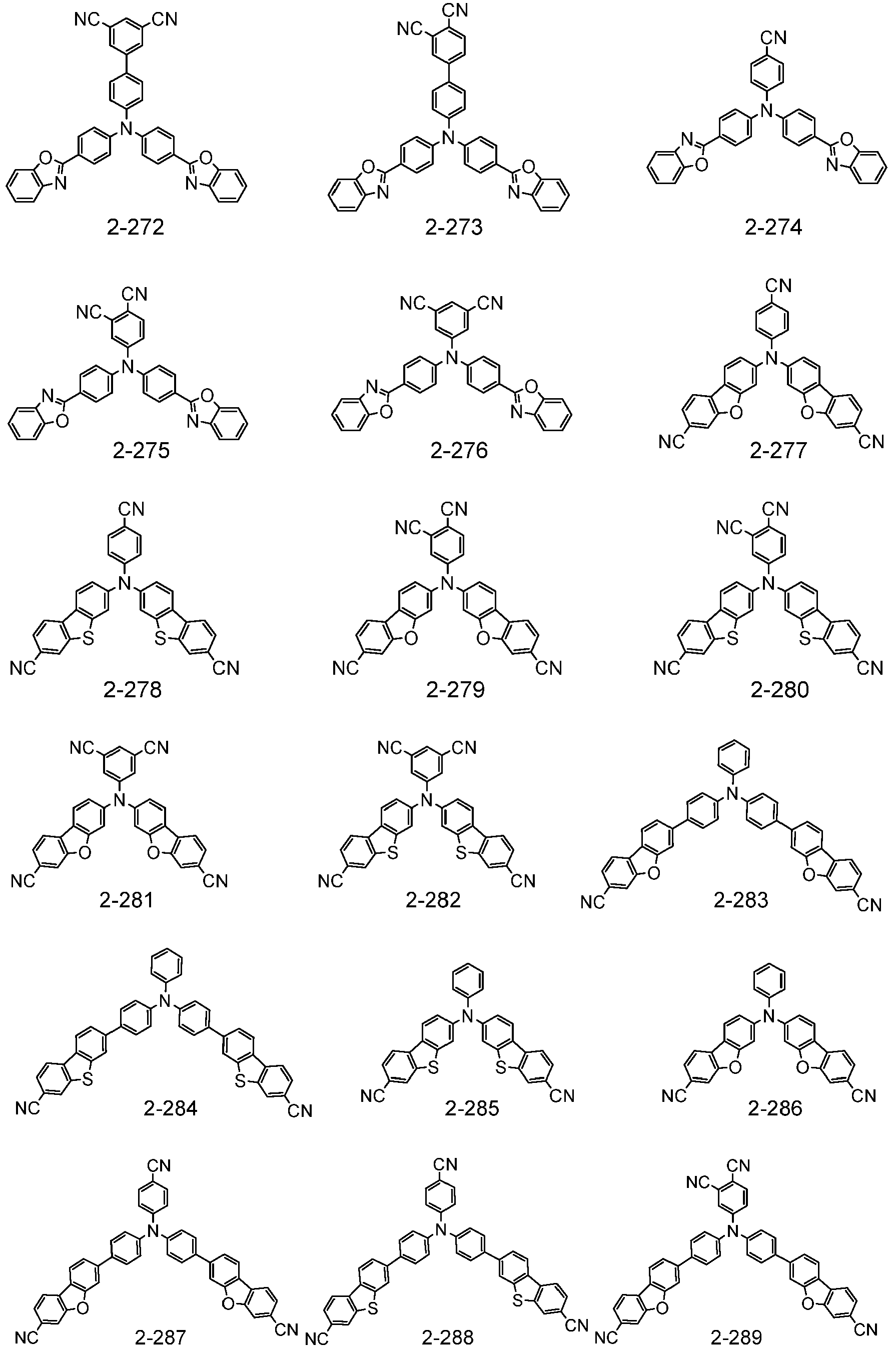

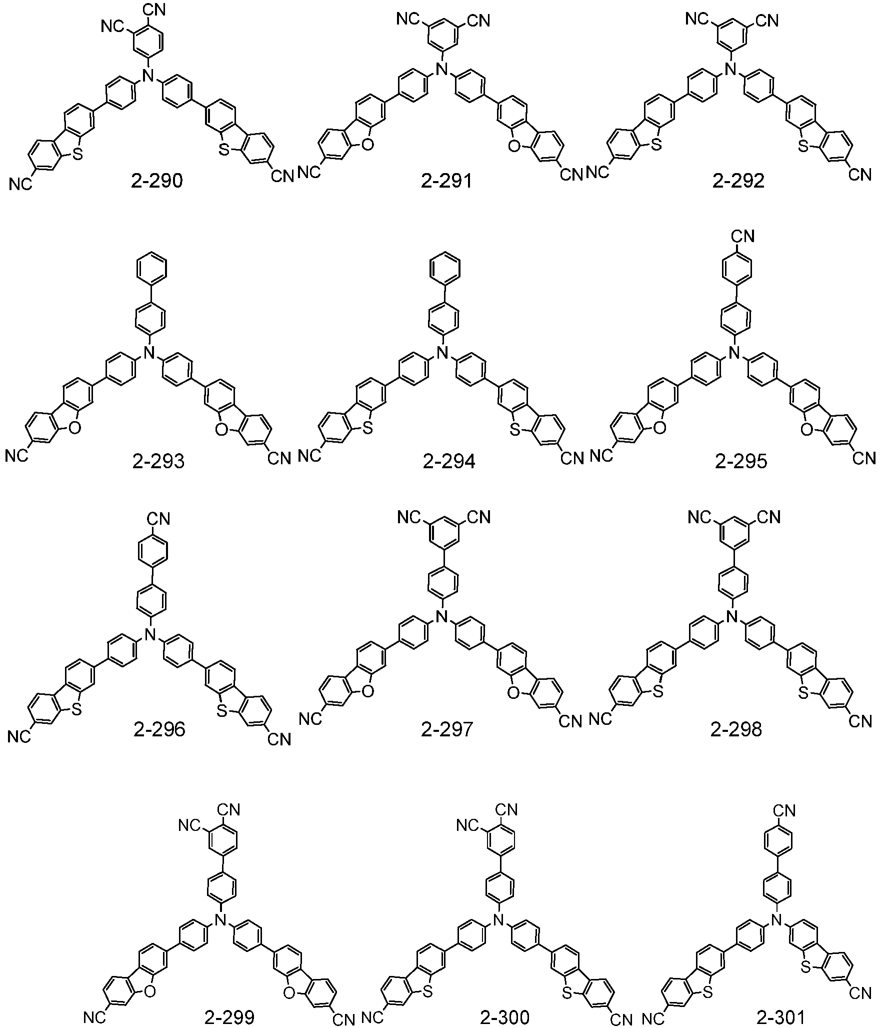

The compounds represented by Formulae 2 may be further substituted.

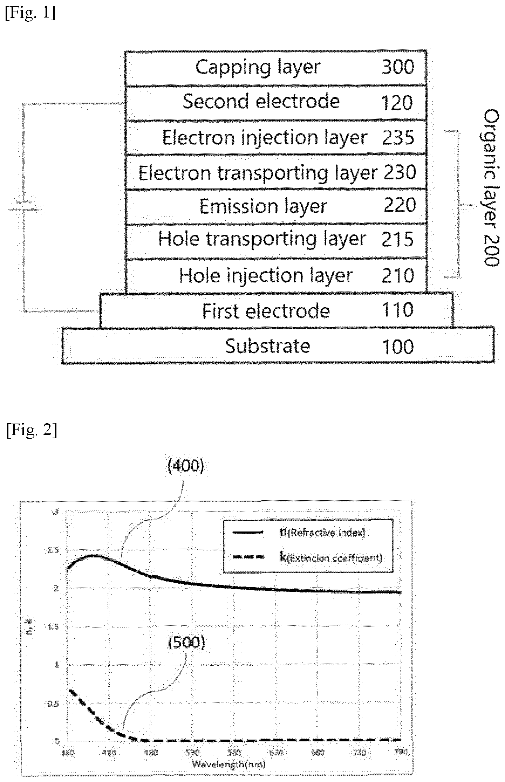

Exemplary embodiments of the present invention will now be described with reference to Figs. 1 and 2.

Fig. 1 is a schematic cross-sectional view of an organic electroluminescent device according to one embodiment of the present invention. Referring to Fig. 1, the organic electroluminescent device includes a first electrode 110, a hole injection layer 210, a hole transporting layer 215, an emission layer 220, an electron transporting layer 230, an electron injection layer 235, a second electrode 120, and a capping layer 300 stacked in this order on a substrate 100.

The first electrode 110 and the second electrode 120 are arranged opposite to each other and an organic layer 200 is arranged therebetween. The hole injection layer 210, the hole transporting layer 215, the emission layer 220, the electron transporting layer 230, and the electron injection layer 235 together form the organic layer 200.

The capping layer 300 is a feature of the present invention. The capping layer 300 is a functional layer deposited on the second electrode 120 and includes an organic material represented by Formula 1.

In the organic electroluminescent device illustrated in Fig. 1, the first electrode 110 is a conductive electrode and may be made of a metal alloy or a conductive material. The first electrode 110 is generally an anode but its function as an electrode is not limited.

The first electrode 110 may be prepared by depositing an electrode material on the substrate 100. Alternatively, electron beam evaporation or sputtering may be used instead of deposition. The material for the first electrode 110 may be selected from high work function materials that facilitate the injection of holes into the organic electroluminescent device.

The capping layer 300 is provided when the organic electroluminescent device is of a top emission type. In this case, a reflective electrode is used as the first electrode 110. Suitable materials for the first electrode include metals and alloys rather than oxides, for example, magnesium (Mg), aluminum (Al), aluminum-lithium (Al-Li), calcium (Ca), magnesium-indium (Mg-In), and magnesium-silver (Mg-Ag). Carbon-based flexible materials such as carbon nanotubes (CNTs) and graphene have also been used in recent years.

The organic layer 200 may consist of a plurality of layers. In this case, the organic layer 200 may include a hole transporting region 210-215 arranged on the first electrode 110, an emission layer 220 arranged on the hole transporting region, and an electron transporting region 230-235 arranged on the emission layer 220.

The capping layer 300 may include an organic compound represented by Formula 1, which will be described below.

The hole transporting region 210-215 is provided on the first electrode 110. The hole transporting region 210-215 may include a hole injection layer 210, a hole transporting layer 215, a hole buffer layer and/or an electron blocking layer (EBL). The hole transporting region 210-215 serves to ensure smooth hole injection and transport into the emission layer. The hole transporting region 210-215 is generally larger in thickness than the electron transporting region because the hole mobility is larger than the electron mobility.

The hole transporting region 210-215 may have a monolayer structure composed of a single material, a monolayer structure composed of different materials or a multilayer structure consisting of a plurality of layers composed of different materials.

For example, the hole transporting region 210-215 may include a hole injection layer 210 and/or a hole transporting layer 215. Alternatively, the hole transporting region 210-215 may have a monolayer structure composed of a hole injection material and a hole transporting material. Alternatively, the hole transporting region 210-215 may have a monolayer structure composed of a plurality of different materials. Alternatively, the hole transporting region 210-215 may have a multilayer structure consisting of a hole injection layer 210 and a hole transporting layer 215 sequentially stacked on the first electrode 110. Alternatively, the hole transporting region 210-215 may have a multilayer structure consisting of a hole injection layer 210, a hole transporting layer 215, and a hole buffer layer sequentially stacked on the first electrode 110. Alternatively, the hole transporting region 210-215 may have a multilayer structure consisting of a hole injection layer 210, a hole buffer layer, a hole transporting layer 215, and a hole buffer layer sequentially stacked on the first electrode 110. Alternatively, the hole transporting region 210-215 may have a multilayer structure consisting of a hole injection layer 210, a hole transporting layer 215, and an electron blocking layer (EBL) sequentially stacked on the first electrode 110. However, the hole transporting region is not limited to the above structures.

The hole injection layer 210 of the hole transporting region 210-215 may be formed on the anode by any suitable method such as vacuum deposition, spin coating, casting or LB deposition. For example, vacuum deposition for the formation of the hole injection layer 210 may be performed at a rate of about 1 Å/s at 100 to 500 °C. The vacuum deposition conditions are not particularly limited and can be freely controlled depending on the kind of a material for the hole injection layer 210 and the desired structure and thermal properties of the hole injection layer 210. Alternatively, the hole injection layer 210 may be formed by spin coating. The coating conditions may vary depending on the kind of a material for the hole injection layer 210 and the characteristics of layers forming interfaces with the hole injection layer 210. An appropriate coating speed and a suitable thermal process for solvent removal after coating are required to make the hole injection layer 210 even.

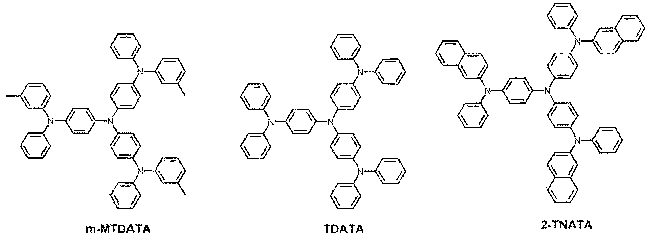

For example, the hole transporting region 210-215 may include one or more of m-MTDATA, TDATA, 2-TNATA, NPB, β-NPB, TPD, spiro-TPD, spiro-NPB, methylated-NPB, TAPC, HMTPD, 4,4',4"-tris(N-carbazolyl)triphenylamine (TCTA), polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline/poly(4-styrenesulfonate) (Pani/PSS).

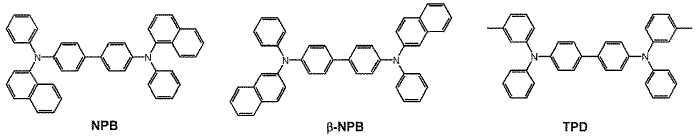

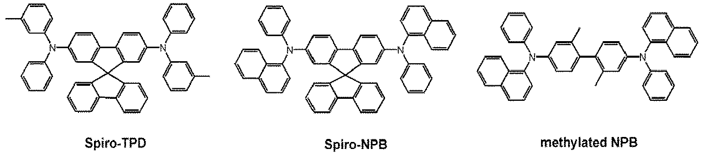

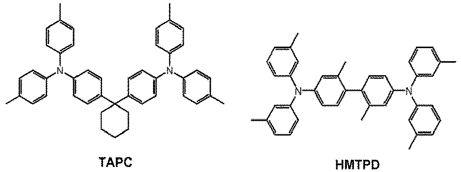

The hole transporting region 210-215 may have a thickness of 100 to 10,000 Å and the constituent organic layers of the hole transporting region 210-215 do not need to have the same thickness. For example, the hole injection layer 210 may have a thickness of 50 Å, the hole transporting layer 215 may have a thickness of 1000 Å, and the electron blocking layer may have a thickness of 500 Å. The thickness of the hole transporting region 210-215 can be determined to such a degree that the efficiency and lifetime of the organic electroluminescent device are satisfactory without an excessive increase in the driving voltage of the organic electroluminescent device.

The organic layer 200 may include one or more layers selected from the group consisting of a hole injection layer 210, a hole transporting layer 215, a functional layer having functions of injecting and transporting holes, a buffer layer, an electron blocking layer, an emission layer 220, a hole blocking layer, an electron transporting layer 230, an electron injection layer 235, and a functional layer having functions of transporting and injecting electrons.

The hole transporting region 210-215 may be doped with a charge generating material to improve the characteristics of the organic electroluminescent device, like the emission layer 220. This doping into the hole transporting region 210-215 can improve the electrical properties of the organic electroluminescent device.

The charge generating material is generally a material with very low HOMO and LUMO energy levels. For example, the LUMO energy level of the charge generating material is similar to the HOMO energy level of a material for the hole transporting layer 215. The low LUMO energy level facilitates hole transfer to the adjacent hole transporting layer 215 based on the electron vacancy in the LUMO, achieving improved electrical properties.

The charge generating material may be, for example, a p-type dopant. Non-limiting examples of such p-type dopants include: quinone derivatives such as tetracyanoquinodimethane (TCNQ) and 2,3,5,6-tetrafluoro-tetracyano-1,4-benzoquinodimethane (F4-TCNQ); metal oxides such as tungsten oxides and molybdenum oxides; and cyano group-containing compounds.

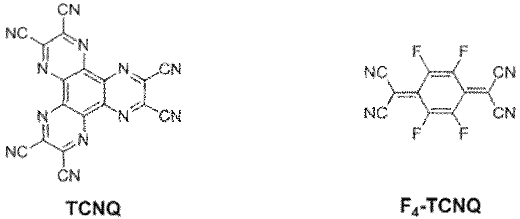

The hole transporting region 210-215 may further include a charge generating material to achieve improved conductivity, in addition to the aforementioned materials.

The charge generating material may be uniformly or non-uniformly dispersed in the hole transporting region 210-215. The charge generating material may be, for example, a p-type dopant. Non-limiting examples of such p-type dopants include: quinone derivatives such as tetracyanoquinodimethane (TCNQ) and 2,3,5,6-tetrafluoro-tetracyano-1,4-benzoquinodimethane (F4-TCNQ); metal oxides such as tungsten oxides and molybdenum oxides; and cyano group-containing compounds.

As described above, the hole transporting region 210-215 may further include a hole buffer layer and/or an electron blocking layer, in addition to the hole injection layer 210 and the hole transporting layer 215. The hole buffer layer may compensate for a resonance distance of light emitted from the emission layer 220 depending on the wavelength of the light to enhance the efficiency of light emission. The hole buffer layer may include the same material as the hole transporting region 210-215.

The electron blocking layer serves to prevent the injection of electrons from the electron transporting region 230-235 into the hole transporting region 210-215. The electron blocking layer may be formed using a material having a high T1 value that can not only block the migration of electrons to the hole transporting region but also can prevent excitons formed in the emission layer 220 from diffusing into the hole transporting region 210-215. For example, a host with a high T1 value for the emission layer 220 is usually used as a material for the electron blocking layer.

The emission layer 220 is provided on the hole transporting region 210-215. For example, the emission layer 220 may have a thickness of 100 Å to 1000 Å or 100 Å to 300 Å. The emission layer 220 may have a monolayer structure composed of a single material, a monolayer structure composed of different materials or a multilayer structure consisting of a plurality of layers composed of different materials.

The emission layer 220 is a region where holes recombine with electrons to form excitons. Materials for the emission layer 220 should have appropriate energy band gaps such that excellent luminescent properties are obtained and a desired color of light is emitted. The materials for the emission layer 220 are typically a host and a dopant but are not limited thereto.

The host may include at least one of TPBi, TBADN, ADN (also called "DNA"), CBP, CDBP, TCP, and mCP but is not limited thereto.

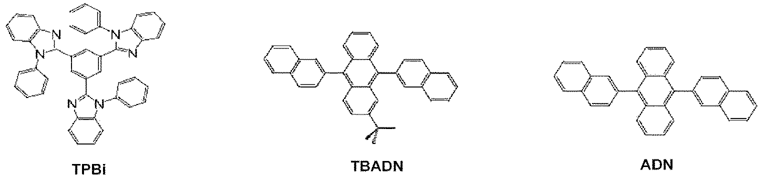

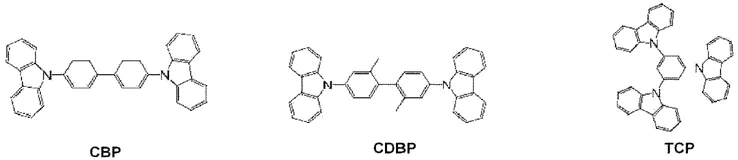

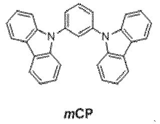

In one embodiment, the dopant may be an organometallic complex. The content of the dopant may generally be selected from 0.01 to 20% but is not limited thereto.

The electron transporting region 230-235 is provided on the emission layer 220. The electron transporting region 230-235 may include a hole blocking layer, an electron transporting layer 230, and/or an electron injection layer 235 but is not limited thereto.

The electron transporting region 230-235 may have a monolayer structure composed of a single material, a monolayer structure composed of different materials or a multilayer structure consisting of a plurality of layers composed of different materials.

For example, the electron transporting region 230-235 may include an electron injection layer 235 and/or an electron transporting layer 230. Alternatively, the electron transporting region 230-235 may have a monolayer structure composed of an electron injection material and an electron transporting material. Alternatively, the electron transporting region 230-235 may have a monolayer structure composed of a plurality of different materials. Alternatively, the electron transporting region 230-235 may have a multilayer structure consisting of an electron transporting layer 230 and an electron injection layer 235 sequentially stacked on the emission layer 220. Alternatively, the electron transporting region 230-235 may have a multilayer structure consisting of a hole blocking layer, an electron transporting layer 230, and an electron injection layer 235 sequentially stacked on the emission electrode 220. However, the electron transporting region is not limited to the above structures. The thickness of the electron transporting region 230-235 may be, for example, 1000 Å to 1500 Å.

The electron transporting region 230-235 may be formed by any suitable method such as vacuum deposition, spin coating, casting, Langmuir-Blodgett (LB) deposition, inkjet printing, laser printing or laser induced thermal imaging (LITI).

The electron transporting layer 230 of the electron transporting region 230-235 may include an anthracene-based compound. Non-limiting examples of suitable materials for the electron transporting layer 230 include, but are not limited to, tris(8-hydroxyquinolinato) aluminum (Alq3), 1,3,5-tri[(3-pyridyl)-phen-3-yl]benzene, 2,4,6-tris(3'-(pyridin-3-yl)biphenyl-3-yl)-1,3,5-triazine, 2-(4-(N-phenylbenzoimidazol-1-ylphenyl)-9,10-dinaphthylanthracene, 1,3,5-tri(1-phenyl-1H-benzo[d]imidazol-2-yl)phenyl) (TPBi), 2,9-dimethyl-4,7-diphenyl-1,10- phenanthroline (BCP), 4,7-diphenyl-1,10-phenanthroline (Bphen), 3-(4-biphenylyl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenylyl)-5-(4-tert-butylphenyl)-1,3,4-oxadiazole (tBu-PBD), bis(2-methyl-8-quinolinolato-N1,O8)-(1,1'-biphenyl-4-olato)aluminum (BAlq), beryllium bis(benzoquinolin-10-olate) (Bebq2), 9,10-di(naphthalene-2-yl)anthracene (ADN). These materials may be used alone or as a mixture thereof.

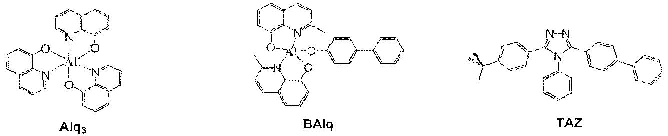

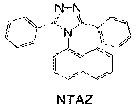

The material for the electron transporting layer 230 may be selected from materials with either high or low electron mobility. The selection varies depending on the structure of the organic light emitting device. The electron transporting layer 230 may be optionally doped with Liq or Li.

The thickness of the electron transporting layer 230 may be in the range of 100 Å to 1000 Å, for example, 150 Å to 500 Å. Within this range, satisfactory electron transporting properties can be obtained without a substantial increase in driving voltage.

The electron injection layer 235 of the electron transporting region 230-235 may include a metal material that facilitates electron injection. Examples of suitable metal materials for the electron injection layer 235 include, but are not limited to, LiF, lithium quinolate (LiQ), Li₂O, BaO, NaCl, CsF, lanthanide metals such as Yb, and metal halides such as RbCl and RbI.

The electron injection layer 235 may be formed of a mixture of an electron transporting material and an insulating organometallic salt. The organometallic salt may be a material having an energy band gap of approximately 4 eV or more. Specific examples of suitable organometallic salts for the electron injection layer include metal acetates, metal benzoates, metal acetoacetates, metal acetylacetonates, and metal stearates. The thickness of the electron injection layer 235 is in the range of 1 Å to 100 Å, preferably 3 Å to 90 Å. Within this range, satisfactory electron injection properties can be obtained without a substantial increase in driving voltage.

As mentioned previously, the electron transporting region 230-235 may include a hole blocking layer. For example, the hole blocking layer may include at least of 2,9-dimethyl-4,7-diphenyl-1,10-phenanthroline (BCP), 4,7-diphenyl-1,10-phenanthroline (Bphen), and Balq. However, the material for the hole blocking layer is not limited.

The second electrode 120 is provided on the electron transporting region 230-235. The second electrode 120 may be a common electrode or a cathode. The second electrode 120 may be a transmissive or transflective electrode. Unlike the first electrode 110, the second electrode 120 may be made of a combination of a relatively low work function metal, an electrically conductive compound, an alloy, etc.

The second electrode 120 is a transflective or reflective electrode. The second electrode 120 may include lithium (Li), magnesium (Mg), aluminum (Al), aluminum-lithium (Al-Li), calcium (Ca), magnesium-indium (Mg-In), magnesium-silver (Mg-Ag), or a compound or mixture containing the metal or alloy (e.g., a mixture of Ag and Mg). Alternatively, the second electrode 120 may have a multilayer structure consisting of a reflective or transflective film and a transparent conductive film. The material for the reflective or transflective film is the same as that described above for the transflective or reflective electrode. For example, the transparent conductive film may be formed of indium tin oxide (ITO), indium zinc oxide (IZO), zinc oxide (ZnO) or indium tin zinc oxide (ITZO).

Although not shown, the second electrode 120 may be connected to an auxiliary electrode. This connection can reduce the resistance of the second electrode 120.

The substrate 100 on which the electrodes and the organic layer are disposed may be made of a rigid or flexible material. For example, the rigid material may be soda-lime glass, alkali-free glass or aluminosilicate glass and the flexible material may be polycarbonate (PC), polyethersulfone (PES), a cyclic olefin copolymer (CEC), polyethylene terephthalate (PET) or polyethylene naphthalate (PEN).

When a voltage is applied to the first and second electrodes 110 and 120 of the organic electroluminescent device, holes are injected from the first electrode 110 and migrate to the emission layer 220 through the hole transporting region 210-215 and electrons are injected from the second electrode 120 and migrate to the emission layer 220 through the electron transporting region 230-235. The electrons recombine with the holes in the emission layer 220 to form excitons, which fall from the excited state to the ground state to emit light.

The path of light generated in the emission layer 220 tends to vary depending on the refractive indices of the organic/inorganic materials constituting the organic electroluminescent device. Only rays of light incident on the second electrode 120 at angles smaller than the critical angle of the second electrode 120 can pass through the second electrode 120. Rays of light coming into contact with the second electrode 120 at angles larger than the critical angle of the second electrode 120 are totally reflected or reflected, and as a result, they cannot be emitted outside the organic electroluminescent device.

A high refractive index of the capping layer 300 reduces the total reflection or reflection of light entering the capping layer 300, contributing to an improvement in luminescent efficiency. An appropriate thickness of the capping layer 300 maximizes the number of micro-cavities, contributing to high efficiency and improved color purity.

The capping layer 300 is placed at the outermost position of the organic electroluminescent device and has a great influence on the characteristics of the device without affecting the driving of the device at all. Therefore, the capping layer 300 is important from both viewpoints of protecting the inside of the organic electroluminescent device and improving the characteristics of the device. The organic materials absorb light energy in specific wavelength ranges depending on their energy band gaps. When the energy band gaps are adjusted such that light in the UV region capable of affecting the organic materials present in the organic electroluminescent device is absorbed, the capping layer 300 containing the organic materials can be used to improve the optical properties of the organic electroluminescent device while protecting the organic electroluminescent device.

### Mode for Carrying out the Invention

Organic electroluminescent devices and tribenzazole amine derivatives according to exemplary embodiments of the present invention will be specifically explained with reference to the following examples, including comparative examples. However, these examples are merely illustrative to assist in understanding the present invention and the scope of the present invention is not limited thereto.

### [PREPARATIVE EXAMPLES]

### Intermediate Synthesis Example 1: Synthesis of Intermediate 1

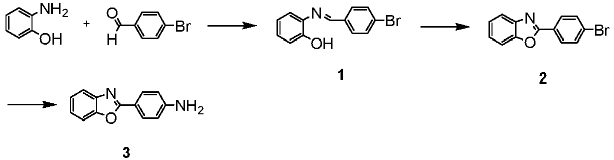

### (Synthesis of Intermediate 2)

10.0 g (91.6 mmol) of 2-aminophenol, 16.9 g (91.6 mmol) of 4-bromobenzaldehyde, and 114 mL of ethanol were stirred at room temperature for 6 h. The reaction mixture was concentrated under reduced pressure and dried to afford crude Intermediate **1.**

Intermediate **1** was dissolved in 370 mL of dichloromethane, and then 22.8 g (100.4 mmol) of 2,3-dichloro-5,6-dicyano-p-benzoquinone (DDQ) was slowly added thereto with stirring at room temperature. After stirring overnight, the reaction mixture was purified by column chromatography to afford 30.5 g (yield 94.0%) of Intermediate **2** as a white solid.

### (Synthesis of Intermediate 3)

10.0 g (36.50 mmol) of Intermediate **2,** 7.9 g (43.8 mmol) of benzophenone imine, 1.1 g (1.8 mmol) of Pd(dba)₂, 2.3 g (3.7 mmol) of BINAP, 35.7 g (109.6 mmol) of cesium carbonate, and 243 mL of toluene were stirred under reflux for 12 h. The reaction mixture was cooled to room temperature and dissolved in chloroform. The solution was passed through a pad of celite and concentrated under reduced pressure. The resulting residue was suspended in 182 mL of tetrahydrofuran and then 30 mL of concentrated hydrochloric acid was slowly added thereto. The mixture was stirred at room temperature overnight. The resulting precipitate was filtered under reduced pressure and washed with chloroform. The filtered wet product was suspended in 600 mL of water, adjusted to pH ≥ 8 with a saturated sodium carbonate solution, and extracted with chloroform. The chloroform layer was separated, dried over anhydrous sodium sulfate, filtered, and concentrated under reduced pressure. The residue was slurried with dichloromethane and n-hexane to afford 5.9 g (yield 78.0%) of Intermediate **3** as a yellow solid.

### Intermediate Synthesis Example 2: Synthesis of Intermediate 4

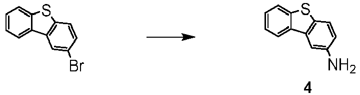

50.0 g (190.0 mmol) of 2-bromodibenzo[b,d]thiophene, 37.8 g (208.6 mmol) of benzophenone imine, 5.5 g (9.5 mmol) of Pd(dba)₂, 11.8 g (19.0 mmol) of BINAP, 45.6 g (474.5 mmol) of sodium *tert*-butoxide, and 800 mL of toluene were stirred under reflux for 12 h. The reaction mixture was cooled to room temperature and dissolved in chloroform. The solution was passed through a pad of celite and concentrated under reduced pressure. The resulting residue was suspended in 600 mL of tetrahydrofuran and then 30 mL of concentrated hydrochloric acid was slowly added thereto. The mixture was stirred at room temperature overnight. The resulting precipitate was filtered under reduced pressure and washed with chloroform. The filtered wet product was suspended in 300 mL of water, adjusted to pH ≥ 8 with a saturated sodium carbonate solution, and extracted with chloroform. The chloroform layer was separated, dried over anhydrous sodium sulfate, filtered, and concentrated under reduced pressure. The residue was slurried with dichloromethane and n-hexane to afford 25.6 g (yield 68.0%) of Intermediate **4** as a yellow solid.

### Intermediate Synthesis Example 3: Synthesis of Intermediate 6

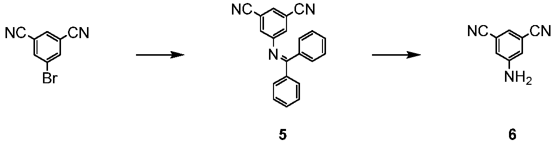

### (Synthesis of Intermediate 5)

40.0 g (193.2 mmol) of 5-bromoisophthalonitrile, 42.0 mL (231.8 mmol) of diphenylmethanimine, 11.1 g (19.3 mmol) of Pd(dba)₂, 24.1 g (38.6 mmol) of BINAP, 188.8 g (579.6 mmol) of Cs₂CO₃, and 966 mL of toluene were placed and mixed in a 3 L one-neck flask. The mixture was stirred under reflux for 17 h. After completion of the reaction, the reaction mixture was cooled to room temperature, filtered through celite, concentrated, purified by silica gel column chromatography (Hex:DCM), and washed with methanol to afford 40.5 g (yield 68.2%) of Intermediate **5** as a pale yellow solid.

### (Synthesis of Intermediate 6)

40.5 g (131.8 mmol) of Intermediate **5** was added to and dissolved in 440 mL of THF in a 1 L one-neck flask, and then 175.7 mL (1.0 mol) of a 6 N hydrochloric acid solution was slowly added dropwise thereto. The mixture was stirred at room temperature for 2 days. The resulting mixture was neutralized by slow dropwise addition of 42.2 g of NaOH. After stirring for 30 min, the reaction was terminated. To the reaction mixture were added dichloromethane and distilled water. The organic layer was separated and dried over anhydrous magnesium sulfate. The solvent was removed under reduced pressure to precipitate a solid. The solid was washed with dichloromethane to afford 14.8 g (yield 78.7%) of Intermediate **6** as a white solid.

### Intermediate Synthesis Example 4: Synthesis of Intermediate 7

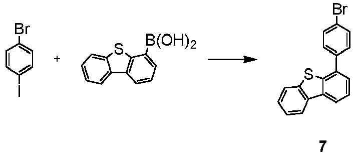

37.2 g (131.5 mmol) of 1-bromo-4-iodobenzene, 30.0 g (131.5 mmol) of dibenzo[b,d]thiophen-4-ylboronic acid, 4.6 g (3.9 mmol) of Pd(PPh₃)₄, and 400 mL of toluene were added together and stirred in a 1000 mL one-neck flask, and then 200 mL of ethanol and 27.3 g (197.3 mmol) of K₂CO₃/200 mL of water were added thereto. The mixture was heated to reflux with stirring for 8 h. After completion of the reaction, the reaction mixture was cooled to room temperature and the solvent was removed. The resulting mixture was added with water and extracted with dichloromethane. The organic phase was dried over anhydrous MgSO₄ and purified by column chromatography (DCM) to afford 29.1 g (yield 65.3%) of Intermediate **7** as a slightly yellow solid.

### Intermediate Synthesis Example 5: Synthesis of Intermediate 9

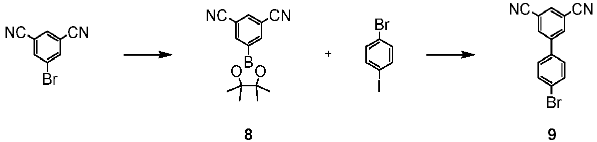

### (Synthesis of Intermediate 8)

10.0 g (48.3 mmol) of 5-bromoisophthalonitrile, 14.7 g (58.0 mmol) of bis(pinacolato)diboron, 1.6 g (1.9 mmol) of Pd(dppf)Cl₂, 9.5 g (96.6 mmol) of potassium acetate (KOAc), and 300 mL of dioxane were placed together in a 500 mL one-neck flask. The mixture was refluxed at 100 °C under a nitrogen atmosphere for one day. After completion of the reaction, the reaction mixture was evaporated to remove the solvent, added with water, and extracted with chloroform. The organic layer was separated, dried over anhydrous MgSO₄, and purified by column chromatography (Hex:CHCl₃) to afford 7.6 g (yield 62.1%) of Intermediate **8** as a white solid.

### (Synthesis of Intermediate 9)

7.6 g (30.0 mmol) of Intermediate **8,** 8.5 g (30.0 mmol) of 1-bromo-4-iodobenzene, 1.0 g (0.9 mmol) of Pd(PPh₃)₄, and 150 mL of toluene were added together and stirred in a 500 mL one-neck flask, and then 80 mL of ethanol, 8.3 g (59.8 mmol) of K₂CO₃, and 80 mL of distilled water were added thereto. The mixture was heated to reflux with stirring for one day. After completion of the reaction, the reaction mixture was cooled to room temperature and the solvent was removed. The resulting mixture was added with distilled water and extracted with dichloromethane. The organic layer was dried over anhydrous MgSO₄ and purified by column chromatography (Hex:DCM) to afford 2.9 g (yield 34.1%) of Intermediate **9** as a light yellow solid.

### Intermediate Synthesis Example 6: Synthesis of Intermediate 10

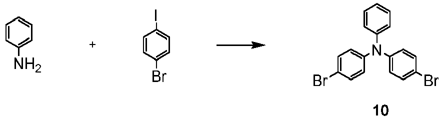

1.5 mL (16.4 mmol) of aniline, 10.2 g (36.1 mmol) of 1-bromo-4-iodobenzene, 0.5 g (0.8 mmol) of Pd(dba)₂, 0.9 g (1.6 mmol) of DPPF, 4.7 g (49.3 mmol) of NaOtBu, and 65 mL of toluene were stirred under reflux in a 250 mL one-neck flask for 5 h. The reaction mixture was cooled to room temperature and filtered through celite to remove impurities. After removal of the solvent, the resulting mixture was purified by silica gel column chromatography (EA:HEX) to afford 4.1 g (yield 61.8%) of Intermediate **10** as a colorless liquid.

### Intermediate Synthesis Example 7: Synthesis of Intermediate 12

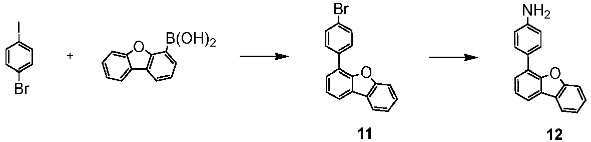

### (Synthesis of Intermediate 11)

A mixture of 10.0 g (35.3 mmol) of 4-bromo-1-iodobenzene, 8.2 g (38.9 mmol) of dibenzofuran-4-ylboronic acid, 0.2 g (1.1 mmol) of Pd(PPh₃)₄, 36.0 mL (72.0 mmol) of a 2 M sodium carbonate solution, 90 mL of toluene, and 36 mL of ethanol was stirred under reflux for 12 h. The reaction mixture was cooled to room temperature, diluted with 100 mL of toluene, and washed with water. The organic layer was separated, dried over anhydrous magnesium sulfate, filtered, and concentrated to afford 10.7 g (yield 93%) of Intermediate **11** as a solid.

### (Synthesis of Intermediate 12)

A mixture of 60.0 g (185.7 mmol) of Intermediate **11,** 37.0 g (204.2 mmol) of benzophenone imine, 5.3 g (9.3 mmol) of Pd(dba)₂, 11.5 g (18.5 mmol) of BINAP, 44.6 g (464.1 mmol) of sodium tert-butoxide, and 600 mL of toluene was stirred under reflux for 12 h. The reaction mixture was cooled to room temperature, dissolved in chloroform, passed through a pad of celite, and concentrated under reduced pressure. The residue was dissolved in 500 mL of tetrahydrofuran and then 80 mL of 6 N hydrochloric acid was slowly added thereto. The mixture was stirred at room temperature overnight. The resulting precipitate was filtered under reduced pressure and washed with chloroform. The filtered wet product was suspended in 600 mL of water, adjusted to pH ≥ 8 with a saturated sodium carbonate solution, and extracted with chloroform. The chloroform layer was separated, dried over anhydrous sodium sulfate, filtered, and concentrated under reduced pressure. The residue was slurried with dichloromethane and n-hexane to afford 28.0 g (yield 58%) of Intermediate **12** as a yellow solid.

### Intermediate Synthesis Example 8: Synthesis of Intermediate 16

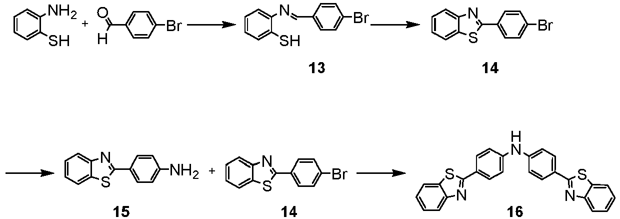

### (Synthesis of Intermediate 14)

10.0 g (76.88 mmol) of 2-aminobenzothiol, 12.8 g (69.42 mol) of 4-bromobenzaldehyde, and 130 mL of ethanol were stirred at room temperature for 6 h. After completion of the reaction, the reaction mixture was distilled under reduced pressure to remove the solvent and dried to afford crude Intermediate **13.** The intermediate was used for the subsequent reaction without further purification.

Intermediate **13** was dissolved in 320 mL of dichloromethane, and then 19.9 g (0.09 mol) of 2,3-dichloro-5,6-dicyano-p-benzoquinone was slowly added thereto with stirring at room temperature. After stirring for one day, the reaction mixture was purified by column chromatography (DCM) and solidified with methanol to afford 26.6 g (yield 90.2%) of Intermediate **14** as a white solid.

### (Synthesis of Intermediate 15)

26.6 g (91.67 mmol) of Intermediate **14,** 19.9 g (110.00 mmol) of benzophenone imine, and 270 mL of toluene were placed in a 1000 mL one-neck flask, and then 2.6 g (4.58 mmol) of Pd(dba)₂, 5.7 g (9.17 mmol) of BINAP, and 89.6 g (275.00 mmol) of Cs₂CO₃ were added thereto. The mixture was stirred at 110 °C throughout the day. After completion of the reaction, the reaction mixture was cooled to room temperature, passed through a pad of celite with chloroform under reduced pressure, and distilled under reduced pressure to remove the solvent. The resulting residue was diluted with 300 mL of THF and acidified to pH < 2 by slow addition of 30 mL of concentrated hydrochloric acid. The mixture was stirred at room temperature throughout the day to precipitate a solid. The solid was collected by filtration, washed with chloroform, adjusted to a basic pH (pH > 8) with a saturated Na₂CO₃ solution, extracted with chloroform, dried over MgSO₄, and distilled under reduced pressure to remove the solvent. The residue was slurried with DCM and hexane to afford 17.8 g (yield 85.8%) of Intermediate **15** as a yellow solid.

### (Synthesis of Intermediate 16)

10.0 g (44.2 mmol) of Intermediate **15,** 12.8 g (44.2 mmol) of Intermediate **14,** 4.7 g (48.6 mmol) of NaOtBu, and 150 mL of xylene were added together and stirred in a 250 mL one-neck flask, and 0.8 g (1.3 mmol) of Pd(dba)₂ and 0.6 mL (2.7 mmol) of P(t-Bu)₃ were added thereto. The mixture was heated to reflux with stirring throughout the day. After completion of the reaction, the reaction mixture was cooled to room temperature and the solvent was removed. The resulting mixture was added with methanol, stirred for 30 min, and filtered to obtain a solid. The solid was dissolved in hot monochlorobenzene. The solution was passed through a pad of celite and distilled under reduced pressure to remove the solvent. After the addition of acetone, the resulting mixture was stirred for 30 min and filtered to afford 13.5 g (yield 70.3%) of Intermediate **16** as a yellowish green solid.

### Intermediate Synthesis Example 9: Synthesis of Intermediate 18

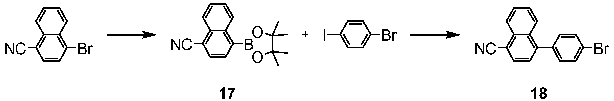

### (Synthesis of Intermediate 17)

30.0 g (129 mmol) of 4-bromo-1-naphthonitrile, 39.4 g (155 mmol) of PIN₂B₂, 5.3 g (6.5 mmol) of Pd(dppf)Cl₂-DCM, 38.1 g (388 mmol) of KOAc, and 650 mLof 1,4-dioxane were stirred under reflux for one day. The reaction mixture was cooled to room temperature and filtered through celite to remove impurities. After complete removal of the solvent, the resulting mixture was purified by silica gel column chromatography (DCM:HEX) and filtered with a mixed solution (acetone/methanol) to afford 30.4 g (yield 84.3%) of Intermediate **17** as a yellow solid.

### (Synthesis of Intermediate 18)

24.0 g (86.0 mmol) of Intermediate **17,** 26.8 g (94.6 mmol) of 1-bromo-4-iodobenzene, 5.0 g (4.3 mmol) of Pd(PPh₃)₄, 120 mL (258.0 mmol) of 2 M K₂CO₃, 240 mL of toluene, and 120 mL of ethanol were stirred under reflux in a 1 L one-neck flask for 2 h. The reaction mixture was cooled to room temperature and extracted with ethyl acetate. After removal of water and the solvent, the resulting mixture was dissolved in chloroform, purified by silica gel column chromatography (CHCl₃:HEX), and filtered with a mixed solution (acetone/methanol) to afford 17.0 g (yield 61.7%) of Intermediate **18** as a white solid.

### Intermediate Synthesis Example 10: Synthesis of Intermediate 19

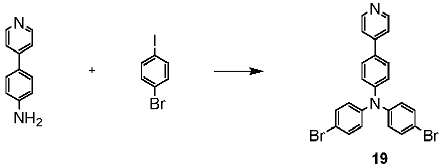

3.0 g (17.6 mmol) of 4-(pyridin-4-yl)aniline, 11.0 g (38.8 mmol) of 1-bromo-4-iodobenzene, 0.5 g (0.9 mmol) of Pd(dba)₂, 1.0 g (1.8 mmol) of DPPF, 5.1 g (52.9 mmol) of NaOtBu, and 90 mL of toluene were stirred under reflux for 8 h. The reaction mixture was cooled to room temperature and filtered through celite to remove impurities. After removal of the solvent, the resulting mixture was purified by silica gel column chromatography (EA:DCM) and filtered with hexane to afford 7.1 g (yield 83.6%) of Intermediate **19** as a brown solid.

### Intermediate Synthesis Example 11: Synthesis of Intermediate 21

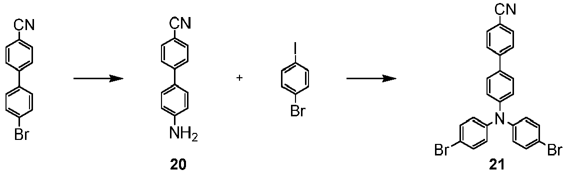

### (Synthesis of Intermediate 20)

51.1 g (198.0 mmol) of 4'-bromo-(1,1'-biphenyl)-4-carbonitrile, 39.5 g (217.8 mmol) of benzophenone imine, 57.1 g (593.9 mmol) of NaOtBu, and 500 mL of toluene were added together and stirred in a 1000 mL one-neck flask, and then 3.4 g (5.9 mmol) of Pd(dba)₂ and 7.4 g (11.9 mmol) of BINAP were added thereto. The mixture was heated to reflux with stirring throughout the day. After completion of the reaction, the reaction mixture was cooled to room temperature, evaporated to remove the solvent, added with water, and extracted with dichloromethane. The organic phase was dried over anhydrous MgSO₄, passed through a pad of celite with chloroform, and distilled under reduced pressure to remove the solvent. The resulting residue as a slightly brown liquid was added to 500 mL of tetrahydrofuran, stirred, adjusted to pH 2 with 4 N HCl, and stirred at 50 °C for 4 h. After completion of the reaction, the solvent was removed. The resulting mixture was added with acetone, stirred for 30 min, and filtered to obtain a red solid. The solid was adjusted to pH ≥ 8 with NaOH, stirred for 30 min, and extracted with dichloromethane. After removal of the solvent, purification by column chromatography (Hex:CHCl₃) afforded 12.0 g (yield 31.1%) of Intermediate **20** as a slightly red solid.

### (Synthesis of Intermediate 21)

3.0 g (15.4 mmol) of Intermediate **20,** 9.6 g (34.0 mmol) of 1-bromo-4-iodobenzene, 0.4 g (0.8 mmol) of Pd(dba)₂, 0.9 g (1.5 mmol) of DPPF, 4.5 g (46.3 mmol) of NaOtBu, and 80 mL of toluene were stirred under reflux in a 250 mL one-neck flask for 8 h. The reaction mixture was cooled to room temperature and filtered through celite to remove impurities. After removal of the solvent, the resulting mixture was dissolved in dichloromethane, purified by silica gel column chromatography (DCM:HEX), and filtered with a mixed solution (acetone/ethanol) to afford 4.7 g (yield 60.9%) of Intermediate **21** as a yellow solid.

### Intermediate Synthesis Example 12: Synthesis of Intermediate 22

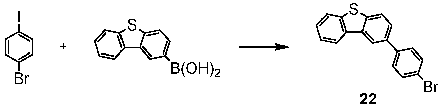

13.6 g (48.2 mmol) of 1-bromo-4-iodobenzene, 10.0 g (43.8 mmol) of dibenzo[b,d]thiophen-2-ylboronic acid, 1.5 g (1.3 mmol) of Pd(PPh₃)₄, and 150 mL of toluene were added together and stirred in a 500 mL one-neck flask, and then 70 mL of ethanol, 9.1 g (65.8 mmol) of K₂CO₃, and 70 mL of water were added thereto. The mixture was heated to reflux with stirring for 8 h. After completion of the reaction, the reaction mixture was cooled to room temperature, evaporated to remove the solvent, added with water, and extracted with dichloromethane. The organic phase was separated, dried over anhydrous MgSO₄, and purified by column chromatography (DCM) to afford 9.3 g (yield 62.6%) of Intermediate **22** as a slightly yellow solid.

### Intermediate Synthesis Example 13: Synthesis of Intermediate 23

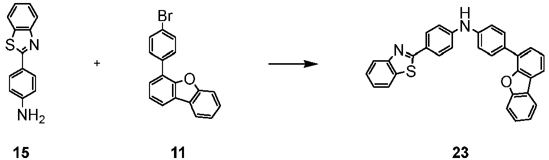

A mixture of 20.0 g (88.4 mmol) of Intermediate **15,** 28.6 g (88.4 mmol) of Intermediate **11,** 0.4 g (1.8 mmol) of Pd(OAc)₂, 17.0 g (176.8 mmol) of sodium *tert-*butoxide, 1.4 g (3.5 mmol) of tri-*tert*-butylphosphine (50 wt% toluene solution), and 200 mL of toluene was stirred under reflux for 12 h. The reaction mixture was cooled to room temperature, stirred for 1 h, added with 400 mL of distilled water, and stirred for additional 30 min. The resulting mixture was filtered under reduced pressure, washed with toluene and methanol, and dried under reduced pressure. The dried precipitate was dissolved by heating in chloroform, filtered through a pad of celite, and washed with chloroform. The filtrate was concentrated under reduced pressure and the residue was dissolved by heating in toluene. The mixed solution was slowly cooled to room temperature and allowed to stand for 3 h. The resulting precipitate was collected by filtration, washed with toluene and methanol, and dried under reduced pressure to afford 22.4 g (yield 54.1%) of Intermediate **23** as a pale brown solid.

### Intermediate Synthesis Example 14: Synthesis of Intermediate 24

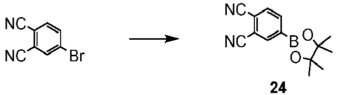

15.0 g (72.5 mmol) of 4-bromophthalonitrile, 22.1 g (86.9 mmol) of PIN₂B₂, 5.9 g (7.2 mmol) of Pd(dppf)Cl₂-DCM, 35.6 g (362 mmol) of KOAc, and 300 mLof 1,4-dioxane were stirred under reflux in a 1 L one-neck flask for one day. The reaction mixture was cooled to room temperature and filtered through celite to remove impurities. After complete removal of the solvent, the resulting mixture was purified by silica gel column chromatography (DCM:HEX) and filtered with hexane to afford 13.7 g (yield 74.6%) of Intermediate **24** as a white solid.

### Intermediate Synthesis Example 15: Synthesis of Intermediate 25

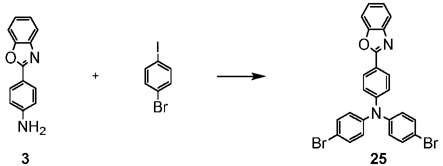

3.5 g (16.5 mmol) of Intermediate **3,** 14.0 g (49.4 mmol) of 1-bromo-4-iodobenzene, and 1.0 g (1.6 mmol) of Pd(dba)₂, 1.8 g (3.3 mmol) of DPPF, 4.7 g (49.4 mmol) of NaOtBu, and 82 mL of toluene were mixed in a 250 mL one-neck flask. The mixture was stirred under reflux for 17 h. After completion of the reaction, the reaction mixture was cooled to room temperature and filtered through celite. The filtrate was concentrated and purified by silica gel column chromatography (Hex:DCM) to afford 2.5 g (yield 29.1%) of Intermediate **25** as a beige solid.

### Intermediate Synthesis Example 16: Synthesis of Intermediate 26

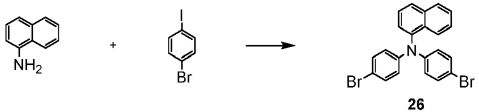

2.0 g (14.0 mmol) of 1-naphthylamine, 8.7 g (30.7 mmol) of 1-bromo-4-iodobenzene, 0.4 g (0.7 mmol) of Pd(dba)₂, 0.8 g (1.4 mmol) of DPPF, 4.0 g (41.9 mmol) of NaOtBu, and 70 mL of toluene were stirred under reflux in a 250 mL one-neck flask for 2 h. The reaction mixture was cooled to room temperature and filtered through celite to remove impurities. After removal of the solvent, the resulting mixture was purified by silica gel column chromatography (EA:HEX) to afford 3.4 g (yield 53.6%) of Intermediate **26** as a white solid.

### Intermediate Synthesis Example 17: Synthesis of Intermediate 27

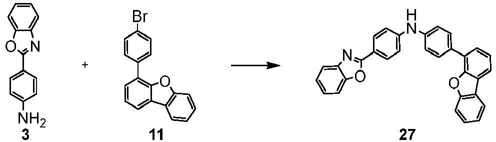

10.0 g (45.6 mmol) of Intermediate 3, 15.4 g (45.6 mmol) of Intermediate **11,** 2.7 g (4.8 mmol) of Pd(dba)₂, 2.3 mL (9.5 mmol) of t-BusP (50 wt% toluene solution), 11.4 g (118.9 mmol) of t-BusONa, and 236 mL of toluene were mixed in a 500 mL one-neck flask. The mixture was heated to reflux for 2 h. After completion of the reaction, the reaction mixture was added with distilled water and extracted with dichloromethane. The organic layer was separated, dried by the addition of Na₂SO₄, and filtered. After removal of the solvent, the concentrate was purified by column chromatography (HEX:EA) and recrystallized from dichloromethane and methanol to afford 10.4 g (yield 48.3%) of Intermediate **27** as a pale yellow solid.

### Intermediate Synthesis Example 18: Synthesis of Intermediate 28

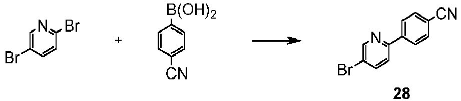

10.0 g (68.1 mmol) of (4-cyanophenyl)boronic acid, 19.4 g (817.0 mmol) of 2,5-dibromopyridine, 3.9 g (3.4 mmol) of Pd(PPh₃)₄, 18.8 g (136.1 mmol) of K₂CO₃, and 280 mL of toluene/ethanol/distilled water (4/2/1) were mixed in a 500 mL one-neck flask. The mixture was heated to reflux for 5 h. After completion of the reaction, the reaction mixture was cooled to room temperature, filtered, washed sequentially with distilled water and methanol, and dried under reduced pressure to afford 8.8 g (yield 49.9%) of Intermediate **28** as an off-white solid.

### Intermediate Synthesis Example 19: Synthesis of Intermediate 30

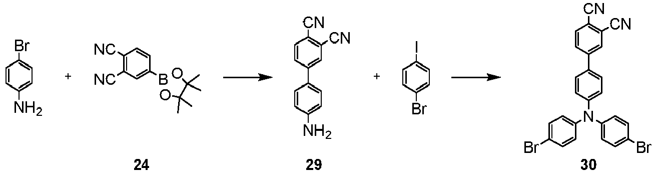

### (Synthesis of Intermediate 29)

17.7 g (69.7 mmol) of Intermediate **24,** 10.0 g (58.1 mmol) of 4-bromoaniline, 2.0 g (1.7 mmol) of Pd(PPh₃)₄, 58 mL (116.2 mmol) of 2 M K₂CO₃, 110 mL of toluene, and 55 mL of ethanol were stirred under reflux in a 500 mL one-neck flask for one day. The mixture was cooled to room temperature and extracted with ethyl acetate. After removal of water and the solvent, the resulting mixture was dissolved in chloroform, purified by silica gel column chromatography (EA:CHCl₃), and filtered with a mixed solution (acetone/hexane) to afford 5.2 g (yield 40.8%) of Intermediate **29** as a yellow solid.

### (Synthesis of Intermediate 30)

2.0 g (14.0 mmol) of Intermediate **29,** 8.7 g (30.7 mmol) of 1-bromo-4-iodobenzene, 0.4 g (0.7 mmol) of Pd(dba)₂, 0.8 g (1.4 mmol) of DPPF, 4.0 g (41.9 mmol) of NaOtBu, and 70 mL of toluene were stirred under reflux in a 250 mL one-neck flask for 2 h. The reaction mixture was cooled to room temperature and filtered through celite to remove impurities. After removal of the solvent, the resulting mixture was purified by silica gel column chromatography (EA:HEX) to afford 3.4 g (yield 53.6%) of Intermediate **26** as a white solid.

### Intermediate Synthesis Example 20: Synthesis of Intermediate 35

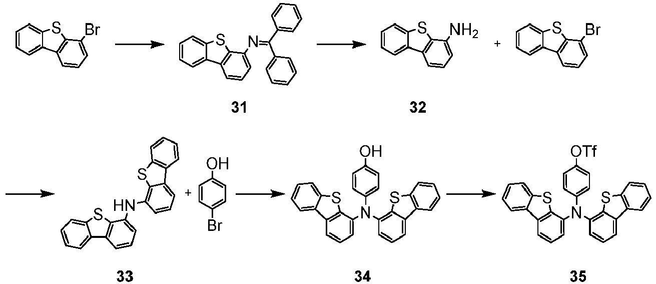

### (Synthesis of Intermediate 31)

20.0 g (76.0 mmol) of 4-bromodibenzo[b,d]thiophene, 15.3 mL (91.2 mmol) of benzophenone imine, 4.4 g (7.6 mmol) of Pd(dba)₂, 9.5 g (15.2 mmol) of BINAP, 74.3 g (228.0 mmol) of Cs₂CO₃, and 380 mL of toluene were mixed in a 1000 mL one-neck flask. The mixture was stirred under reflux for 17 h. After completion of the reaction, the reaction mixture was cooled to room temperature, filtered through celite, and concentrated to afford 49.9 g (yield 100.0%) of Intermediate **31** as a black liquid precipitate. The intermediate was used for the subsequent reaction without further purification.

### (Synthesis of Intermediate 32)

49.9 g (76.0 mmol) of Intermediate **31** was added to and dissolved in 253 mL of tetrahydrofuran (THF) in a 500 mL one-neck flask, and then 101 mL (608.0 mmol) of a 6 N hydrochloric acid solution was slowly added dropwise thereto. The mixture was stirred at room temperature for 17 h. The resulting mixture was neutralized by slow dropwise addition of NaHCOs. After stirring for 30 min, the reaction was terminated. To the reaction mixture were added dichloromethane and distilled water. The organic layer was separated and dried over anhydrous magnesium sulfate. The solvent was removed under reduced pressure. The residue was purified by silica gel column chromatography (Hex:DCM:EA) and recrystallized from methanol and hexane to afford 10.9 g (yield 72.2%) of Intermediate **32** as a yellow solid.

### (Synthesis of Intermediate 33)

9.1 g (45.6 mmol) of Intermediate **32,** 8.0 g (30.4 mmol) of 4-bromodibenzo[b,d]thiophene, 0.3 g (1.5 mmol) of Pd(OAc)₂, 1.3 g (3.0 mmol) of S-Phos, 19.8 g (60.8 mmol) of Cs₂CO₃, and 200 mL of toluene were mixed in a 500 mL one-neck flask. The mixture was heated to 85 °C and stirred for 5 h. After completion of the reaction, the reaction mixture was cooled to room temperature, filtered through a pad of silica gel, and washed with a mixed solution (DCM:EA). The filtrate was distilled under reduced pressure to remove the solvent. The residue was crystallized from a mixed solution (DCM:acetone) to afford 8.2 g (yield 70.6%) of Intermediate **33** as a beige solid.

### (Synthesis of Intermediate 34)

2.5 g (6.4 mmol) of Intermediate **33,** 1.2 g (7.1 mmol) of 4-bromophenol, 0.2 g (0.3 mmol) of Pd(dba)₂, 0.3 mL (0.6 mmol) of 50% P(t-Bu)₃, 1.9 g (19.3 mmol) of NaOtBu, and 25 mL of xylene were stirred under reflux for 30 min. The reaction mixture was cooled to room temperature and filtered through celite to remove impurities. After complete removal of the solvent, the resulting mixture was purified by silica gel column chromatography (DCM:HEX) to afford 2.6 g (yield 86.1%) of Intermediate **34** as a green solid.

### (Synthesis of Intermediate 35)

2.6 g (15.5 mmol) of Intermediate **34** and 1.4 mL (16.5 mmol) of pyridine were added to and stirred in 40 mL of dichloromethane in a 250 mL one-neck flask, and then 1.4 mL (8.3 mmol) of trifluoromethanesulfonic anhydride was slowly added dropwise thereto at 0 °C. The mixture was stirred at room temperature for 2 h. After confirming the reaction, distilled water was added to quench the reaction. The reaction mixture was extracted with dichloromethane. After removal of water and the solvent, the organic layer was purified by silica gel column chromatography (DCM:HEX) to afford 2.9 g (yield 88.3%) of Intermediate **35** as a white solid.

### Intermediate Synthesis Example 21: Synthesis of Intermediate 36

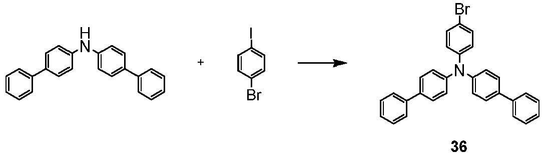

5.0 g (15.6 mmol) of di([1,1'-biphenyl]-4-yl)amine, 6.6 g (23.3 mmol) of 1-bromo-4-iodobenzene, 3.0 g (31.1 mmol) of NaOtBu, and 150 mL of xylene were added together and stirred in a 250 mL one-neck flask, and then 0.3 g (0.5 mmol) of Pd(dba)₂ and 0.6 g (1.6 mmol) of P(t-Bu)₃ were added thereto. The mixture was heated to reflux with stirring for 4 h. After completion of the reaction, the reaction mixture was cooled to room temperature and dichloromethane and distilled water were added thereto. The organic layer was separated, dried over anhydrous sodium sulfate, and distilled under reduced pressure to remove the solvent, purified by silica gel column chromatography (Hex:DCM:EA), and crystallized from methanol to afford 5.3 g (yield 71.5%) of Intermediate **36** as a yellow solid.

### Intermediate Synthesis Example 22: Synthesis of Intermediate 37

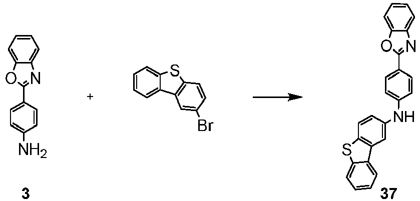

5.0 g (23.8 mmol) of Intermediate **3,** 6.3 g (23.8 mmol) of 2-bromodibenzo[b,d]thiophene, 0.4 g (0.7 mmol) of Pd(dba)₂, 0.6 g (1.4 mmol) of S-Phos, 4.6 g (47.6 mmol) of NaOtBu, and 100 mL of xylene were mixed in a 250 mL one-neck flask. The mixture was heated to 130 °C and stirred for 40 min. After completion of the reaction, the reaction mixture was cooled to room temperature to precipitate a solid. The solid was washed and filtered with distilled water, toluene, and hexane. The resulting solid was dissolved by heating in dichlorobenzene, filtered through celite, and washed with acetone to afford 10.3 g (yield 51.2%) of Intermediate **37** as a beige solid.

### Intermediate Synthesis Example 23: Synthesis of Intermediate 39

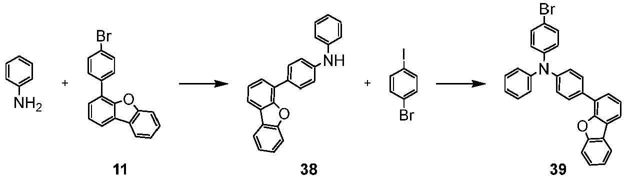

### (Synthesis of Intermediate 38)

2.0 g (21.5 mmol) of aniline, 6.9 g (21.5 mmol) of Intermediate **11,** 4.1 g (43.0 mmol) of NaOtBu, and 100 mL of xylene were added together and stirred in a 250 mL one-neck flask, and then 0.4 g (0.6 mmol) of Pd(dba)₂ and 0.9 g (2.2 mmol) of P(t-Bu)₃ were added thereto. The mixture was heated to reflux with stirring for 4 h. After completion of the reaction, the reaction mixture was cooled to room temperature and the solvent was removed. The resulting mixture was passed through a pad of celite with chloroform, distilled under reduced pressure to remove the solvent, and purified by column chromatography (Hex:CHCl₃) to afford 4.9 g (yield 68.0%) of Intermediate **38** as a slightly red solid.

### (Synthesis of Intermediate 39)

A mixture of 4.9 g (14.6 mmol) of Intermediate **38,** 5.0 g (17.5 mmol) of 1-bromo-4-iodobenzene, 0.3 g (0.4 mmol) of Pd(dba)₂, 2.8 g (29.2 mmol) of sodium *tert-*butoxide, 0.3 g (1.5 mmol) of tri-*tert*-butylphosphine (50 wt% toluene solution), and 50 mL of xylene were stirred under reflux for 12 h. The reaction mixture was cooled to room temperature and methanol was added thereto to precipitate a semi-solid. The precipitate was separated and purified by column chromatography to afford 4.2 g (yield 58.6%) of Intermediate **39** as a beige solid.

### Intermediate Synthesis Example 24: Synthesis of Intermediate 41

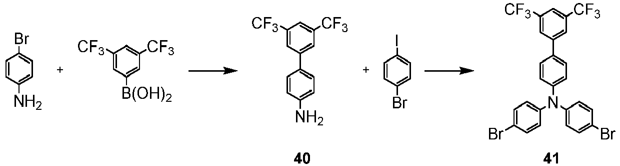

### (Synthesis of Intermediate 40)

18.0 g (69.8 mmol) of (3,5-bis(trifluoromethyl)phenyl)boronic acid, 10.0 g (58.1 mmol) of 4-bromoaniline, 2.0 g (1.7 mmol) of Pd(PPh₃)₄, 58 mL (116.2 mmol) of 2 M K₂CO₃, 110 mL of toluene, and 55 mL of ethanol were stirred under reflux in a 500 mL one-neck flask for one day. The mixture was cooled to room temperature and extracted with ethyl acetate. After removal of water and the solvent, the resulting mixture was dissolved in chloroform and purified by silica gel column chromatography (EA:CHCl₃). The obtained solid was filtered with a mixed solution (acetone/hexane) to afford 10.1 g (yield 56.9%) of Intermediate **40** as a yellow solid.

### (Synthesis of Intermediate 41)

4.0 g (13.1 mmol) of Intermediate **40,** 7.4 g (26.2 mmol) of 1-bromo-4-iodobenzene, 0.4 g (0.7 mmol) of Pd(dba)₂, 0.8 g (1.4 mmol) of DPPF, 4.0 g (39.3 mmol) of NaOtBu, and 70 mL of toluene were stirred under reflux in a 250 mL one-neck flask for 2 h. The mixture was cooled to room temperature and filtered through celite to remove impurities. After removal of the solvent, the resulting mixture was purified by silica gel column chromatography (EA:HEX) to afford 3.8 g (yield 47.1%) of Intermediate **41** as a white solid.

### Intermediate Synthesis Example 25: Synthesis of Intermediate 42

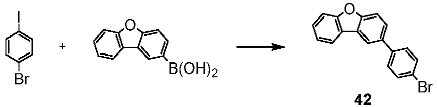

13.6 g (48.2 mmol) of 1-bromo-4-iodobenzene, 10.0 g (43.8 mmol) of dibenzo[b,d]furan-2-ylboronic acid, 1.5 g (1.3 mmol) of Pd(PPh₃)₄, and 150 mL of toluene were added together and stirred in a 500 mL one-neck flask, and then 70 mL of ethanol, 9.1 g (65.8 mmol) of K₂CO₃, and 70 mL of water were added thereto. The mixture was heated to reflux with stirring for 8 h. After completion of the reaction, the reaction mixture was cooled to room temperature, evaporated to remove the solvent, added with water, and extracted with dichloromethane. The organic phase was separated, dried over anhydrous MgSO₄, and purified by column chromatography (DCM) to afford 9.3 g (yield 62.6%) of Intermediate **42** as a slightly yellow solid.

### Intermediate Synthesis Example 26: Synthesis of Intermediate 44

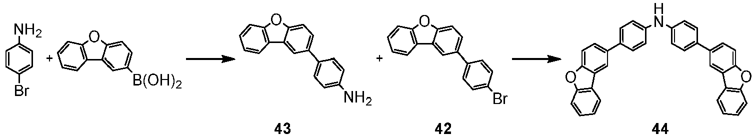

### (Synthesis of Intermediate 43)

5.0 g (29.1 mmol) of 4-bromoaniline, 7.4 g (34.9 mmol) of dibenzo[b,d]furan-2-ylboronic acid, 1.0 g (0.9 mmol) of Pd(PPh₃)₄, 29 mL (58.1 mmol) of 2 M Cs₂CO₃, 60 mL of toluene, and 30 mL of ethanol were stirred under reflux in a 250 mL one-neck flask for one day. The mixture was cooled to room temperature and extracted with ethyl acetate. After removal of water and the solvent, the resulting mixture was dissolved in chloroform and purified by silica gel column chromatography (EA:CHCl₃). The obtained solid was filtered with a mixed solution (acetone/hexane) to afford 4.1 g (yield 54.4%) of Intermediate **43** as a yellow solid.

### (Synthesis of Intermediate 44)

A mixture of 4.1 g (15.8 mmol) of Intermediate **43,** 5.1 g (15.8 mmol) of Intermediate **42,** 0.3 g (0.5 mmol) of Pd(dba)₂, 3.0 g (31.6 mmol) of sodium *tert*-butoxide, 0.6 g (1.6 mmol) of tri-*tert*-butylphosphine (50 wt% toluene solution), and 50 mL of xylene was stirred under reflux for 12 h. The reaction mixture was cooled to room temperature and methanol was added thereto to precipitate a semi-solid. The precipitate was separated and purified by column chromatography to afford 5.8 g (yield 73.1%) of Intermediate **44** as a beige solid.

### Intermediate Synthesis Example 27: Synthesis of Intermediate 45

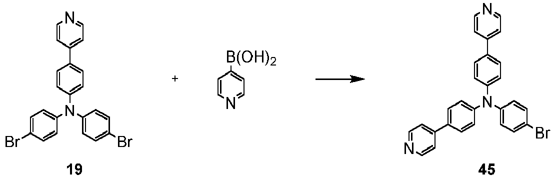

5.2 g (10.8 mmol) of Intermediate **19,** 1.3 g (10.8 mmol) of pyridin-4-ylboronic acid, 0.6 g (0.5 mmol) of Pd(PPh₃)₄, 11 mL (21.6 mmol) of 2 M K₂CO₃, 54 mL of toluene, and 27 mL of ethanol were mixed in a 250 mL one-neck flask. The mixture was stirred under reflux for 2 h. After completion of the reaction, the reaction mixture was cooled to room temperature and dichloromethane and distilled water were added thereto. The organic layer was extracted, dried over anhydrous magnesium sulfate, and filtered through celite. After removal of the solvent under reduced pressure, the resulting mixture was purified by silica gel column chromatography (DCM:EA) to afford 2.3 g (yield 45.2%) of Intermediate **45** as a brown solid.

### Intermediate Synthesis Example 28: Synthesis of Intermediate 46

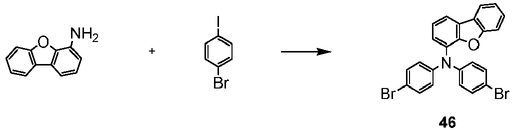

2.6 g (14.2 mmol) of dibenzo[b,d]furan-4-amine, 8.8 g (31.2 mmol) of 1-bromo-4-iodobenzene, 0.4 g (0.7 mmol) of Pd(dba)₂, 0.8 g (1.4 mmol) of DPPF, 4.1 g (42.6 mmol) of NaOtBu, and 60 mL of toluene were stirred under reflux a 250 mL one-neck flask. The reaction mixture was cooled to room temperature and filtered through celite to remove impurities. After removal of the solvent, the resulting mixture was purified by silica gel column chromatography (DCM:HEX) and filtered with hexane to afford 3.7 g (yield 53.5%) of Intermediate **46** as a white solid.

The synthesized intermediates were used to synthesize various tertiary amine derivatives as follows.

### Preparative Example 1: Synthesis of Compound 2-1 (LT19-30-183)

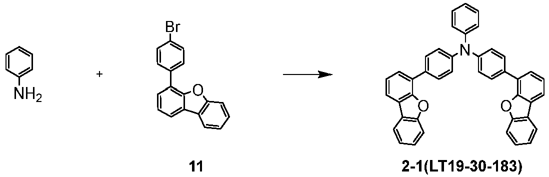

A mixture of 1.0 g (10.7 mmol) of aniline, 7.3 g (22.5 mmol) of Intermediate **11,** 0.6 g (1.1 mmol) of Pd(dba)₂, 5.2 g (53.6 mmol) of sodium *tert*-butoxide, 0.9 g (2.12 mmol) of tri-*tert*-butylphosphine (50 wt% toluene solution), and 50 mL of xylene was stirred under reflux in a 500 mL one-neck flask for 3 h. After completion of the reaction, the reaction mixture was cooled to room temperature and water was added thereto. The resulting mixture was extracted with CHCl₃. The organic phase was dried over anhydrous MgSO₄ and the solvent was removed. The residue was purified by silica gel column chromatography (DCM:HEX) and crystallized from a mixed solution (DCM:EA) to give 5.6 g (yield 90.2%) of Compound **2-1** (LT19-30-183) as a pale yellow solid.

### Preparative Example 2: Synthesis of Compound 2-18 (LT20-30-338)

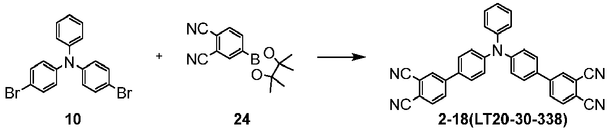

4.1 g (10.1 mmol) of Intermediate **10,** 5.7 g (22.3 mmol) of Intermediate **24,** 0.6 g (1.0 mmol) of Pd(dba)₂, 1.0 g (2.0 mmol) of XPhos, 25 mL (50.7 mmol) of 2 M K₃PO₄, and 50 mL of 1,4-dioxane were stirred at 85 °C in a 250 mL one-neck flask for 6 h. The reaction mixture was cooled to room temperature and extracted with dichloromethane. After removal of water and the solvent, the resulting mixture was purified by silica gel column chromatography (EA:CHCl₃) and filtered with a mixed solution (dichloromethane/acetone) to give 2.5 g (yield 49.3%) of Compound **2-18** (LT20-30-338) as an orange solid.

### Preparative Example 3: Synthesis of Compound 2-25 (LT19-30-187)

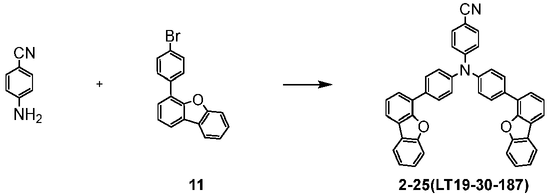

A mixture of 1.0 g (8.5 mmol) of 4-aminobenznitrile, 5.7 g (17.7 mmol) of Intermediate **11, 0.5** g (0.8 mmol) of Pd(dba)₂, 4.1 g (42.3 mmol) of sodium *tert*-butoxide, 0.7 g (1.6 mmol) of tri-*tert*-butylphosphine (50 wt% toluene solution), and 50 mL of xylene was stirred under reflux in a 500 mL one-neck flask for 3 h. After completion of the reaction, the reaction mixture was cooled to room temperature, added with water, and extracted with CHCl₃. The organic phase was separated and dried over anhydrous MgSO₄. After removal of the solvent, the residue was purified by silica gel column chromatography (DCM:HEX) and crystallized from a mixed solution (acetone:methanol) to give 4.3 g (yield 84.6%) of Compound **2-25** (LT19-30-187) as a pale yellow solid.

### Preparative Example 4: Synthesis of Compound 2-29 (LT20-30-182)

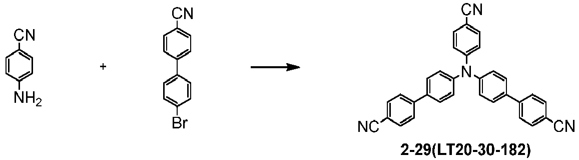

1.5 g (12.7 mmol) of 4-aminobenzonitrile, 13.1 g (50.8 mmol) of 4'-bromo-(1,1'-biphenyl)-4-carbonitrile, 1.5 g (2.5 mmol) of Pd(dba)₂, 2.4 mL (5.1 mmol) of P(t-Bu)₃, 4.9 g (50.8 mmol) of NaOtBu, and 63 mL of xylene were mixed in a 250 mL one-neck flask. The mixture was stirred under reflux for 17 h. After completion of the reaction, the reaction mixture was cooled to room temperature, filtered through celite, purified by silica gel column chromatography (HEX:DCM), and recrystallized from a mixed solution (acetone/MeOH) to give 1.7 g (yield 29.5%) of Compound **2-29** (LT20-30-182) as a beige solid.

### Preparative Example 5: Synthesis of Compound 2-32 (LT19-30-048)

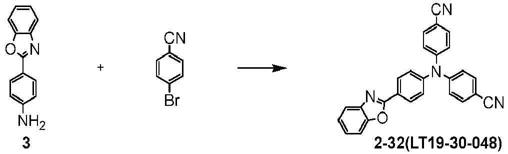

3.5 g (16.7 mmol) of Intermediate **3, 6.7** g (36.6 mmol) of 4-bromobenzonitrile, 6.4 g (66.6 mmol) of NaOtBu, and 150 mL of xylene were added together and stirred in a 250 mL one-neck flask, and then 0.6 g (1.0 mmol) of Pd(dba)₂ and 0.3 mL (1.3 mmol) of P(t-Bu)₃ were added thereto. The mixture was heated to reflux with stirring for 2-3 days. After completion of the reaction, the reaction mixture was cooled to room temperature, evaporated to remove the solvent, passed through a pad of celite with CHCl₃, distilled under reduced pressure to remove the solvent, and purified by column chromatography (CHCl₃:HEX) to give 2.4 g (yield 35.0%) of Compound **2-32** (LT19-30-048) as a slightly yellow solid.

### Preparative Example 6: Synthesis of Compound 2-35 (LT19-30-286)

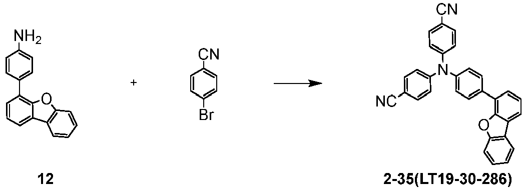

A mixture of 4.0 g (15.4 mmol) of Intermediate **12,** 8.4 g (46.3 mmol) of 4-bromobenzonitrile, 1.8 g (3.1 mmol) of Pd(dba)₂, 3.0 mL (6.2 mmol) of t-Bu₃P (50 wt% toluene solution), 5.9 g (61.7 mmol) of t-BuONa, and 154 mL of toluene was stirred under reflux in a 500 mL one-neck flask for 16 h. The reaction mixture was cooled to room temperature and water was added thereto. The organic layer was separated, dried over anhydrous sodium sulfate, filtered, and concentrated. The concentrate was purified by column chromatography (HEX:EA) and recrystallized from EA to give 1.3 g (yield 17.6%) of Compound **2-35** (LT19-30-286) as an ivory solid.

### Preparative Example 7: Synthesis of Compound 2-49 (LT19-30-275)

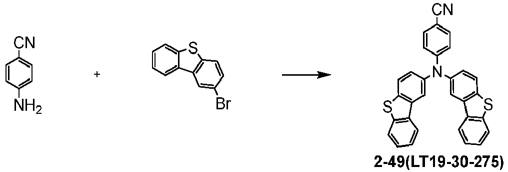

A mixture of 2.0 g (16.9 mmol) of 4-aminobenzonitrile, 9.4 g (35.5 mmol) of 2-bromodibenzo[b,d]thiophene, 1.9 g (3.4 mmol) of Pd(dba)₂, 8.1 g (84.6 mmol) of sodium *tert*-butoxide, 1.4 g (3.3 mmol) of tri-*tert*-butylphosphine (50 wt% toluene solution), and 100 mL of xylene was allowed to react in a 500 mL one-neck flask at 110 °C for 8 h. After completion of the reaction, the reaction mixture was cooled to room temperature, added with water, and extracted with CHCl₃. The organic phase was separated and dried over anhydrous MgSO₄. After removal of the solvent, the resulting mixture was purified by silica gel column chromatography (DCM:HEX) and recrystallized from toluene to give 1.7 g (yield 20.5%) of Compound **2-49** (LT19-30-275) as a white solid.

### Preparative Example 8: Synthesis of Compound 2-50 (LT19-30-278)

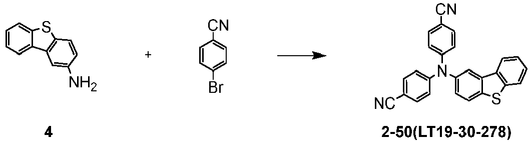

A mixture of 2.5 g (12.5 mmol) of Intermediate **4,** 4.8 g (26.3 mmol) of 4-bromobenzonitrile, 1.43 g (2.5 mmol) of Pd(dba)₂, 6.0 g (62.7 mmol) of sodium *tert-*butoxide, 1.0 g (2.48 mmol) of tri-*tert*-butylphosphine (50 wt% toluene solution), and 100 mL of xylene was allowed to react in a 500 mL one-neck flask at 110 °C for 8 h. After the completion of the reaction was confirmed, the reaction mixture was cooled to room temperature, added with water, and extracted with CHCl₃. The organic phase was separated, dried over anhydrous MgSO₄. After removal of the solvent, the resulting mixture was purified by silica gel column chromatography (DCM:HEX) and crystallized from a mixed solution (acetone/methanol) to give 1.4 g (yield 27.2%) of Compound **2-50** (LT19-30-278) as a white solid.

### Preparative Example 9: Synthesis of Compound 2-51 (LT19-30-608)

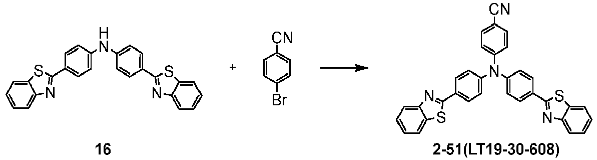

4.0 g (9.2 mmol) of Intermediate **16,** 2.5 g (13.8 mmol) of 4-bromobenzonitrile, 1.8 g (18.4 mmol) of NaOtBu, and 150 mL of xylene were added together and stirred in a 250 mL one-neck flask, and then 0.3 g (0.5 mmol) of Pd(dba)₂ and 0.2 mL (0.9 mmol) of P(t-Bu)₃ were added thereto. The mixture was heated to reflux with stirring throughout the day. After completion of the reaction, the reaction mixture was cooled to room temperature, evaporated to remove the solvent, passed through a pad of celite with chloroform, distilled under reduced pressure to remove the solvent, and purified by column chromatography (HEX:CHCl₃) to give 2.0 g (yield 40.6%) of Compound **2-51** (LT19-30-608) as a light yellow solid.

### Preparative Example 10: Synthesis of Compound 2-70 (LT20-30-272)

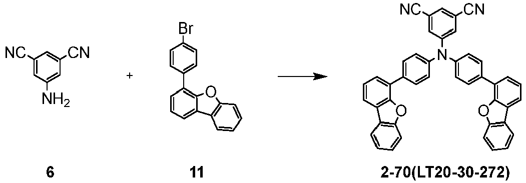

1.3 g (9.1 mmol) of Intermediate 6, 6.2 g (19.1 mmol) of Intermediate **11,** 0.5 g (0.9 mmol) of Pd(dba)₂, 0.8 mL (1.8 mmol) of P(t-Bu)₃, 2.6 g (27.2 mmol) of NaOtBu, and 36 mL of xylene were mixed in a 100 mL one-neck flask. The mixture was heated to 130 °C and stirred for 30 min. After completion of the reaction, the reaction mixture was cooled to room temperature and filtered through celite. The filtrate was concentrated under reduced pressure, purified by silica gel column chromatography (HEX:DCM), and recrystallized from dichloromethane and hexane to give 1.1 g (yield 19.3%) of Compound **2-70** (LT20-30-272) as a pale yellow solid.

### Preparative Example 11: Synthesis of Compound 2-74 (LT20-30-255)

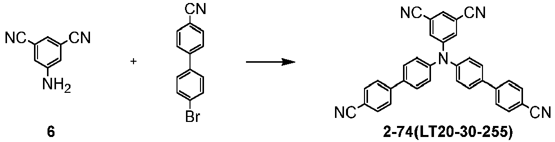

1.5 g (10.5 mmol) of Intermediate **6,** 6.0 g (23.1 mmol) of 4'-bromo-(1,1'-biphenyl)-4-carbonitrile, 10.2 g (31.4 mmol) of Cs₂CO₃, and 150 mL of xylene were added together and stirred in a 250 mL one-neck flask, and then 0.4 g (0.6 mmol) of Pd(dba)₂ and 0.2 mL (0.8 mmol) of P(t-Bu)₃ were added thereto. The mixture was heated to reflux with stirring for 4 h. After completion of the reaction, the reaction mixture was cooled to room temperature and the solvent was removed. The resulting mixture was added with methanol, stirred for 30 min, and filtered. To the resulting solid was added acetone. The mixture was stirred for 30 min and filtered to obtain a solid. The solid was dissolved in hot acetone. The solution was passed through a pad of celite to remove the solvent, added with acetone, stirred for 3-4 h, and filtered to obtain a solid. This procedure was repeated twice to give 1.6 g (yield 31.1%) of Compound **2-74** (LT20-30-255) as a yellowish green solid.

### Preparative Example 12: Synthesis of Compound 2-78 (LT20-30-363)

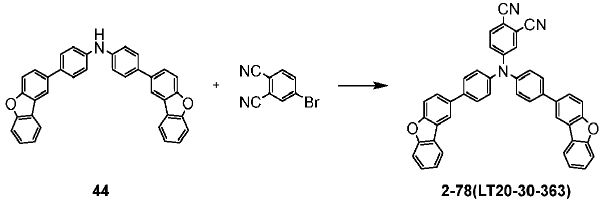

3.7 g (7.4 mmol) of Intermediate **44,** 2.3 g (11.1 mmol) of 4-bromophthalonitrile, 0.2 g (0.4 mmol) of Pd(dba)₂, 0.3 g (0.7 mmol) of S-Phos, 7.4 mL (14.8 mmol) of 2 M K₃PO₄, and 50 mL of xylene were stirred in a 250 mL one-neck flask at 85 °C for 6 h. The reaction mixture was cooled to room temperature and extracted with dichloromethane. After removal of water and the solvent, the resulting mixture was purified by silica gel column chromatography (EA:CHCl₃) and filtered with a mixed solution (dichloromethane/acetone) to give 2.9 g (yield 62.6%) of Compound **2-78** (LT20-30-363) as an orange solid.

### Preparative Example 13: Synthesis of Compound 2-99 (LT20-30-375)

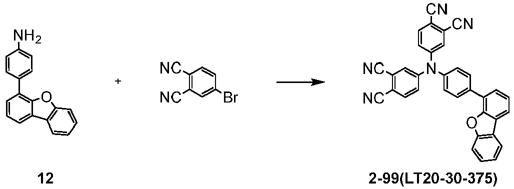

3.0 g (11.6 mmol) of Intermediate **12,** 6.0 g (28.9 mmol) of 4-bromophthalonitrile, 0.7 g (1.2 mmol) of Pd(dba)₂, 0.9 g (1.2 mmol) of P(t-Bu)₃, 3.3 g (34.7 mmol) of NaOtBu, and 30 mL of xylene were mixed in a 100 mL one-neck flask. The mixture was heated to 130 °C and stirred for 30 min. After completion of the reaction, the reaction mixture was cooled to room temperature and filtered through celite. The filtrate was concentrated under reduced pressure, purified by silica gel column chromatography (HEX:DCM), and recrystallized from dichloromethane and hexane to give 1.6 g (yield 27.0%) of Compound **2-99** (LT20-30-375) as a pale yellow solid.

### Preparative Example 14: Synthesis of Compound 2-103 (LT19-30-188)

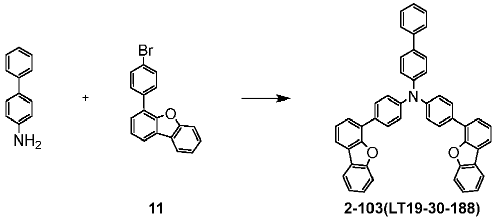

A mixture of 2.0 g (11.8 mmol) of (1,1'-biphenyl)-4-amine, 8.0 g (24.8 mmol) of Intermediate **11,** 0.7 g (1.2 mmol) of Pd(dba)₂, 6.8 g (70.9 mmol) of sodium *tert-*butoxide, 1.0 g (2.3 mmol) of tri-*tert*-butylphosphine (50 wt% toluene solution), and 50 mL of xylene was stirred under reflux in a 500 mL one-neck flask for 3 h. After completion of the reaction, the reaction mixture was cooled to room temperature, added with water, and extracted with CHCl₃. The organic phase was dried over anhydrous MgSO₄ and the solvent was removed. The residue was purified by silica gel column chromatography (DCM:HEX) and crystallized from a mixed solution (acetone/DCM) to give 4.2 g (yield 54.4%) of Compound **2-103** (LT19-30-188) as a pale yellow solid.

### Preparative Example 15: Synthesis of Compound 2-107 (LT20-35-670)

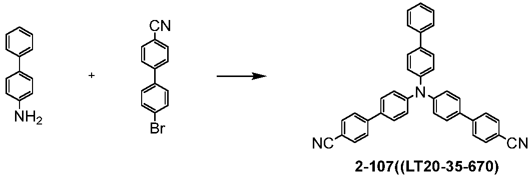

2.0 g (11.8 mmol) of (1,1'-biphenyl)-4-amine, 7.6 g (29.6 mmol) of 4'-bromo-(1,1'-biphenyl)-4-carbonitrile, 0.3 g (0.6 mmol) of Pd(dba)₂, 0.5 g (1.2 mmol) of P(t-Bu)₃, 3.4 g (35.5 mmol) of NaOtBu, and 60 mL of xylene were mixed in a 250 mL one-neck flask. The mixture was stirred under reflux for 17 h. After completion of the reaction, the reaction mixture was cooled to room temperature, filtered through celite, purified by silica gel column chromatography (HEX:DCM), and recrystallized from a mixed solution (acetone/MeOH) to give 2.1 g (yield 33.9%) of Compound **2-107** (LT20-35-670) as a beige solid.

### Preparative Example 16: Synthesis of Compound 2-133 (LT20-30-104)

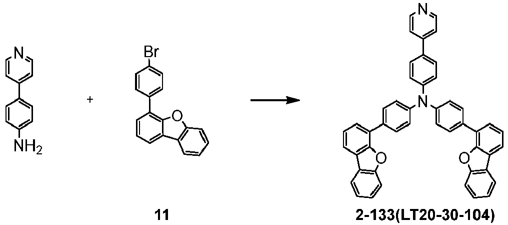

1.3 g (7.6 mmol) of 4-(pyridin-4-yl)aniline, 5.1 g (15.7 mmol) of Intermediate **11, 0.4** g (0.8 mmol) of Pd(dba)₂, 0.7 mL (1.5 mmol) of 50% P(t-Bu)₃, 2.2 g (22.9 mmol) of NaOtBu, and 30 mL of xylene were stirred under reflux in a 250 mL one-neck flask for 20 min. The reaction mixture was cooled to room temperature and filtered through celite to remove impurities. After removal of the solvent, the resulting mixture was dissolved in dichloromethane, purified by silica gel column chromatography (EA:DCM), and filtered with ethanol to give 3.4 g (yield 67.4%) of Compound **2-133** (LT20-30-104) as a yellow solid.

### Preparative Example 17: Synthesis of Compound 2-134 (LT20-30-097)

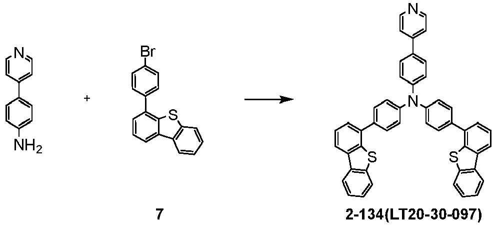

6.6 g (19.4 mmol) of Intermediate **7,** 1.5 g (8.8 mmol) of 4-(pyridin-4-yl)aniline, 2.5 g (26.4 mmol) of NaOtBu, and 150 mL of xylene were added together and stirred in a 250 mL one-neck flask, and then 0.3 g (0.5 mmol) of Pd(dba)₂ and 0.2 mL (0.7 mmol) of P(t-Bu)₃ were added thereto. The mixture was heated to reflux with stirring for 4 h. After completion of the reaction, the reaction mixture was cooled to room temperature, evaporated to remove the solvent, passed through a pad of celite with chloroform, distilled under reduced pressure to remove the solvent, and purified by column chromatography (HEX:EA) to give 2.6 g (yield 43.1%) of Compound **2-134** (LT20-30-097) as a slightly yellow solid.

### Preparative Example 18: Synthesis of Compound 2-146 (LT20-30-196)

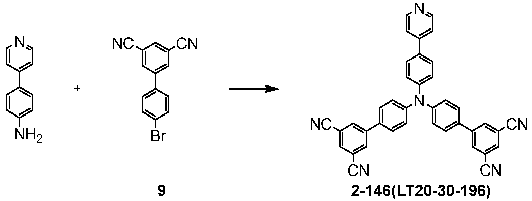

1.6 g (9.4 mmol) of 4-(pyridin-4-yl)aniline, 5.6 g (19.7 mmol) of Intermediate **9,** 2.7 g (28.2 mmol) of NaOtBu, and 150 mL of xylene were added together and stirred in a 250 mL one-neck flask, and then 0.3 g (0.6 mmol) of Pd(dba)₂ and 0.2 mL (0.8 mmol) of P(t-Bu)₃ were added thereto. The mixture was heated to reflux with stirring for 4 h. After completion of the reaction, the reaction mixture was cooled to room temperature, evaporated to remove the solvent, passed through a pad of celite with chloroform, distilled under reduced pressure to remove the solvent, and purified by column chromatography (Hex:CHCl₃) to give 1.7 g (yield 31.5%) of Compound **2-146** (LT20-30-196) as a light yellow solid.

### Preparative Example 19: Synthesis of Compound 2-147 (LT20-30-197)

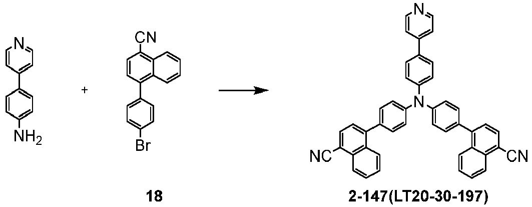

1.4 g (8.2 mmol) of 4-(pyridin-4-yl)aniline, 5.2 g (16.9 mmol) of Intermediate **18,** 0.5 g (0.8 mmol) of Pd(dba)₂, 0.8 mL (1.7 mmol) of 50% P(t-Bu)₃, 2.4 g (24.7 mmol) of NaOtBu, and 35 mL of xylene were stirred under reflux in a 250 mL one-neck flask for 3 h. The reaction mixture was cooled to room temperature, filtered through celite to remove impurities, dissolved in dichloromethane, and purified by silica gel column chromatography (EA:DCM). The resulting solid was dissolved in dichloromethane, solidified with acetone, and filtered to give 1.7 g (yield 33.9%) of Compound **2-147** (LT20-30-197) as a yellow solid.

### Preparative Example 20: Synthesis of Compound 2-150 (LT20-30-284)

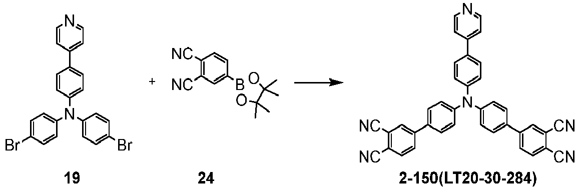

3.8 g (7.9 mmol) of Intermediate **19,** 5.0 g (19.8 mmol) of Intermediate **24**, 0.5 g (0.8 mmol) of Pd(dba)₂, 0.8 g (1.6 mmol) of XPhos, 16 mL (33.5 mmol) of 2 M K₃PO₄, and 40 mL of 1,4-dioxane were stirred in a 250 mL one-neck flask at 85 °C for 3 h. The reaction mixture was cooled to room temperature. The obtained solid was collected by filtration, washed with ethanol, boiled in chloroform, and purified by silica gel column chromatography (EA:CHCl₃). The resulting solid was boiled in chloroform, cooled, and filtered to give 3.2 g (yield 70.1%) of Compound **2-150** (LT20-30-284) as a yellow solid.

### Preparative Example 21: Synthesis of Compound 2-159 (LT20-30-112)

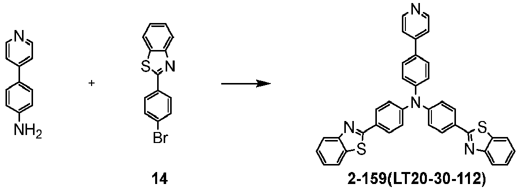

1.5 g (8.8 mmol) of 4-(pyridin-4-yl)aniline, 5.2 g (18.1 mmol) of Intermediate **14, 0.5** g (0.9 mmol) of Pd(dba)₂, 0.9 mL (1.8 mmol) of 50% P(t-Bu)₃, 2.5 g (26.4 mmol) of NaOtBu, and 35 mL of xylene were stirred under reflux in a 250 mL one-neck flask for 20 min. The reaction mixture was cooled to room temperature, solidified with ethanol, and filtered. The resulting solid was boiled in chloroform, purified by silica gel column chromatography (EA:CHCl₃), and filtered with a mixed solution (dichloromethane/acetone) to give 1.9 g (yield 37.1%) of Compound **2-159** (LT20-30-112) as a yellow solid.

### Preparative Example 22: Synthesis of Compound 2-160 (LT20-30-115)

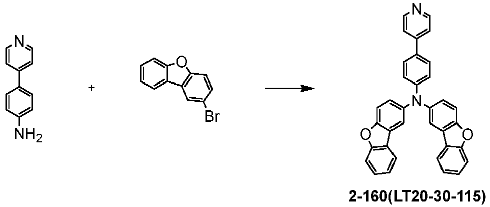

1.7 g (10.0 mmol) of 4-(pyridin-4-yl)aniline, 5.1 g (20.5 mmol) of 2-bromodibenzofuran, 0.6 g (1.0 mmol) of Pd(dba)₂, 1.0 mL (2.0 mmol) of 50% P(t-Bu)₃, 2.9 g (30.0 mmol) of NaOtBu, and 40 mL of xylene were stirred under reflux in a 250 mL one-neck flask for 20 min. The reaction mixture was cooled to room temperature and filtered through celite to remove impurities. After removal of the solvent, the resulting mixture was dissolved in chloroform, purified by silica gel column chromatography (EA:CHCl₃), and filtered with a mixed solution (dichloromethane/hexane) to give 4.3 g (yield 86.1%) of Compound **2-160** (LT20-30-115) as a brown solid.

### Preparative Example 23: Synthesis of Compound 2-161 (LT20-30-120)

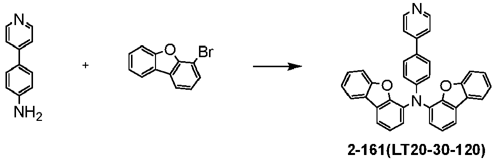

1.7 g (10.0 mmol) of 4-(pyridin-4-yl)aniline, 5.1 g (20.5 mmol) of 4-bromodibenzofuran, 0.6 g (1.0 mmol) of Pd(dba)₂, 1.0 mL (2.0 mmol) of 50% P(t-Bu)₃, 2.9 g (30.0 mmol) of NaOtBu, and 40 mL of xylene were stirred under reflux in a 250 mL one-neck flask for 20 min. The reaction mixture was cooled to room temperature and filtered through celite to remove impurities. After removal of the solvent, the resulting mixture was dissolved in dichloromethane, purified by silica gel column chromatography (EA:DCM), and filtered with a mixed solution (dichloromethane/hexane) to give 1.4 g (yield 28.5%) of Compound **2-161** (LT20-30-120) as a white solid.

### Preparative Example 24: Synthesis of Compound 2-162 (LT20-30-118)

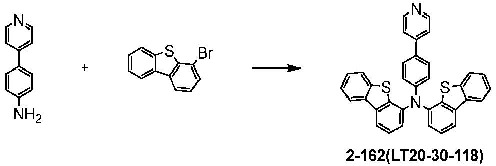

2.2 g (12.9 mmol) of 4-(pyridin-4-yl)aniline, 7.1 g (27.1 mmol) of 4-bromodibenzo[b,d]thiophene, 3.7 g (38.8 mmol) of NaOtBu, and 150 mL of xylene were added together and stirred in a 250 mL one-neck flask, and then 0.4 g (0.8 mmol) of Pd(dba)₂ and 0.3 mL (1.0 mmol) of P(t-Bu)₃ were added thereto. The mixture was heated to reflux with stirring for 4 h. After completion of the reaction, the reaction mixture was cooled to room temperature, evaporated to remove the solvent, passed through a pad of celite with chloroform, distilled under reduced pressure to remove the solvent, and purified by column chromatography (HEX:EA) to give 3.0 g (yield 43.4%) of Compound **2-162** (LT20-30-118) as a slightly yellow solid.

### Preparative Example 25: Synthesis of Compound 2-169 (LT19-30-491)

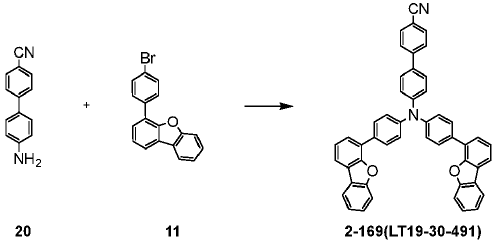

A mixture of 1.5 g (5.8 mmol) of Intermediate 20, 3.9 g (12.2 mmol) of Intermediate **11,** 0.3 g (0.6 mmol) of Pd(dba)₂, 3.3 g (34.8 mmol) of NaOtBu, 0.9 g (2.3 mmol) of SPhos, and 80 mL of toluene was stirred under reflux in a 250 mL one-neck flask for 16 h. After completion of the reaction, the reaction mixture was cooled to room temperature, added with methanol, filtered, purified by silica gel column chromatography (DCM), and solidified with EA to give 0.9 g (yield 22.8%) of Compound **2-169** (LT19-30-491) as a white solid.

### Preparative Example 26: Synthesis of Compound 2-170 (LT19-30-495)

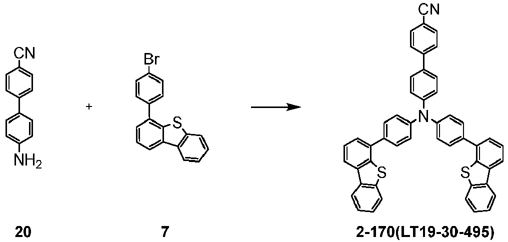

9.6 g (28.3 mmol) of Intermediate **7**, 2.5 g (12.9 mmol) of Intermediate **20,** 4.9 g (51.5 mmol) of NaOtBu, and 150 mL of xylene were added together and stirred in a 250 mL one-neck flask, and then 0.4 g (0.8 mmol) of Pd(dba)₂ and 0.2 mL (1.0 mmol) of P(t-Bu)₃ were added thereto. The mixture was heated to reflux with stirring throughout the day. After completion of the reaction, the reaction mixture was cooled to room temperature, evaporated to remove the solvent, passed through a pad of celite with chloroform, distilled under reduced pressure to remove the solvent, and purified by column chromatography (HEX:CHCl₃) to give 2.7 g (yield 29.5%) of Compound **2-170** (LT19-30-495) as a slightly red solid.

### Preparative Example 27: Synthesis of Compound 2-171 (LT20-30-176)

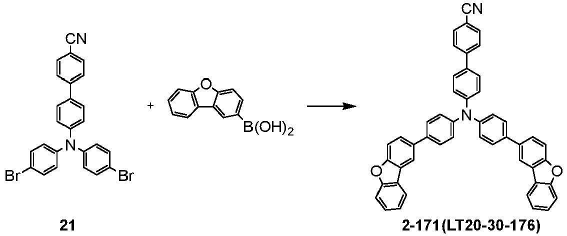

3.7 g (7.3 mmol) of Intermediate **21**, 3.9 g (18.3 mmol) of dibenzofuran-2-boronic acid, 0.4 g (0.7 mmol) of Pd(dba)₂, 0.7 g (1.5 mmol) of XPhos, 15 mL (29.4 mmol) of 2 M K₃PO₄, and 35 mL of 1,4-dioxane were stirred in a 250 mL one-neck flask at 85 °C for 3 h. The reaction mixture was cooled to room temperature. The resulting solid was collected by filtration, washed with ethanol, dissolved in chloroform, purified by silica gel column chromatography (CHCl₃:HEX), and filtered with a mixed solution (dichloromethane/acetone) to give 4.0 g (yield 81.3%) of Compound **2-171** (LT20-30-176) as a yellow solid.

### Preparative Example 28: Synthesis of Compound 2-172 (LT20-30-123)

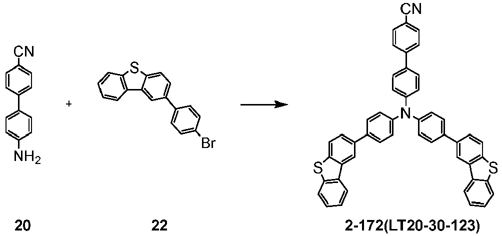

2.0 g (10.3 mmol) of Intermediate **20,** 7.3 g (21.6 mmol) of Intermediate **22,** 3.0 g (30.9 mmol) of NaOtBu, and 150 mL of xylene were added together and stirred in a 250 mL one-neck flask, and then 0.4 g (0.6 mmol) of Pd(dba)₂ and 0.2 mL (0.8 mmol) of P(t-Bu)₃ were added thereto. The mixture was stirred under reflux for 4 h. After completion of the reaction, the reaction mixture was cooled to room temperature, evaporated to remove the solvent, passed through a pad of celite with chloroform, distilled under reduced pressure to remove the solvent, and purified by column chromatography (HEX:CHCl₃) to give 2.5 g (yield 34.1%) of Compound **2-172** (LT20-30-123) as a yellow solid.

### Preparative Example 29: Synthesis of Compound 2-173 (LT19-30-353)

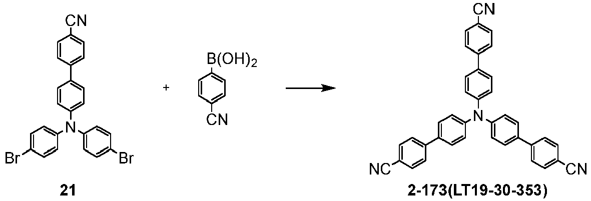

9.7 g (19.2 mmol) of Intermediate **21,** 8.5 g (57.7 mmol) of 4-cyanophenylboronic acid, 1.3 g (1.2 mmol) of Pd(PPh₃)₄, and 80 mL of toluene were added together and stirred in a 500 mL one-neck flask, and then 40 mL of ethanol, 8.0 g (57.7 mmol) of K₂CO₃, and 40 mL of distilled water were added thereto. The mixture was heated to reflux with stirring for 4 h. After completion of the reaction, the reaction mixture was cooled to room temperature, evaporated to remove the solvent, passed through a pad of celite with chloroform, distilled under reduced pressure to remove the solvent, and purified by column chromatography (HEX:CHCl₃) to give 5.8 g (yield 55.1%) of Compound **2-173** (LT19-30-353) as a yellow solid.

### Preparative Example 30: Synthesis of Compound 2-174 (LT19-30-354)

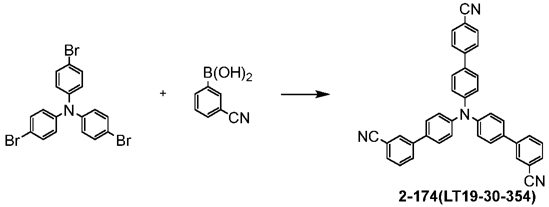

5.0 g (10.4 mmol) of tris(4-bromophenyl)amine, 7.6 g (51.9 mmol) of 3-cyanophenylboronic acid, 1.2 g (1.0 mmol) of Pd(PPh₃)₄, and 80 mL of toluene were added together and stirred in a 250 mL one-neck flask, and then 40 mL of ethanol, 7.2 g (51.9 mmol) of K₂CO₃, and 40 mL of distilled water were added thereto. The mixture was heated to reflux with stirring throughout the day. After completion of the reaction, the reaction mixture was cooled to room temperature, evaporated to remove the solvent, passed through a pad of celite with chloroform, distilled under reduced pressure to remove the solvent, and purified by column chromatography (HEX:EA) to give 3.0 g (yield 52.7%) of Compound **2-174** (LT19-30-354) as a light yellow solid.

### Preparative Example 31: Synthesis of Compound 2-175 (LT19-30-043)

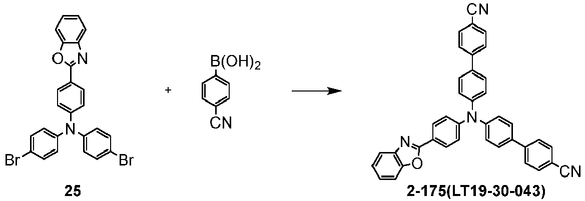

4.5 g (8.7 mmol) of Intermediate **25**, 3.8 g (26.0 mmol) of 4-cyanophenylboronic acid, 0.5 g (0.9 mmol) of Pd(dba)₂, 0.8 g (1.7 mmol) of XPhos, 17 mL (34.6 mmol) of 2 M K₃PO₄, and 35 mL of 1,4-dioxane were stirred in a 250 mL one-neck flask at 85 °C for 3 h. The mixture was cooled to room temperature. The resulting solid was collected by filtration, washed with ethanol, dissolved in chloroform, and purified by silica gel column chromatography (EA:CHCl₃). The obtained solid was boiled in dichloromethane, cooled, and filtered to give 1.8 g (yield 37.5%) of Compound **2-175** (LT19-30-043) as a yellow solid.

### Preparative Example 32: Synthesis of Compound 2-179 (LT20-30-128)

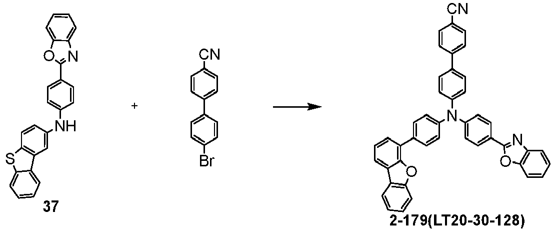

4.0 g (10.2 mmol) of Intermediate **37**, 2.9 g (11.2 mmol) of 4'-bromo-(1,1'-biphenyl)-4-carbonitrile, 0.3 g (0.5 mmol) of Pd(dba)₂, 0.5 mL (1.0 mmol) of P(t-Bu)₃, 2.9 g (30.6 mmol) of NaOtBu, and 41 mL of xylene were mixed in a 250 mL one-neck flask. The mixture was heated to 130 °C and stirred for 30 min. After completion of the reaction, the reaction mixture was cooled to room temperature to precipitate a solid. The solid was collected by filtration, filtered through celite, and purified by silica gel column chromatography (HEX:DCM) to give 1.7 g (yield 29.3%) of Compound **2-179** (LT20-30-128) as a yellow solid.

### Preparative Example 33: Synthesis of Compound 2-181 (LT20-30-116)

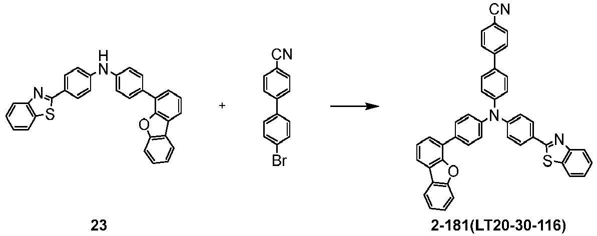

4.0 g (8.5 mmol) of Intermediate **23,** 2.4 g (9.4 mmol) of 4'-bromo-(1.1'-biphenyl)-4-carbonitrile, 1.0 g (10.2 mmol) of NaOtBu, and 150 mL of xylene were added together and stirred in a 250 mL one-neck flask, and then 0.2 g (0.3 mmol) of Pd(dba)₂ and 0.1 mL (0.5 mmol) of P(t-Bu)₃ were added thereto. The mixture was heated to reflux with stirring throughout the day. After completion of the reaction, the reaction mixture was cooled to room temperature and the solvent was removed. The resulting mixture was passed through a pad of celite with chloroform, distilled under reduced pressure to remove the solvent, and purified by column chromatography (HEX:CHCl₃) to give 2.7 g (yield 49.0%) of Compound **2-181** (LT20-30-116) as a light yellow solid.

### Preparative Example 34: Synthesis of Compound 2-182 (LT20-30-063)

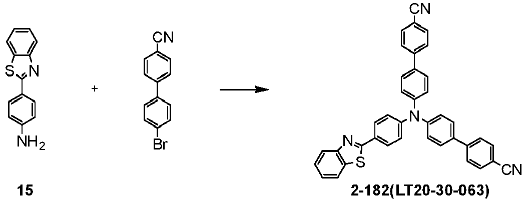

2.0 g (8.8 mmol) of 4-(benzo[d]thiazol-2-yl)aniline, 4.8 g (18.5 mmol) of 4'-bromo-(1,1'-biphenyl)-4-carbonitrile, 0.5 g (0.9 mmol) of Pd(dba)₂, 0.8 mL (1.8 mmol) of P(t-Bu)₃, 2.5 g (26.5 mmol) of NaOtBu, and 35 mL of xylene were mixed in a 250 mL one-neck flask. The mixture was heated to 130 °C and stirred for 1 h and 20 min. After completion of the reaction, the reaction mixture was cooled to room temperature, added with hexane, and stirred for 30 min to precipitate a solid. The solid was collected by filtration, washed with hexane, filtered through celite, purified by silica gel column chromatography (HEX:DCM), and washed with acetone to give 2.1 g (yield 41.2%) of Compound **2-182** (LT20-30-063) as a pale yellow solid.

### Preparative Example 35: Synthesis of Compound 2-188 (LT20-35-675)

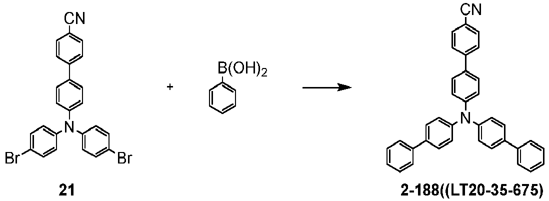

9.7 g (19.2 mmol) of Intermediate **21,** 8.5 g (57.7 mmol) of phenylboronic acid, 1.3 g (1.2 mmol) of Pd(PPh₃)₄, and 80 mL of toluene were added together and stirred in a 500 mL one-neck flask, and then 40 mL of ethanol, 8.0 g (57.7 mmol) of K₂CO₃, and 40 mL of distilled water were added thereto. The mixture was heated to reflux with stirring for 4 h. After completion of the reaction, the reaction mixture was cooled to room temperature, evaporated to remove the solvent, passed through a pad of celite with chloroform, distilled under reduced pressure to remove the solvent, and purified by column chromatography (HEX:CHCl₃) to give 5.8 g (yield 55.1%) of Compound **2-188** (LT20-35-675) as a yellow solid.

### Preparative Example 36: Synthesis of Compound 2-196 (LT19-30-605)

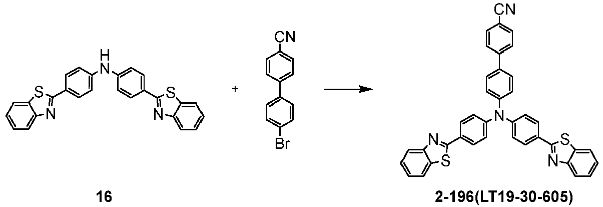

3.6 g (8.3 mmol) of Intermediate **16,** 3.2 g (12.4 mmol) of 4'-bromo-(1.1'-biphenyl)-4-carbonitrile, 1.6 g (16.5 mmol) of NaOtBu, and 150 mL of xylene were added together and stirred in a 250 mL one-neck flask, and then 0.2 g (0.4 mmol) of Pd(dba)₂ and 0.2 mL (0.8 mmol) of P(t-Bu)₃ were added thereto. The mixture was heated to reflux with stirring throughout the day. After completion of the reaction, the reaction mixture was cooled to room temperature and the solvent was removed. The resulting mixture was passed through a pad of celite with chloroform, distilled under reduced pressure to remove the solvent, and purified by column chromatography (HEX:CHCl₃) to give 2.0 g (yield 39.5%) of Compound **2-196** (LT19-30-605) as a light yellow solid.

### Preparative Example 37: Synthesis of Compound 2-197 (LT20-30-133)

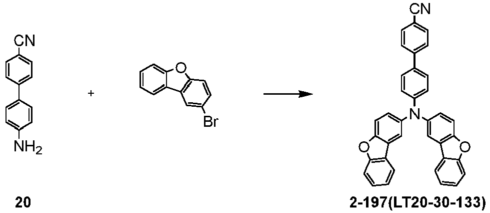

1.9 g (9.8 mmol) of Intermediate **20,** 5.0 g (20.1 mmol) of 2-bromodibenzofuran, 0.6 g (1.0 mmol) of Pd(dba)₂, 0.9 mL (2.0 mmol) of 50% P(t-Bu)₃, 2.8 g (29.3 mmol) of NaOtBu, and 40 mL of xylene were heated to reflux with stirring in a 250 mL one-neck flask for 20 min. The reaction mixture was cooled to room temperature and filtered through celite to remove impurities. After removal of the solvent, the resulting mixture was dissolved in dichloromethane, purified by silica gel column chromatography (DCM:HEX), and filtered with a mixed solution (dichloromethane/acetone) to give 3.6 g (yield 69.8%) of Compound **2-197** (LT20-30-133) as a yellow solid.

### Preparative Example 38: Synthesis of Compound 2-198 (LT20-30-136)

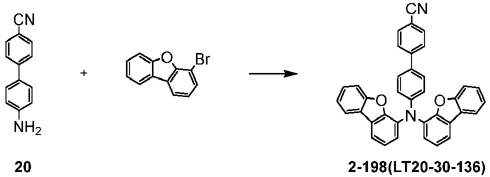

1.9 g (9.8 mmol) of Intermediate **20,** 5.0 g (20.1 mmol) of 4-bromodibenzofuran, 0.6 g (1.0 mmol) of Pd(dba)₂, 1.0 mL (2.0 mmol) of 50% P(t-Bu)₃, 2.8 g (29.3 mmol) of NaOtBu, and 40 mL of xylene were stirred under reflux in a 250 mL one-neck flask for 20 min. The reaction mixture was cooled to room temperature and filtered through celite to remove impurities. After removal of the solvent, the resulting mixture was dissolved in dichloromethane, purified by silica gel column chromatography (DCM:HEX), and filtered with a mixed solution (dichloromethane/hexane) to give 1.6 g (yield 31.1%) of Compound **2-198** (LT20-30-136) as a white solid.

### Preparative Example 39: Synthesis of Compound 2-199 (LT20-30-137)

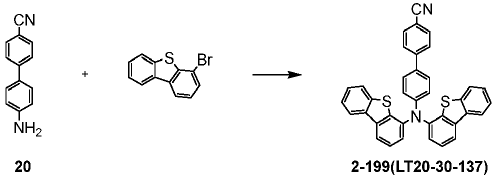

3.0 g (15.4 mmol) of Intermediate **20,** 8.5 g (32.4 mmol) of 4-bromodibenzo[b,d]thiophene, 4.5 g (46.3 mmol) of NaOtBu, and 150 mL of xylene were added together and stirred in a 250 mL one-neck flask, and then 0.5 g (0.9 mmol) of Pd(dba)₂ and 0.3 mL (1.2 mmol) of P(t-Bu)₃ were added thereto. The mixture was heated to reflux with stirring for 4 h. After completion of the reaction, the reaction mixture was cooled to room temperature and the solvent was removed. The resulting mixture was passed through a pad of celite with chloroform, distilled under reduced pressure to remove the solvent, and purified by column chromatography (HEX:CHCl₃) to give 3.0 g (yield 34.8%) of Compound **2-199** (LT20-30-137) as a slightly yellow solid.

### Preparative Example 40: Synthesis of Compound 2-213 (LT20-30-214)

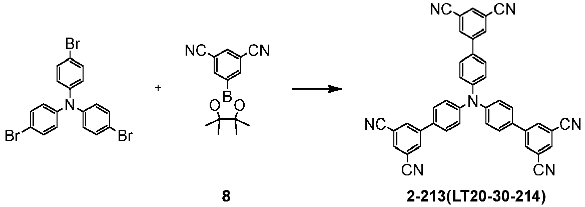

10.5 g (19.7 mmol) of Intermediate **8,** 5.0 g (10.4 mmol) of tris(4-bromophenyl)aniline, 1.2 g (1.0 mmol) of Pd(PPh₃)₄, and 80 mL of toluene were added together and stirred in a 250 mL one-neck flask, and then 40 mL of ethanol, 7.2 g (51.9 mmol) of K₂CO₃, and 40 mL of distilled water were added thereto. The mixture was heated to reflux with stirring throughout the day. After completion of the reaction, the reaction mixture was cooled to room temperature and the solvent was removed. The resulting mixture was added with methanol, stirred for 30 min, and filtered to obtain a solid. The solid was added with acetone, stirred for 30 min, and filtered. The resulting solid was dissolved in hot N-methyl-2-pyrrolidinone. The solution was passed through a pad of celite and acetone was added thereto. The mixture was stirred for 3-4 h and filtered to obtain a solid. This procedure was repeated twice to give 1.5 g (yield 23.2%) of Compound **2-213** (LT20-30-214) as a light yellow solid.

### Preparative Example 41: Synthesis of Compound 2-247 (LT20-30-308)

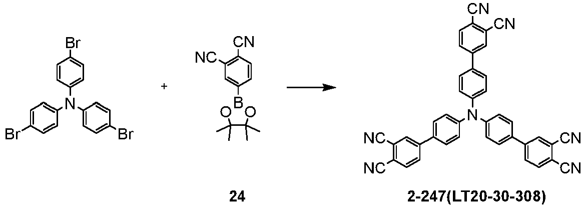

10.5 g (19.7 mmol) of Intermediate **24,** 5.0 g (10.4 mmol) of tris(4-bromophenyl)aniline, 1.2 g (1.0 mmol) of Pd(PPh₃)₄, and 80 mL of toluene were added together and stirred in a 250 mL one-neck flask, and then 40 mL of ethanol, 7.2 g (51.9 mmol) of K₂CO₃, and 40 mL of distilled water were added thereto. The mixture was heated to reflux with stirring throughout the day. After completion of the reaction, the reaction mixture was cooled to room temperature and the solvent was removed. The resulting mixture was added with methanol, stirred for 30 min, and filtered to obtain a solid. The solid was added with acetone, stirred for 30 min, and filtered. The resulting solid was dissolved in hot N-methyl-2-pyrrolidinone. The solution was passed through a pad of celite and methanol was added thereto. The mixture was stirred for 3-4 h and filtered to obtain a solid. This procedure was repeated twice to give 1.6 g (yield 24.7%) of Compound **2-247** (LT20-30-308) as a slightly pink solid.

### Preparative Example 42: Synthesis of Compound 2-248 (LT20-30-369)

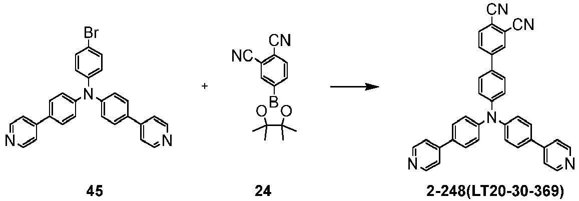

2.2 g (4.6 mmol) of Intermediate **45,** 2.3 g (9.2 mmol) of Intermediate **24,** 0.2 g (0.5 mmol) of Pd(dba)₂, 0.4 g (0.9 mmol) of X-Phos, 2.9 g (13.8 mmol) of K₃PO₄, 18 mL of toluene, and 9 mL of distilled water were mixed in a 250 mL one-neck flask. The mixture was heated to 100 °C and stirred for 3 days. After completion of the reaction, the reaction mixture was cooled to room temperature, added with 100 mL of methanol, stirred for 30 min, filtered, washed with methanol, and filtered through celite. After removal of the solvent under reduced pressure, the resulting mixture was purified by silica gel column chromatography (DCM:EA) to give 1.1 g (yield 47.9%) of Compound **2-248** (LT20-30-369) as a yellow solid.

### Preparative Example 43: Synthesis of Compound 2-249 (LT20-30-330)

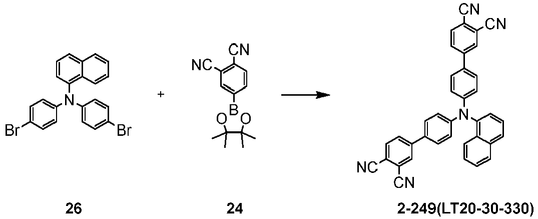

3.4 g (7.5 mmol) of Intermediate **26,** 4.8 g (18.7 mmol) of Intermediate **24,** 0.4 g (0.8 mmol) of Pd(dba)₂, 0.7 g (1.5 mmol) of XPhos, 20 mL (37.4 mmol) of 2 M K₃PO₄, and 40 mL of 1,4-dioxane were stirred in a 250 mL one-neck flask at 85 °C for one day. The mixture was cooled to room temperature. The resulting solid was collected by filtration, washed with ethanol, boiled in chloroform, purified by silica gel column chromatography (EA:CHCl₃), and filtered with dichloromethane to give 1.6 g (yield 39.2%) of Compound **2-249** (LT20-30-330) as a yellow solid.

### Preparative Example 44: Synthesis of Compound 2-251 (LT20-30-396)

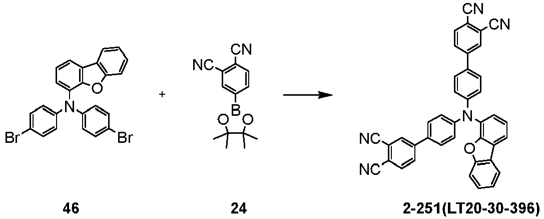

3.7 g (7.6 mmol) of Intermediate **46,** 4.2 g (16.7 mmol) of Intermediate **24,** 0.4 g (0.8 mmol) of Pd(dba)₂, 0.7 g (1.5 mmol) of XPhos, 20 mL (37.9 mmol) of 2 M K₃PO₄, and 40 mL of 1,4-dioxane were heated to reflux with stirring in a 250 mL one-neck flask for one day. The reaction mixture was cooled to room temperature and filtered to remove impurities. The filtrate was extracted with ethyl acetate, and water and the solvent were removed. The resulting mixture was purified by silica gel column chromatography ((DCM:HEX) and filtered with a mixed solution (hexane/dichloromethane) to give 2.9 g (yield 64.0%) of Compound **2-251** (LT20-30-396) as an orange solid.

### Preparative Example 45: Synthesis of Compound 2-257 (LT20-30-349)

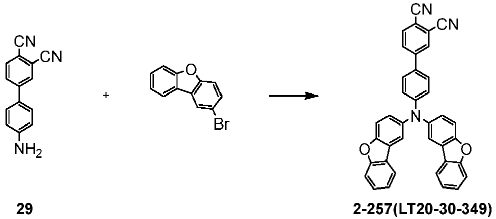

1.9 g (8.7 mmol) of Intermediate **29,** 4.7 g (19.1 mmol) of 2-bromodibenzo[b,d]furan, 2.5 g (26.0 mmol) of NaOtBu, and 150 mL of xylene were added together and stirred in a 250 mL one-neck flask, and then 0.3 g (0.5 mmol) of Pd(dba)₂ and 0.3 mL (1.0 mmol) of P(t-Bu)₃ were added thereto. The mixture was stirred under reflux for 4 h. After completion of the reaction, the reaction mixture was cooled to room temperature and the solvent was removed. The resulting mixture was passed through a pad of celite with chloroform, distilled under reduced pressure to remove the solvent, and purified by column chromatography (HEX:CHCl₃) to give 1.7 g (yield 35.1%) of Compound **2-257** (LT20-30-349) as a slightly red solid.

### Preparative Example 46: Synthesis of Compound 2-259 (LT20-30-347)

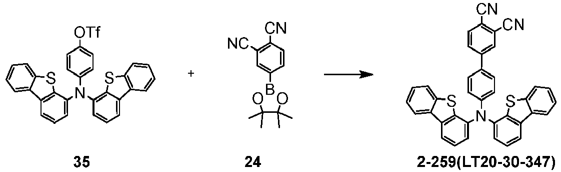

3.0 g (4.9 mmol) of Intermediate **35,** 1.5 g (5.8 mmol) of Intermediate **24**, 0.1 g (0.2 mmol) of Pd(dba)₂, 0.2 g (0.5 mmol) of XPhos, 7 mL (14.6 mmol) of 2 M K₃PO₄, and 25 mL of 1,4-dioxane were stirred in a 250 mL one-neck flask at 80 °C for one day. The mixture was cooled to room temperature and extracted with dichloromethane. After the removal of water and the solvent, the resulting mixture was purified by silica gel column chromatography (DCM:HEX) and filtered with a mixed solution (dichloromethane/hexane) to give 2.5 g (yield 86.7%) of Compound **2-259** (LT20-30-347) as an orange solid.

### Preparative Example 47: Synthesis of Compound 2-260 (LT20-30-370)

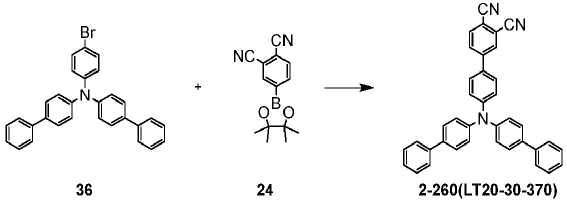

4.0 g (8.4 mmol) of Intermediate **36,** 3.2 g (12.6 mmol) of Intermediate **24, 0.2** g (0.4 mmol) of Pd(dba)₂, 0.4 g (0.8 mmol) of X-Phos, and 126 mL of dioxane were added together and stirred in a 250 mL one-neck flask, and then 5.3 g (25.2 mmol) of K₃PO₄ and 42 mL of distilled water were added thereto. The mixture was heated to reflux with stirring throughout the day. After completion of the reaction, the reaction mixture was cooled to room temperature and the solvent was removed. The resulting mixture was passed through a pad of celite with chloroform, distilled under reduced pressure to remove the solvent, and purified by column chromatography (HEX:CHCl₃) to give 1.5 g (yield 35.1%) of Compound **2-260** (LT20-30-370) as a yellow solid.

### Preparative Example 48: Synthesis of Compound 2-265 (LT19-30-234)

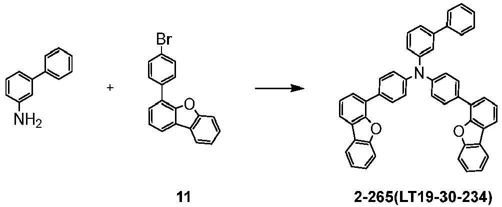

1.7 g (10.1 mmol) of (1,1'-biphenyl)-3-amine, 7.1 g (22.1 mmol) of Intermediate **11,** 2.9 g (30.1 mmol) of NaOtBu, and 150 mL of xylene were added together and stirred in a 250 mL one-neck flask, and then 0.4 g (0.6 mmol) of Pd(dba)₂ and 0.2 mL (0.8 mmol) of P(t-Bu)₃ were added thereto. The mixture was heated to reflux with stirring throughout the day. After completion of the reaction, the reaction mixture was cooled to room temperature and the solvent was removed. The resulting mixture was passed through a pad of celite with CHCl₃, distilled under reduced pressure to remove the solvent, and purified by column chromatography (HEX:EA) to give 3.4 g (yield 51.8%) of Compound **2-265** (LT19-30-234) as a light yellow solid.

### Preparative Example 49: Synthesis of Compound 2-266 (LT19-30-511)

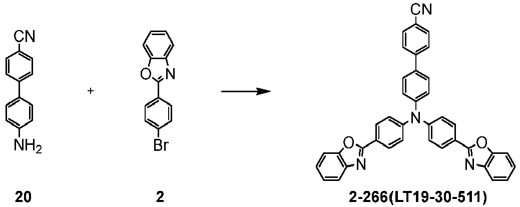

1.6 g (8.3 mmol) of Intermediate **20,** 4.8 g (17.5 mmol) of Intermediate **2,** 0.5 g (0.8 mmol) of Pd(dba)₂, 4.8 g (49.9 mmol) of NaOtBu, 1.4 g (3.3 mmol) of SPhos, and 100 mL of toluene was stirred under reflux in a 250 mL one-neck flask for 16 h. After completion of the reaction, the reaction mixture was cooled to room temperature, filtered, purified by silica gel column chromatography (DCM:EA), and recrystallized from a mixed solution (EA/MeOH) to give 1.1 g (yield 22.7%) of Compound **2-266** (LT19-30-511) as a yellow solid.

### Preparative Example 50: Synthesis of Compound 2-267 (LT20-30-128)

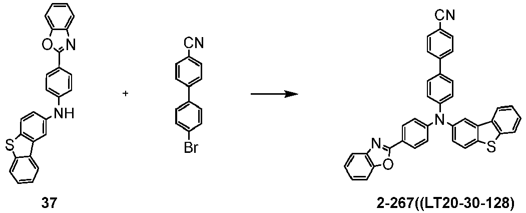

4.0 g (10.2 mmol) of Intermediate **37,** 2.9 g (11.2 mmol) of 4'-bromo-(1,1'-biphenyl)-4-carbonitrile, 0.3 g (0.5 mmol) of Pd(dba)₂, 0.5 mL (1.0 mmol) of P(t-Bu)₃, 2.9 g (30.6 mmol) of NaOtBu, and 41 mL of xylene were mixed in a 250 mL one-neck flask. The mixture was heated to 130 °C and stirred for 30 min. After completion of the reaction, the reaction mixture was cooled to room temperature to precipitate a solid. The solid was collected by filtration, filtered through celite, and purified by silica gel column chromatography (HEX:DCM) to give 1.7 g (yield 29.3%) of Compound **2-267** (LT20-30-128) as a yellow solid.

### Preparative Example 51: Synthesis of Compound 2-268 (LT20-30-107)

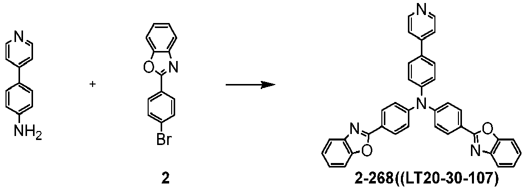

2.0 g (11.7 mmol) of 4-(pyridin-4-yl)aniline, 6.8 g (24.7 mmol) of Intermediate **2,** 0.7 g (1.2 mmol) of Pd(dba)₂, 1.1 mL (2.4 mmol) of P(t-Bu)₃, 3.4 g (35.3 mmol) of NaOtBu, and 47 mL of xylene were mixed in a 250 mL one-neck flask. The mixture was heated to 130 °C and stirred for 30 min. After completion of the reaction, the precipitated solid was collected by filtration, washed with methanol, added to toluene, dissolved by heating, filtered through celite, and recrystallized from toluene to give 1.1 g (yield 17.6%) of Compound **2-268** (LT20-30-107) as a beige solid.

### Preparative Example 52: Synthesis of Compound 2-269 (LT20-30-170)

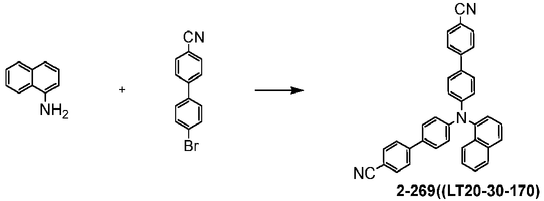

1.5 g (10.5 mmol) of naphthalen-1-amine, 5.7 g (22.0 mmol) of 4'-bromo-(1,1'-biphenyl)-4-carbonitrile, 0.6 g (1.0 mmol) of Pd(dba)₂, 1.0 mL (2.1 mmol) of P(t-Bu)₃, 3.0 g (31.4 mmol) of NaOtBu, and 42 mL of xylene were mixed in a 250 mL one-neck flask. The mixture was stirred under reflux for 17 h. After completion of the reaction, the reaction mixture was cooled to room temperature, filtered through celite, purified by silica gel column chromatography (HEX:toluene), and recrystallized from acetone and hexane to give 1.2 g (yield 23.1%) of Compound **2-269** (LT20-30-170) as a yellow solid.

### Preparative Example 53: Synthesis of Compound 2-270 (LT20-30-379)

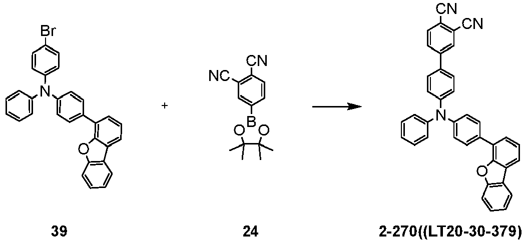

4.0 g (8.2 mmol) of Intermediate **39,** 3.1 g (12.2 mmol) of Intermediate **24,** 0.2 g (0.4 mmol) of Pd(dba)₂, 0.4 g (0.8 mmol) of X-Phos, and 126 mL of dioxane were added together and stirred in a 250 mL one-neck flask, and then 5.2 g (24.5 mmol) of K₃PO₄ and 41 mL of distilled water were added thereto. The mixture was heated to reflux with stirring throughout the day. After completion of the reaction, the reaction mixture was cooled to room temperature and the solvent was removed. The resulting mixture was passed through a pad of celite with chloroform, distilled under reduced pressure to remove the solvent, and purified by column chromatography (HEX:CHCl₃) to give 1.7 g (yield 38.3%) of Compound **2-270** (LT20-30-379) as a yellow solid.

### Preparative Example 54: Synthesis of Compound 2-271 (LT20-30-384)

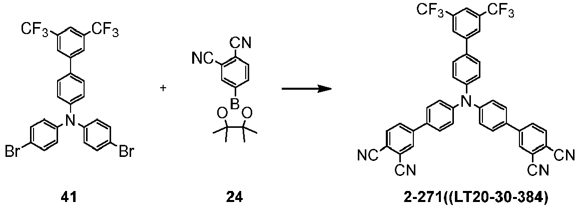

4.0 g (6.5 mmol) of Intermediate **41, 5.0** g (19.5 mmol) of Intermediate **24,** 0.4 g (0.7 mmol) of Pd(dba)₂, 0.6 g (1.3 mmol) of X-Phos, and 100 mL of dioxane were added together and stirred in a 250 mL one-neck flask, and then 4.1 g (19.5 mmol) of K₃PO₄ and 33 mL of distilled water were added thereto. The mixture was heated to reflux with stirring throughout the day. After completion of the reaction, the reaction mixture was cooled to room temperature and the solvent was removed. The resulting mixture was passed through a pad of celite with hot tetrahydrofuran, distilled under reduced pressure to remove the solvent, and purified by column chromatography (HEX:THF) to give 1.7 g (yield 36.4%) of Compound **2-271** (LT20-30-384) as a yellow solid.

### <TEST EXAMPLES>

The refractive indices (n) and extinction coefficients (k) of the inventive compounds were measured using an ellipsometer (J.A. WOOLLAM).

Preparation of monolayer films for test examples:
A glass substrate (0.7T) was washed with ethanol, DI water, and acetone (each for 10 min) and each of the inventive compounds was deposited to a thickness of 800 Å on the glass substrate to form a monolayer film, which was used to measure the optical properties of the compound.

Preparation of monolayer films for comparative test examples (Glass/REF01 (80 nm)):
REF01 (80 nm) was deposited on glass to fabricate a device for evaluating its optical properties. Before deposition of the compound, the glass was treated with a 125 W oxygen plasma at 2 × 10⁻² Torr for 2 min. The compound was deposited at a rate of 1 Å/sec and a degree of vacuum of 9 × 10⁻⁷ Torr to prepare a monolayer film.

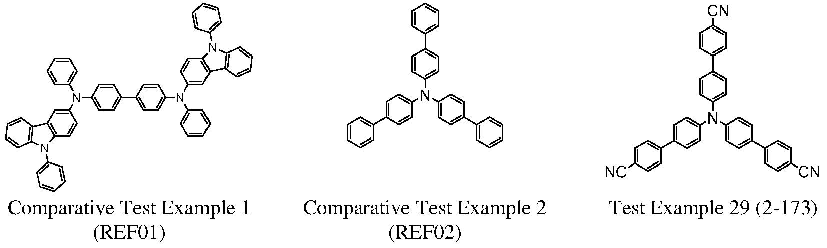

### <Test Examples 1-54>

Monolayer films were prepared in the same manner as in Comparative Test Example 1, except that the corresponding compounds shown in Table 1 were used instead of REF01.

The optical properties of the compounds used in Comparative Test Examples 1-2 and Test Examples 1-54 are shown in Table 1.

The optical properties were refractive indices at wavelengths of 450 nm and 620 nm and absorption rates at a wavelength of 380 nm.

**[Table 1]**

| Test Example No. | Compound | n (450 nm, 620 nm) | k (380 nm) |
|---|---|---|---|
| Comparative Test Example 1 | REF01 | 2.000, 1.846 | 0.193 |
| Comparative Test Example 2 | REF02 | 1.836, 1.730 | 0.155 |
| Test Example 1 | 2-1 (LT19-30-183) | 2.063, 1.915 | 0.298 |
| Test Example 2 | 2-18 (LT20-30-338) | 2.705, 2.095 | 0.808 |
| Test Example 3 | 2-25 (LT19-30-187) | 1.993, 1.852 | 0.281 |
| Test Example 4 | 2-29 (LT20-30-182) | 2.120, 1.948 | 0.391 |
| Test Example 5 | 2-32 (LT19-30-048) | 2.062, 1.907 | 0.297 |
| Test Example 6 | 2-35 (LT19-30-286) | 2.063, 1.915 | 0.312 |
| Test Example 7 | 2-49 (LT19-30-275) | 2.120, 1.948 | 0.432 |
| Test Example 8 | 2-50 (LT19-30-278) | 1.950, 1.821 | 0.721 |
| Test Example 9 | 2-51 (LT19-30-608) | 2.293, 1.978 | 0.972 |
| Test Example 10 | 2-70 (LT20-30-272) | 1.994, 1.882 | 0.280 |
| Test Example 11 | 2-74 (LT20-30-255) | 2.100, 1.874 | 0.524 |
| Test Example 12 | 2-78 (LT20-30-363) | 2.211, 1.990 | 0.501 |
| Test Example 13 | 2-99 (LT20-30-375) | 2.120, 1.948 | 0.432 |
| Test Example 14 | 2-103 (LT19-30-188) | 2.115, 1.939 | 0.486 |
| Test Example 15 | 2-107 (LT20-35-670) | 2.278, 2.081 | 0.666 |
| Test Example 16 | 2-133 (LT20-30-104) | 2.145, 1.955 | 0.664 |
| Test Example 17 | 2-134 (LT20-30-097) | 2.176, 1.999 | 0.482 |
| Test Example 18 | 2-146 (LT20-30-196) | 2.396, 2.080 | 1.061 |
| Test Example 19 | 2-147 (LT20-30-197) | 2.349, 2.027 | 0.721 |
| Test Example 20 | 2-150 (LT20-30-284) | 2.938, 2.231 | 1.102 |
| Test Example 21 | 2-159 (LT20-30-112) | 2.510, 2.047 | 1.098 |
| Test Example 22 | 2-160 (LT20-30-115) | 1.946, 1.817 | 0.288 |
| Test Example 23 | 2-161 (LT20-30-120) | 1.950, 1.821 | 0.721 |
| Test Example 24 | 2-162 (LT20-30-118) | 1.939, 1.823 | 0.835 |
| Test Example 25 | 2-169 (LT19-30-491) | 2.228, 1.986 | 0.776 |
| Test Example 26 | 2-170 (LT19-30-495) | 2.224, 2.010 | 0.573 |
| Test Example 27 | 2-171 (LT20-30-176) | 2.293, 2.042 | 0.557 |
| Test Example 28 | 2-172 (LT20-30-123) | 2.355, 2.117 | 0.721 |
| Test Example 29 | 2-173 (LT19-30-353) | 2.411, 2.031 | 1.120 |
| Test Example 30 | 2-174 (LT19-30-354) | 2.085, 1.903 | 0.596 |
| Test Example 31 | 2-175 (LT19-30-043) | 2.120, 1.948 | 0.721 |
| Test Example 32 | 2-179 (LT20-30-128) | 2.285, 1.983 | 0.811 |
| Test Example 33 | 2-181 (LT20-30-116) | 2.441, 2.052 | 0.940 |
| Test Example 34 | 2-182 (LT20-30-063) | 2.510, 2.061 | 1.231 |
| Test Example 35 | 2-188 (LT20-35-675) | 2.200, 2.000 | 0.517 |
| Test Example 36 | 2-196 (LT19-30-605) | 2.564, 2.062 | 1.188 |
| Test Example 37 | 2-197 (LT20-30-133) | 2.044, 1.864 | 0.524 |
| Test Example 38 | 2-198 (LT20-30-136) | 1.912, 1.793 | 0.358 |
| Test Example 39 | 2-199 (LT20-30-137) | 1.988, 1.855 | 0.431 |
| Test Example 40 | 2-213 (LT20-30-214) | 2.353, 2.036 | 0.902 |
| Test Example 41 | 2-247 (LT20-30-308) | 3.110, 2.396 | 0.862 |
| Test Example 42 | 2-248 (LT20-30-369) | 2.629, 2.113 | 0.849 |
| Test Example 43 | 2-249 (LT20-30-330) | 2.520, 2.020 | 0.792 |
| Test Example 44 | 2-251 (LT20-30-396) | 2.120, 1.948 | 0.835 |
| Test Example 45 | 2-257 (LT20-30-349) | 2.286, 1.929 | 0.386 |
| Test Example 46 | 2-259 (LT20-30-347) | 2.378, 1.949 | 0.364 |
| Test Example 47 | 2-260 (LT20-30-370) | 2.378, 1.949 | 0.397 |
| Test Example 48 | 2-265 (LT19-30-234) | 1.986, 1.851 | 0.255 |
| Test Example 49 | 2-266 (LT19-30-511) | 2.367, 2.013 | 1.204 |
| Test Example 50 | 2-267 (LT20-30-128) | 2.285, 1.983 | 0.811 |
| Test Example 51 | 2-268 (LT20-30-107) | 2.296, 1.979 | 1.106 |
| Test Example 52 | 2-269 (LT20-30-170) | 2.151, 1.905 | 0.843 |
| Test Example 53 | 2-270 (LT20-30-379) | 2.323, 1.925 | 0.435 |
| Test Example 54 | 2-271 (LT20-30-384) | 2.460, 1.941 | 0.764 |

As can be seen from the results in Table 1, the refractive index (2,411) of Compound 2-173 with cyano groups (Test Example 29) was higher than that (1,836) of REF02 without cyano groups (Comparative Test Example 2) despite the similar chemical structures of the two compounds.

The refractive index (n) of REP01 used in Comparative Test Example 1 at 450 nm was 2,000. In contrast, most of the inventive compounds were found to have higher refractive indices than 2,000. The refractive indices of the inventive compounds were high enough to secure wide viewing angles in the blue wavelength range.

The k values of most of the inventive compounds at 380 nm corresponding to the beginning of the UV region were also confirmed to be higher than those of REP01 and REP02. These results indicate that the inventive compounds can effectively absorb light in the UV region from high-energy external light sources to minimize damage to organic materials in organic light emitting devices, contributing to a substantial improvement in the lifetime of the organic electroluminescent devices.

### <EXAMPLES>

### Device fabrication

A transparent ITO electrode was used as an anode, 2-TNATA was used as a material for a hole injection layer, NPB was used as a material for a hole transporting layer, αβ-ADN and Pyene-CN were used as a host and a blue fluorescent dopant for an emission layer, respectively, Liq was used as a material for an electron injection layer, and Mg:Ag was used as a material for a cathode to fabricate a device. The structures of 2-TNATA, NPB, and αβ-ADN are as follow:

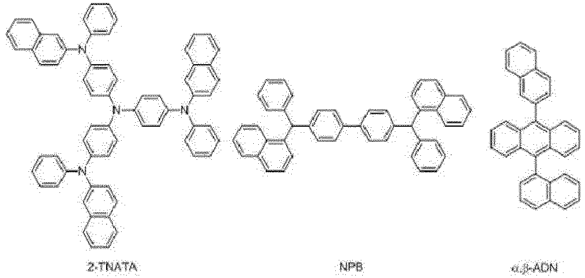

### Comparative Example 1: ITO/2-TNATA (60 nm)/NPB (20 nm)/αβ-ADN: 10% pyrene-CN (30 nm)/Alq₃ (30 nm)/Liq (2 nm)/Mg:Ag (1:9, 10 nm)/REF01 (60 nm)

ITO (180 nm), 2-TNATA (60 nm), NPB (20 nm), αβ-ADN:pyrene-CN 10% (30 nm), Alq₃ (30 nm), Liq (2 nm), and Mg:Ag (1:9, 10 nm) were deposited in this order to fabricate a blue fluorescent organic electroluminescent device. Before deposition of the organic materials, the ITO electrode was treated with a 125 W oxygen plasma at 2 × 10⁻² Torr for 2 min. The organic materials were deposited at a degree of vacuum of 9 × 10⁻⁷ Torr. Liq was deposited at a rate of 0.1 Å/sec, αβ-ADN and pyrene-CN were deposited simultaneously at rates of 0.18 Å/sec and 0.02 Å/sec, respectively, and the other organic materials were deposited at a rate of 1 Å/sec. REF01 was selected and used as a material for a capping layer. The device was sealed in a glove box filled with nitrogen gas to prevent contact with air and moisture. After a barrier was formed with an adhesive tape (3M), barium oxide as a moisture absorbent was placed to remove moisture and a glass plate was attached to the barrier.

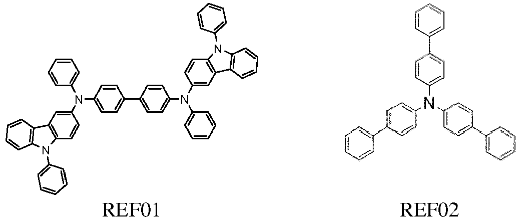

### <Examples 1-54>

Organic electroluminescent devices were fabricated in the same manner as in Comparative Example 1, except that the corresponding compounds shown in Table 2 were used instead of REF01.

The electroluminescent properties of the organic electroluminescent devices fabricated Comparative Examples 1-2 and Examples 1-54 are shown in Table 2.

**[Table 2]**

| Example No. | Compound | Driving voltage (V) | Efficiency (cd/A) | Lifetime (%) |
|---|---|---|---|---|
| Comparative Example 1 | REF01 | 4.50 | 5.10 | 88.92 |
| Comparative Example 2 | REF02 | 4.47 | 5.57 | 96.12 |
| Example 1 | 2-1 (LT19-30-183) | 4.23 | 5.35 | 97.12 |
| Example 2 | 2-18 (LT20-30-338) | 4.42 | 5.83 | 97.32 |
| Example 3 | 2-25 (LT19-30-187) | 4.43 | 5.99 | 97.10 |
| Example 4 | 2-29 (LT20-30-182) | 4.45 | 5.88 | 97.66 |
| Example 5 | 2-32 (LT19-30-048) | 4.51 | 5.68 | 98.12 |
| Example 6 | 2-35 (LT19-30-286) | 4.47 | 6.52 | 98.13 |
| Example 7 | 2-49 (LT19-30-275) | 4.45 | 6.13 | 97.45 |
| Example 8 | 2-50 (LT19-30-278) | 4.38 | 6.23 | 97.40 |
| Example 9 | 2-51 (LT19-30-608) | 4.40 | 6.10 | 97.32 |
| Example 10 | 2-70 (LT20-30-272) | 4.42 | 6.00 | 98.00 |
| Example 11 | 2-74 (LT20-30-255) | 4.49 | 5.99 | 95.61 |
| Example 12 | 2-78 (LT20-30-363) | 4.44 | 6.12 | 97.98 |
| Example 13 | 2-99 (LT20-30-375) | 4.45 | 6.05 | 97.56 |
| Example 14 | 2-103 (LT19-30-188) | 4.44 | 6.01 | 97.55 |
| Example 15 | 2-107 (LT20-35-670) | 4.50 | 5.55 | 98.00 |
| Example 16 | 2-133 (LT20-30-104) | 4.44 | 6.52 | 97.98 |
| Example 17 | 2-134 (LT20-30-097) | 4.51 | 6.12 | 97.47 |
| Example 18 | 2-146 (LT20-30-196) | 4.40 | 6.10 | 97.32 |
| Example 19 | 2-147 (LT20-30-197) | 4.40 | 6.22 | 98.11 |
| Example 20 | 2-150 (LT20-30-284) | 4.42 | 6.11 | 97.54 |
| Example 21 | 2-159 (LT20-30-112) | 4.40 | 6.25 | 97.42 |
| Example 22 | 2-160 (LT20-30-115) | 4.41 | 6.23 | 97.55 |
| Example 23 | 2-161 (LT20-30-120) | 4.41 | 6.25 | 97.44 |
| Example 24 | 2-162 (LT20-30-118) | 4.51 | 5.82 | 97.33 |
| Example 25 | 2-169 (LT19-30-491) | 4.51 | 5.68 | 98.12 |
| Example 26 | 2-170 (LT19-30-495) | 4.40 | 6.25 | 97.42 |
| Example 27 | 2-171 (LT20-30-176) | 4.51 | 5.68 | 98.12 |
| Example 28 | 2-172 (LT20-30-123) | 4.40 | 6.22 | 98.11 |
| Example 29 | 2-173 (LT19-30-353) | 4.49 | 5.99 | 95.61 |
| Example 30 | 2-174 (LT19-30-354) | 4.41 | 6.23 | 97.55 |
| Example 31 | 2-175 (LT19-30-043) | 4.45 | 5.88 | 97.66 |
| Example 32 | 2-179 (LT20-30-128) | 4.38 | 6.23 | 97.40 |
| Example 33 | 2-181 (LT20-30-116) | 4.40 | 6.25 | 97.42 |
| Example 34 | 2-182 (LT20-30-063) | 4.40 | 6.10 | 97.32 |
| Example 35 | 2-188 (LT20-35-675) | 4.48 | 5.12 | 97.99 |
| Example 36 | 2-196 (LT19-30-605) | 4.41 | 6.23 | 97.55 |
| Example 37 | 2-197 (LT20-30-133) | 4.44 | 6.52 | 97.98 |
| Example 38 | 2-198 (LT20-30-136) | 4.51 | 5.68 | 98.12 |
| Example 39 | 2-199 (LT20-30-137) | 4.42 | 6.11 | 97.54 |
| Example 40 | 2-213 (LT20-30-214) | 4.40 | 6.10 | 97.32 |
| Example 41 | 2-247 (LT20-30-308) | 4.44 | 6.12 | 97.98 |
| Example 42 | 2-248 (LT20-30-369) | 4.42 | 6.11 | 97.54 |
| Example 43 | 2-249 (LT20-30-330) | 4.44 | 6.52 | 97.98 |
| Example 44 | 2-251 (LT20-30-396) | 4.42 | 5.83 | 97.32 |
| Example 45 | 2-257 (LT20-30-349) | 4.43 | 5.99 | 97.10 |
| Example 46 | 2-259 (LT20-30-347) | 4.45 | 5.88 | 97.66 |
| Example 47 | 2-260 (LT20-30-370) | 4.51 | 5.68 | 98.12 |
| Example 48 | 2-265 (LT19-30-234) | 4.51 | 5.82 | 97.33 |
| Example 49 | 2-266 (LT19-30-511) | 4.51 | 5.68 | 98.12 |
| Example 50 | 2-267 (LT20-30-128) | 4.44 | 6.01 | 97.55 |
| Example 51 | 2-268 (LT20-30-107) | 4.42 | 6.00 | 98.00 |
| Example 52 | 2-269 (LT20-30-170) | 4.38 | 6.23 | 97.40 |
| Example 53 | 2-270 (LT20-30-379) | 4.40 | 6.25 | 97.42 |
| Example 54 | 2-271 (LT20-30-384) | 4.45 | 6.13 | 97.45 |

The results in Table 2 reveal that the inventive tertiary amine derivatives can be used as materials for capping layers of organic electronic devices (including organic light emitting devices). In addition, organic electronic devices (including organic light emitting devices) using the inventive tertiary amine derivatives have excellent characteristics in terms of efficiency, driving voltage, stability, etc. Particularly, the inventive compounds exhibit high efficiencies due to their outstanding ability to form micro-cavities.

Therefore, it can be concluded that the compound of Formula 1 has unexpectedly desirable characteristics for use as a material for a capping layer of an OLED.

Based on its characteristics, the compound of the present invention can be used in organic electronic devices for industrial applications.

The above synthesis examples are merely illustrative and the reaction conditions may vary as required. The compounds according to exemplary embodiments of the present invention may be substituted with various substituents using suitable methods and materials known in the art. The introduction of various substituents to the core structure of the compound represented by Formula 1 allows the substituted compounds to have characteristics suitable for use in organic electroluminescent devices.

### Industrial Applicability

The tertiary amine derivative of the present invention can be used as a material for an organic layer and/or a capping layer of an organic electroluminescent device to improve the quality of the device.

When the compound of the present invention is used in a capping layer, the optical properties of the compound ensure an improvement in the lifetime of an organic electroluminescent device using the capping layer while allowing the device to exhibit its original characteristics.

## Claims

1. A tertiary amine derivative for a capping layer of an organic electroluminescent device, represented by Formula 1: 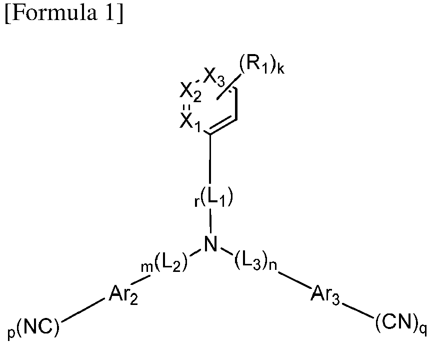 wherein X₁, X₂, and X₃ are each independently CH or N, n, m, and r are each independently 0 to 2, p and q are each independently 0 to 5, k is 0 to 2, R₁ is selected from hydrogen, deuterium, fluoro, cyano, methyl, t-butyl, trimethylsilyl, and trifluoromethyl, L₁, L₂, and L₃ are each independently selected from phenylene, pyridylene, naphthalenyl, dibenzofuran, and dibenzothiophene groups, and Ar₂ and Ar₃ are the same as or different from each other and are each independently selected from substituted or unsubstituted phenyl, substituted or unsubstituted naphthyl, substituted or unsubstituted pyridine, substituted or unsubstituted dibenzofuran, substituted or unsubstituted dibenzothiophene, substituted or unsubstituted benzoxazole, substituted or unsubstituted benzothiazole groups, and substituted or unsubstituted benzimidazole groups.

2. The tertiary amine derivative according to claim1, wherein the tertiary amine derivative is selected from the compounds represented by Formulae 2:

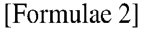

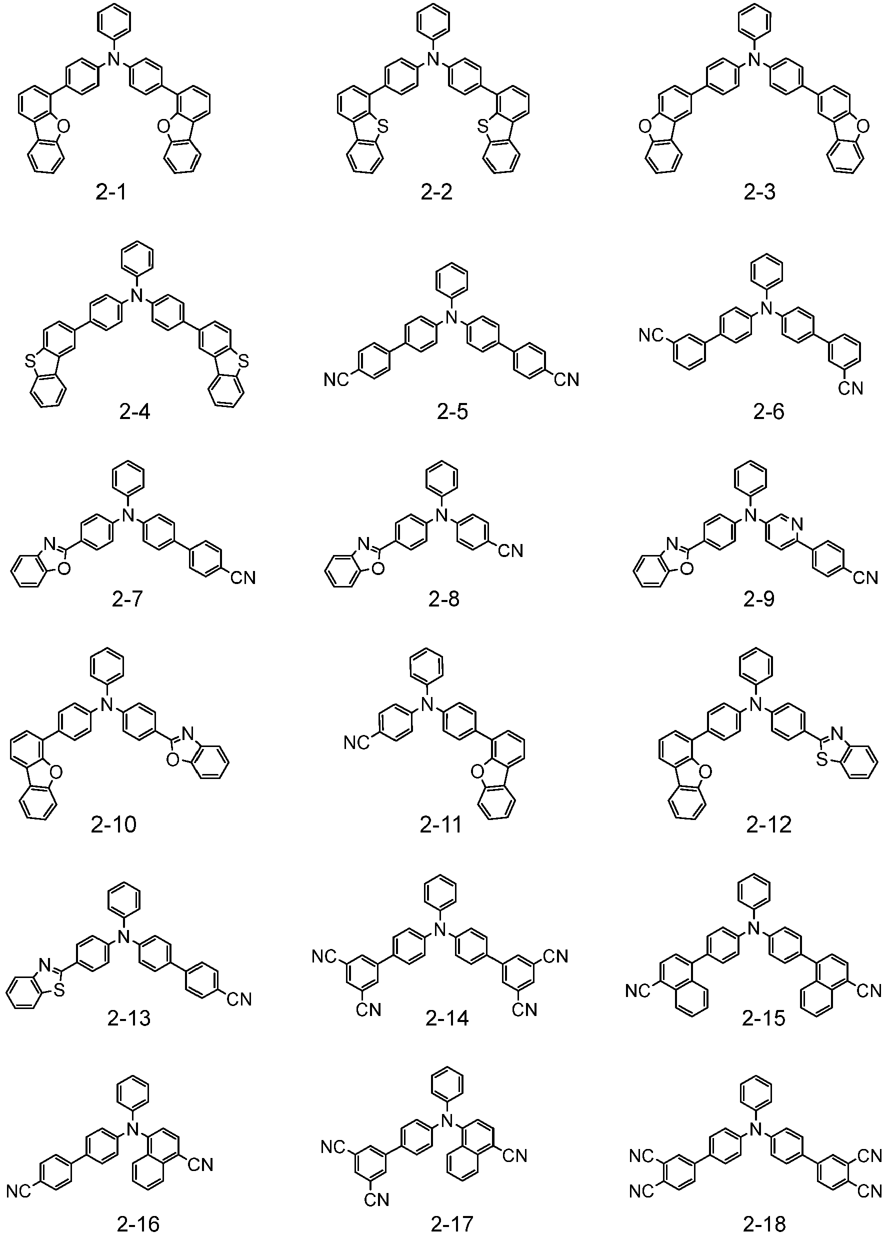

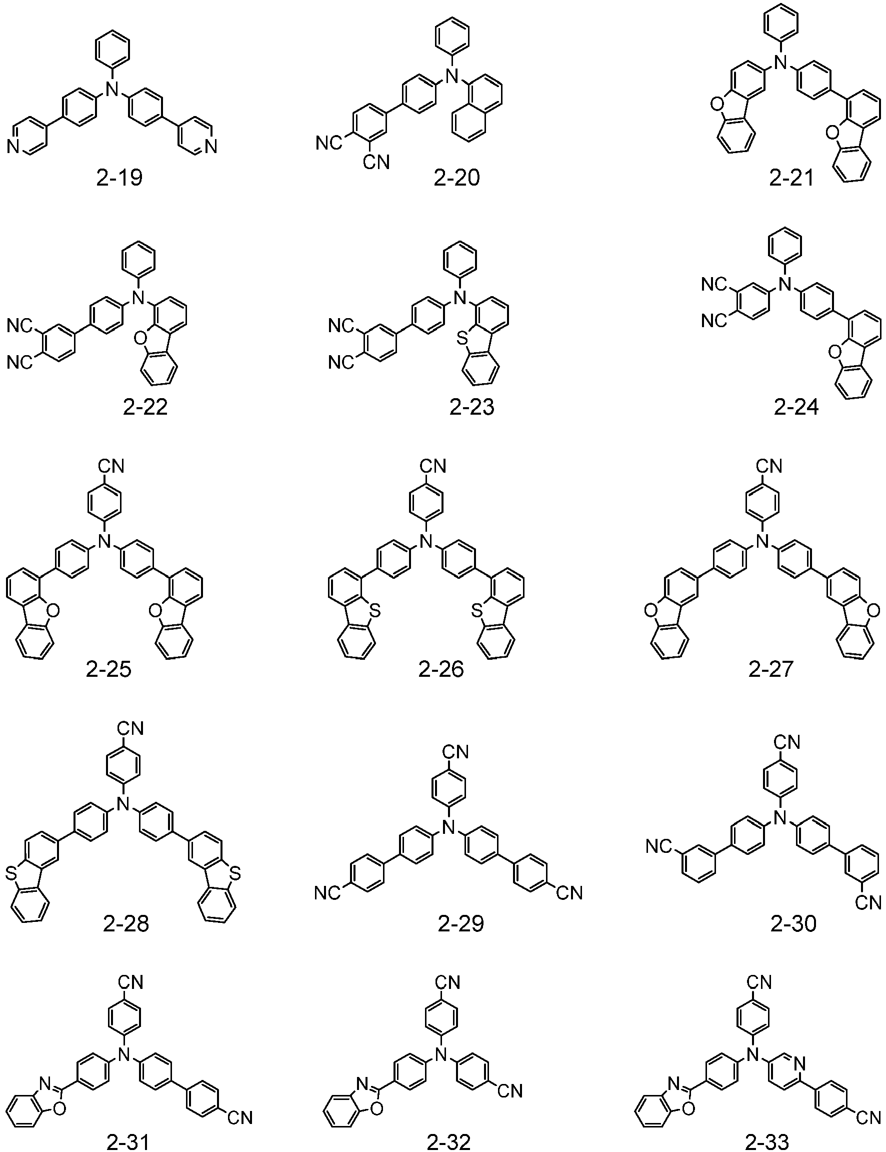

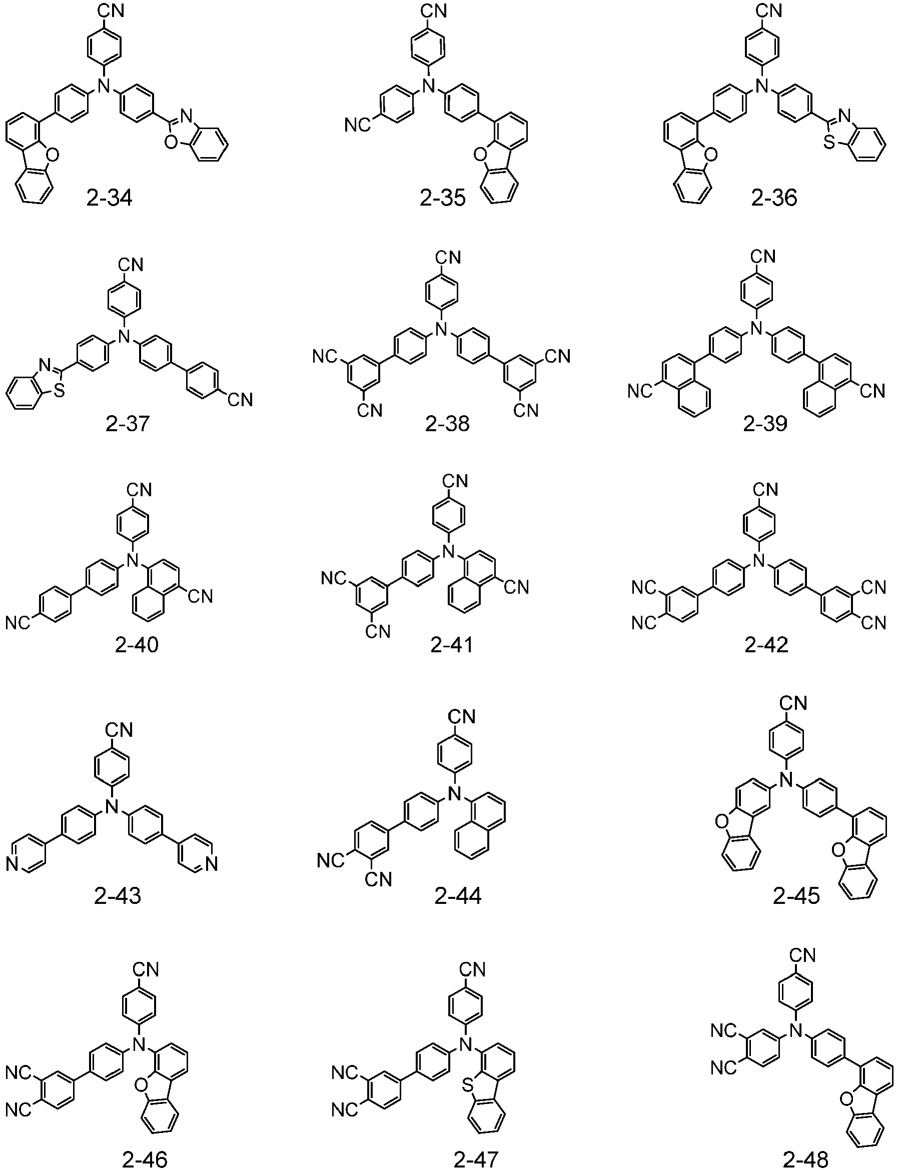

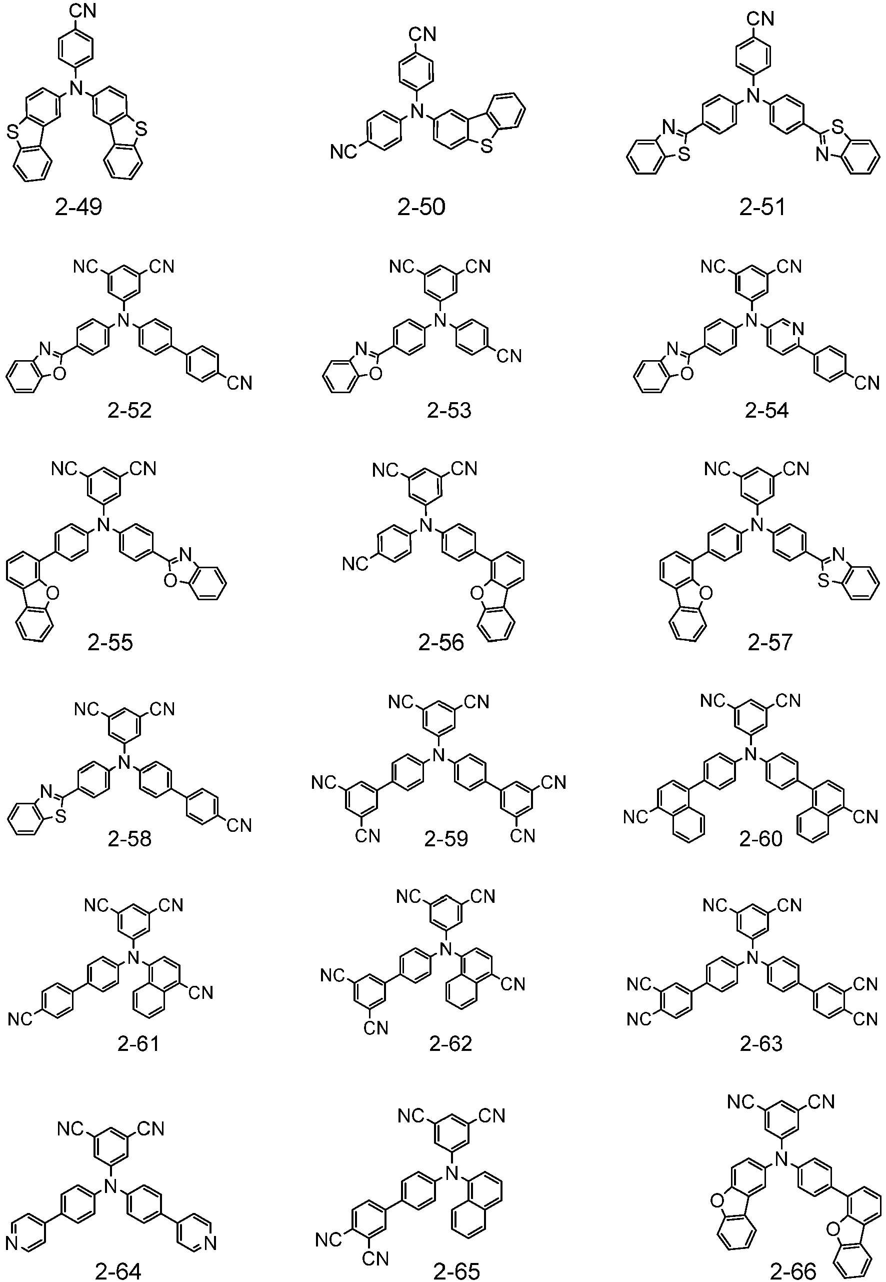

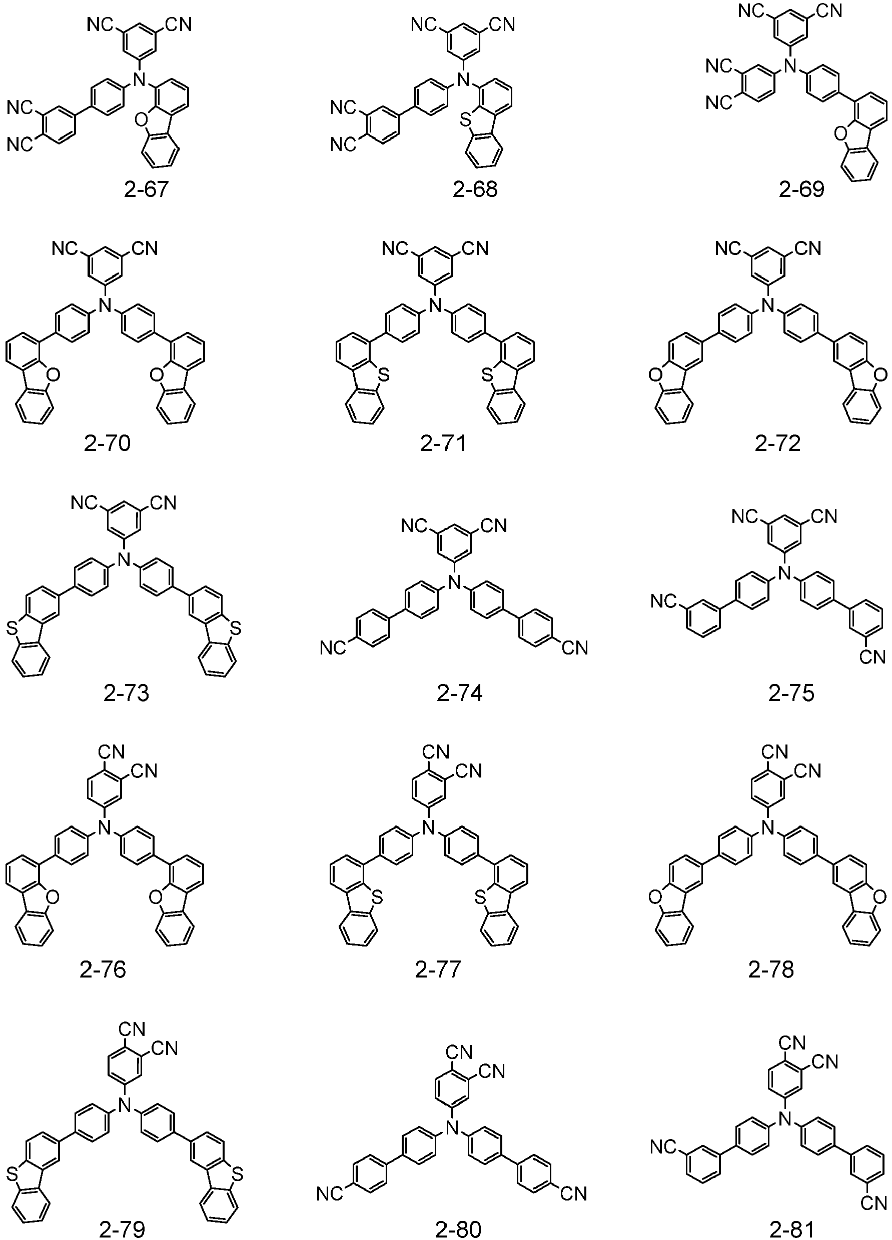

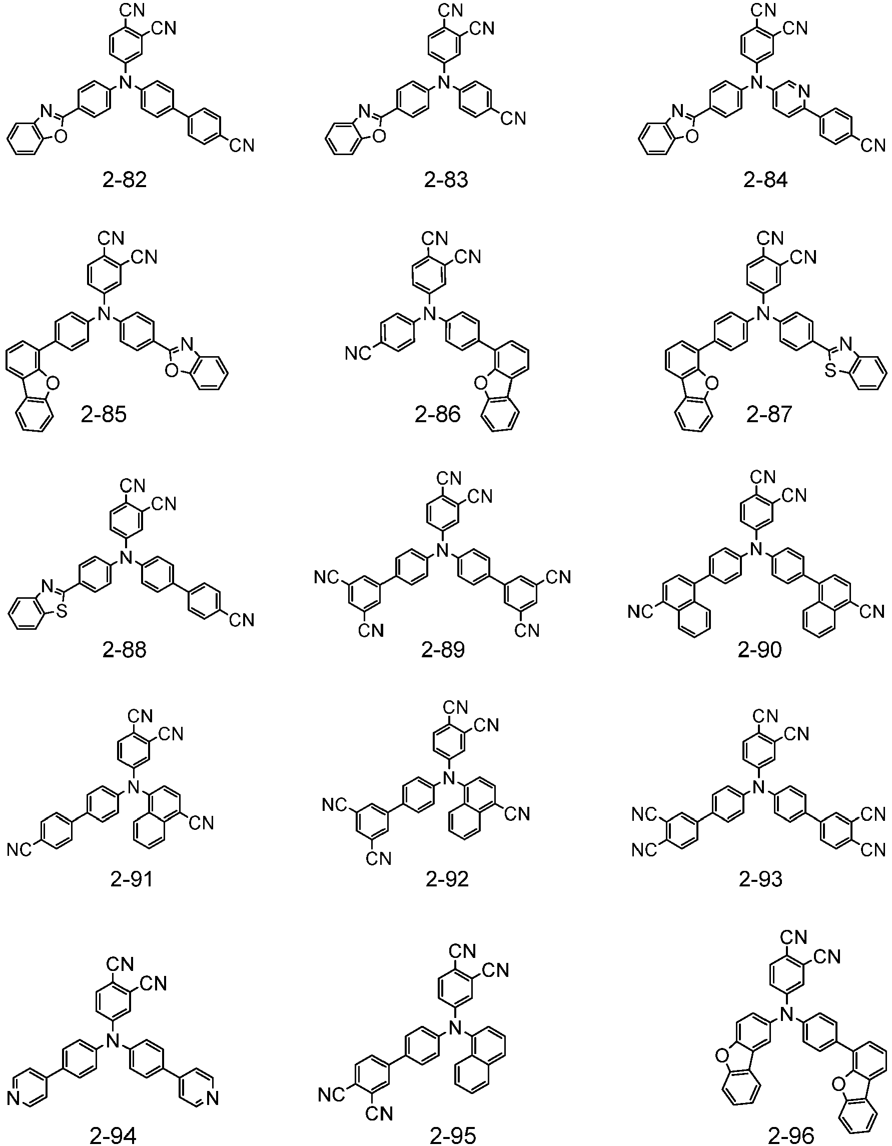

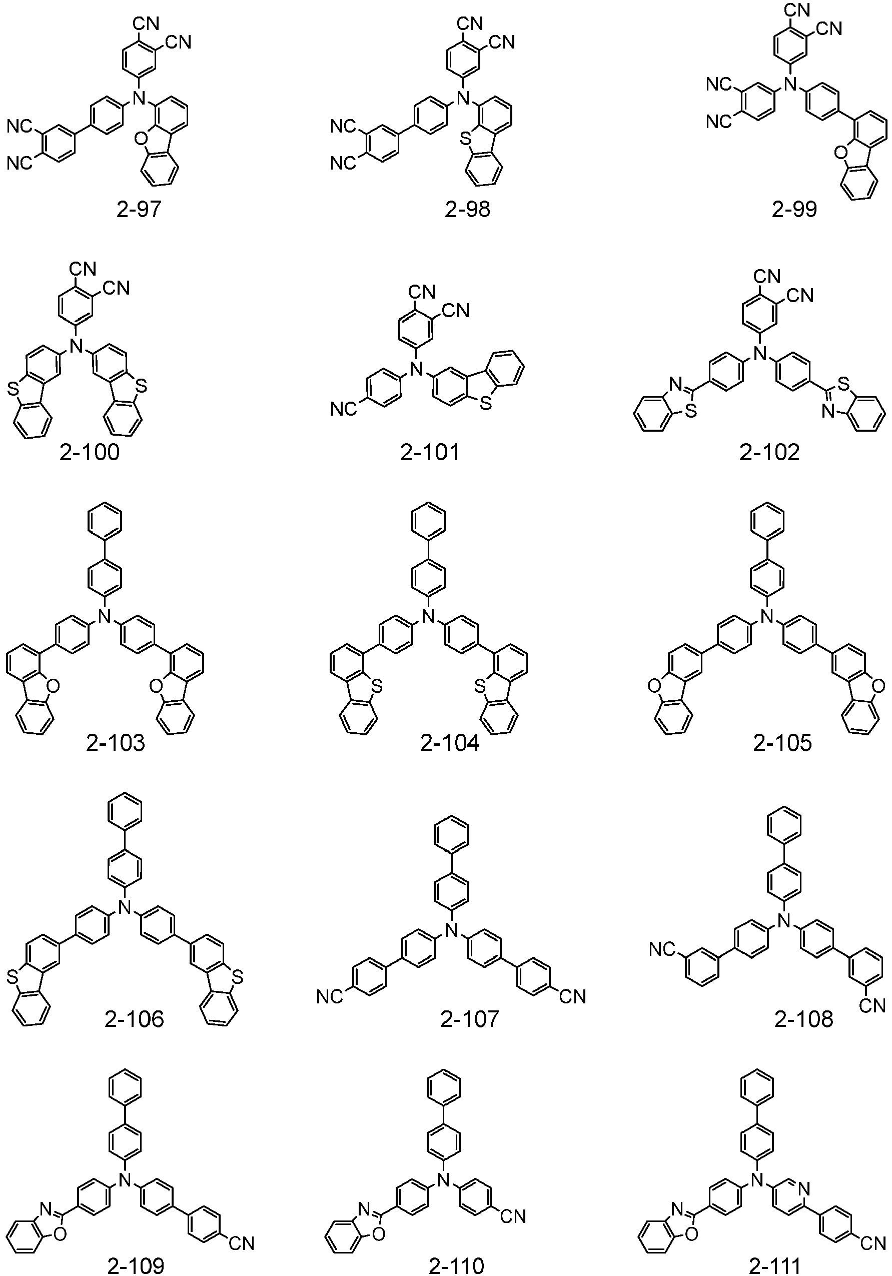

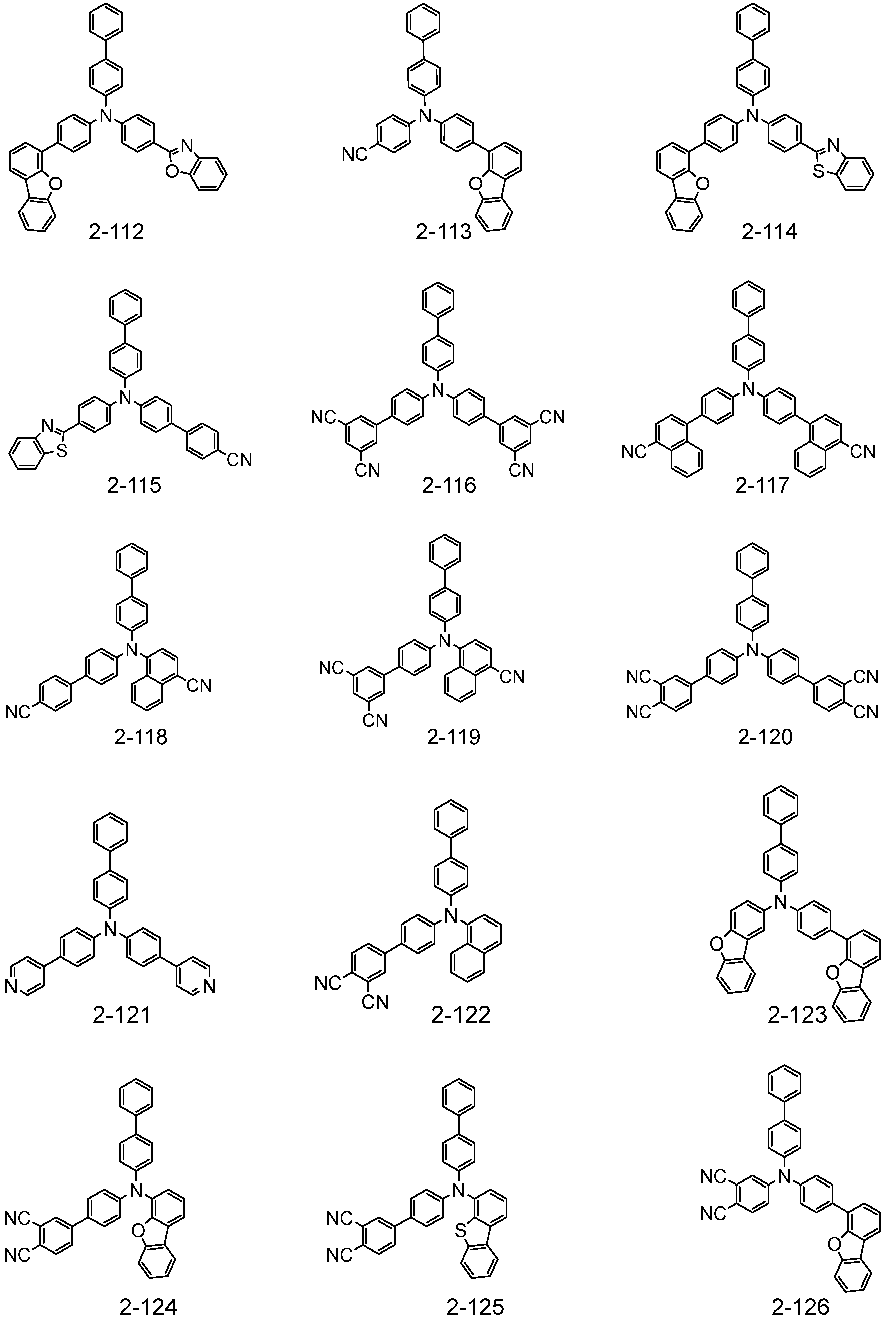

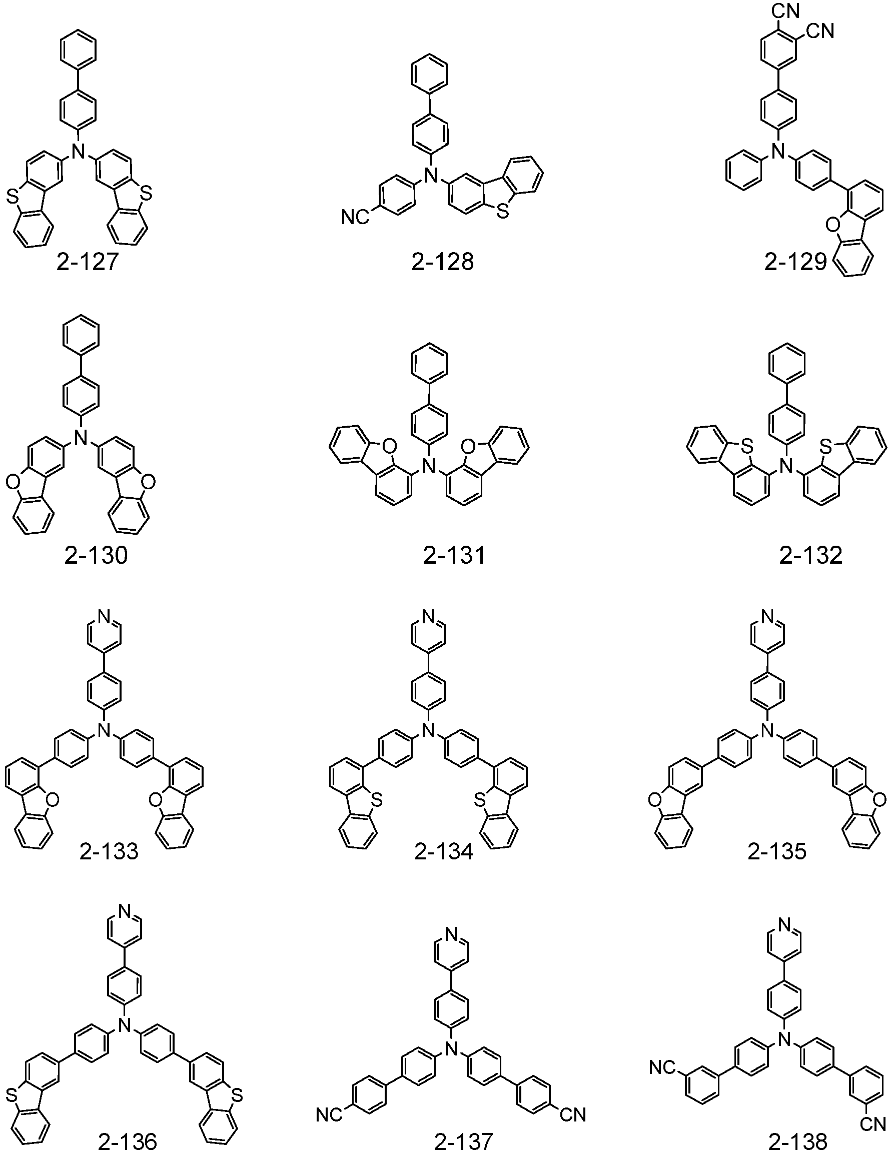

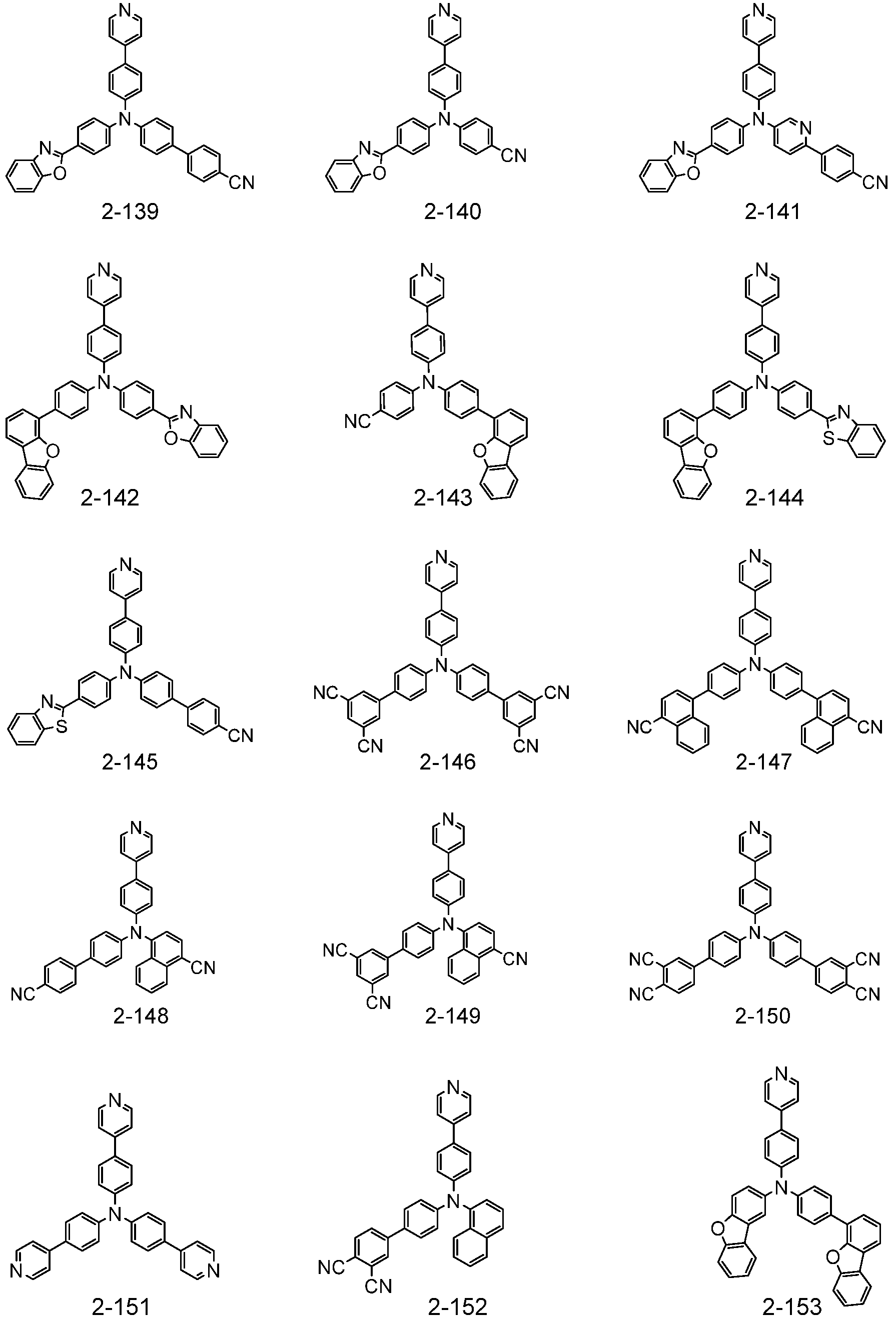

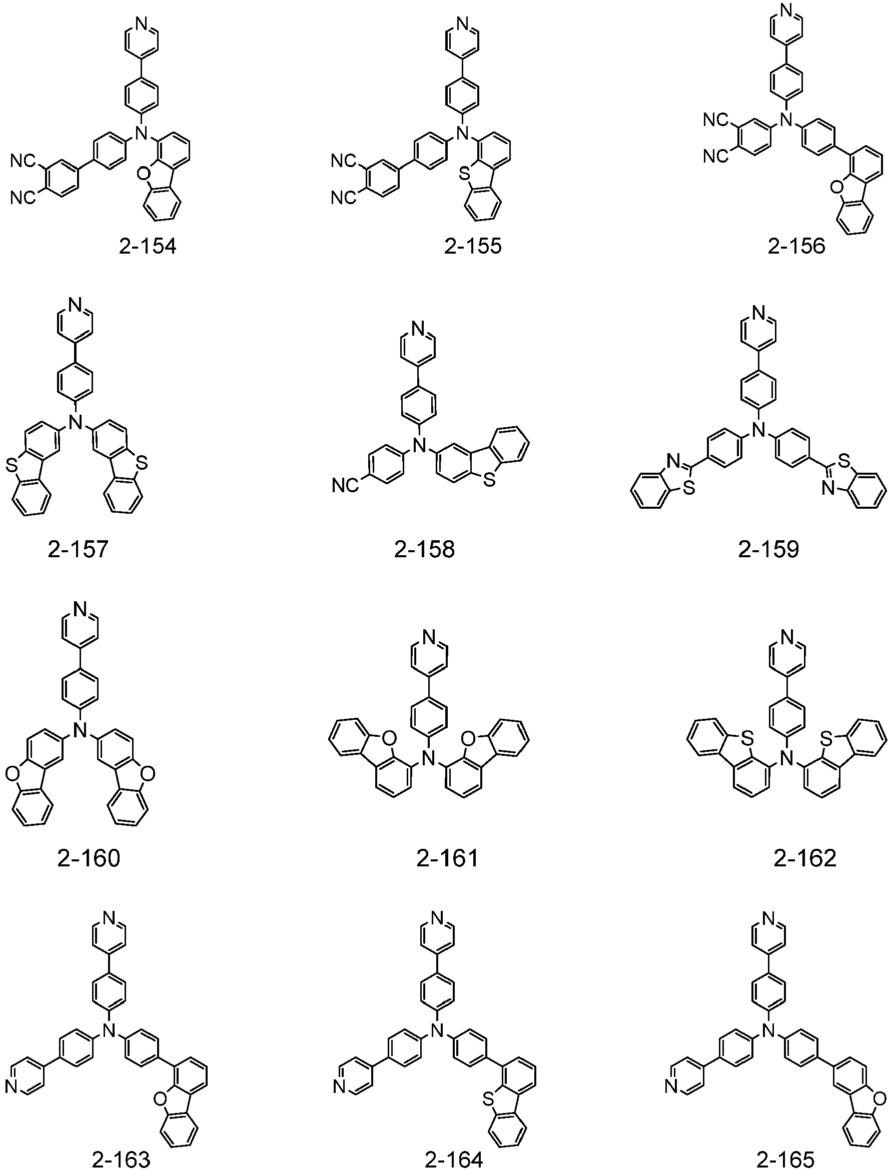

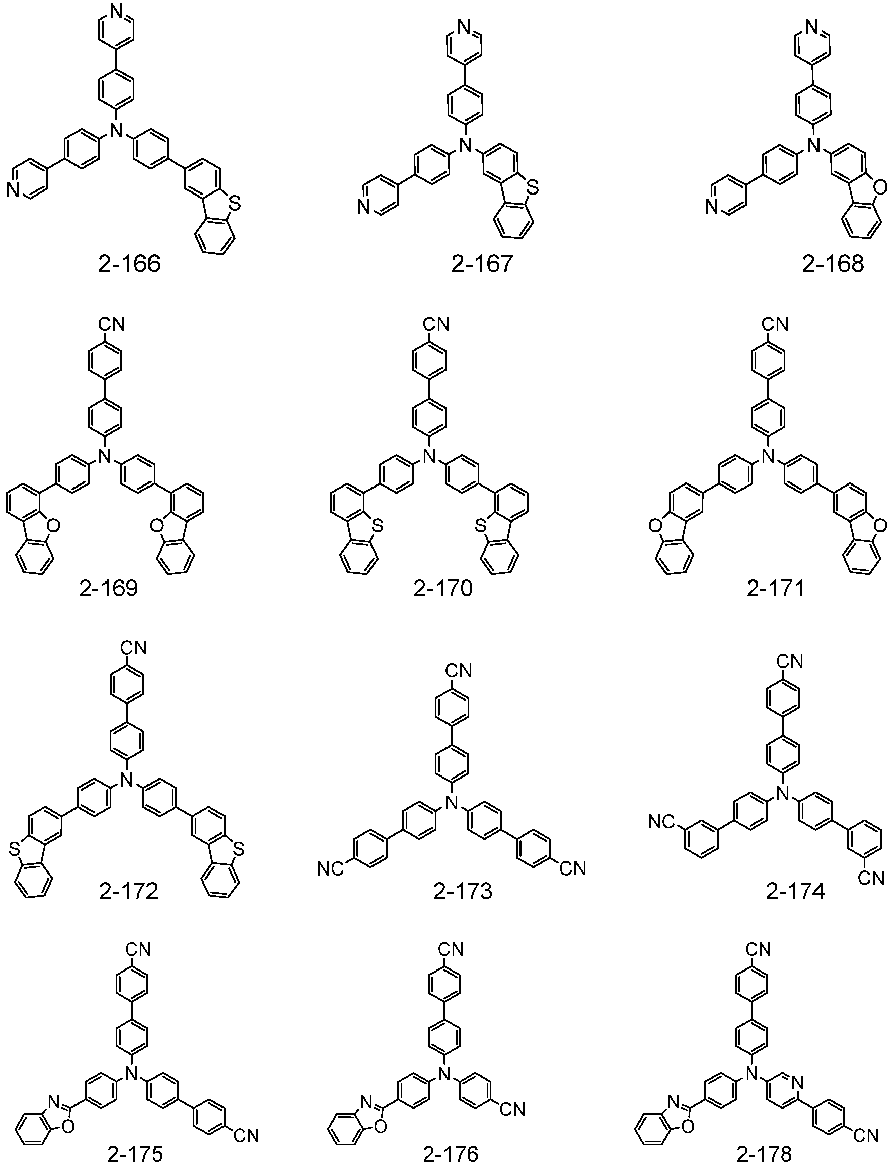

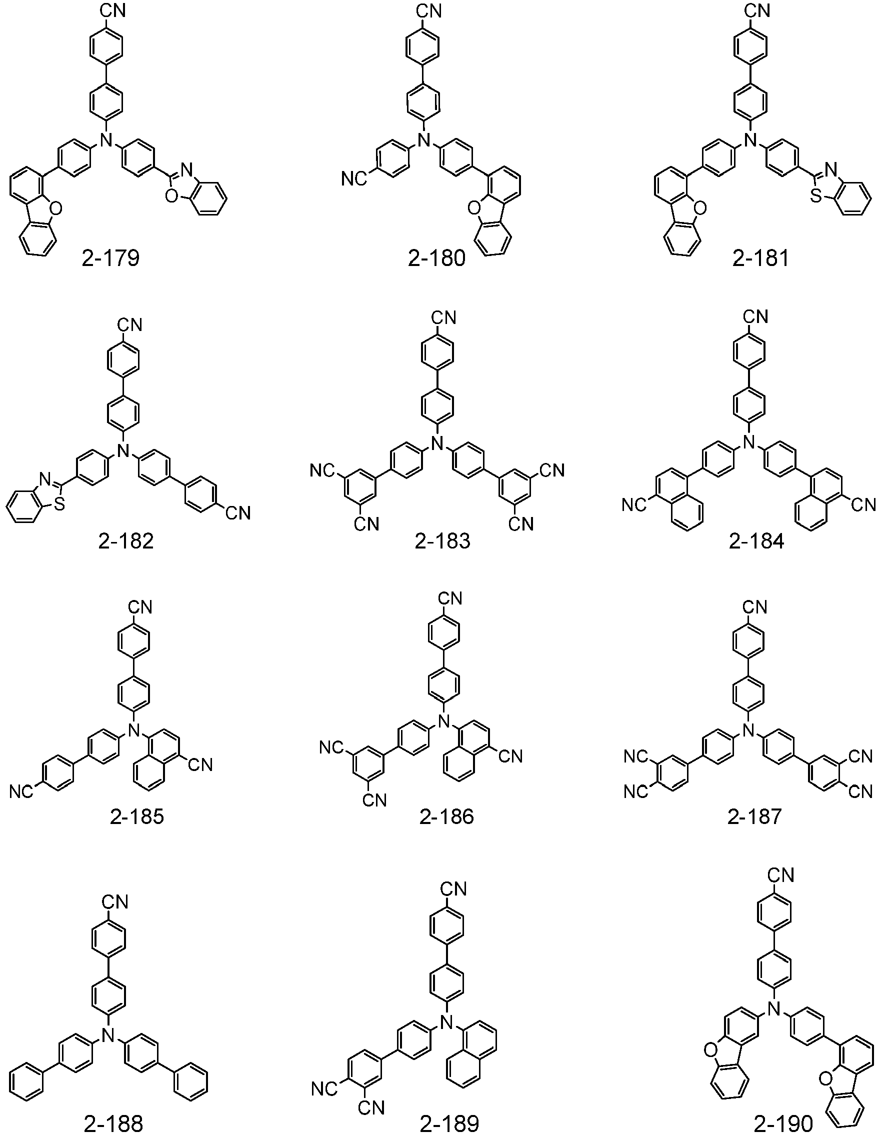

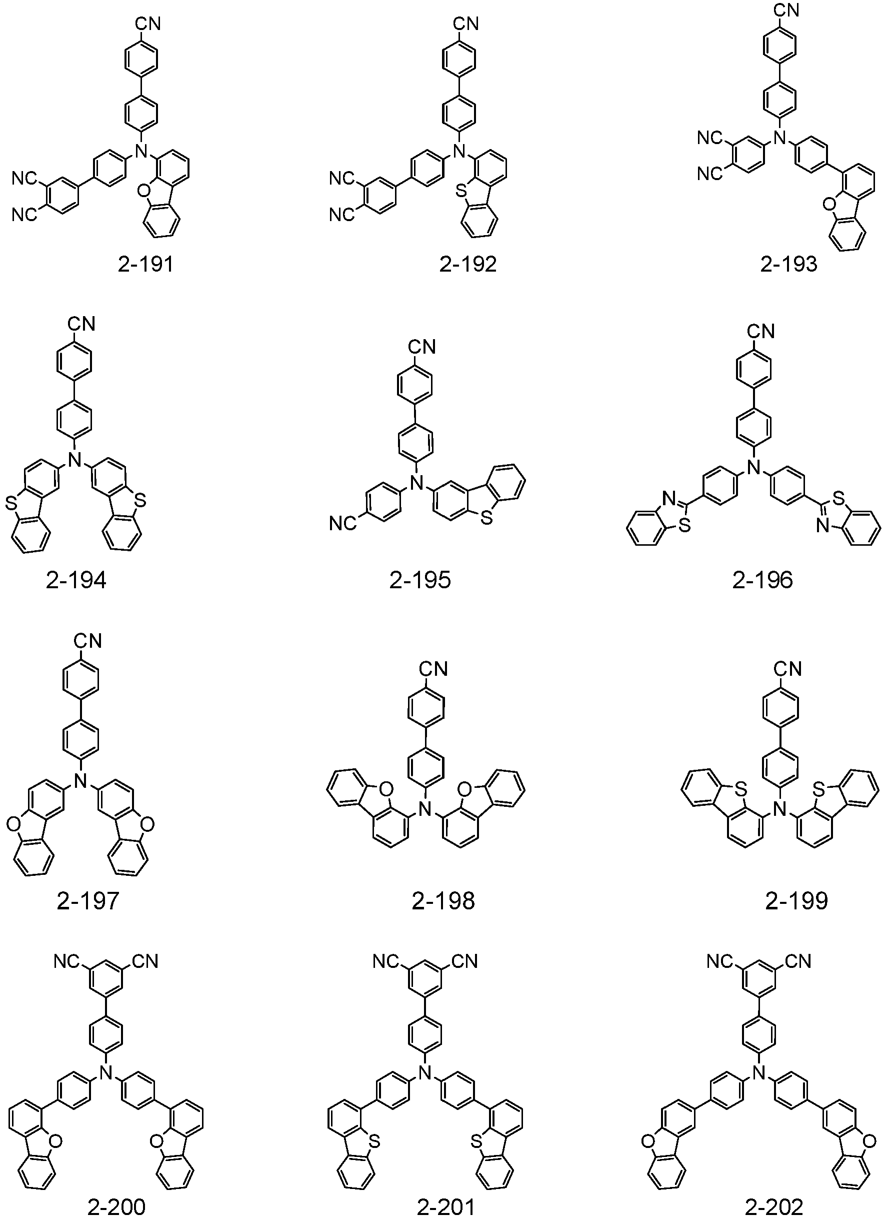

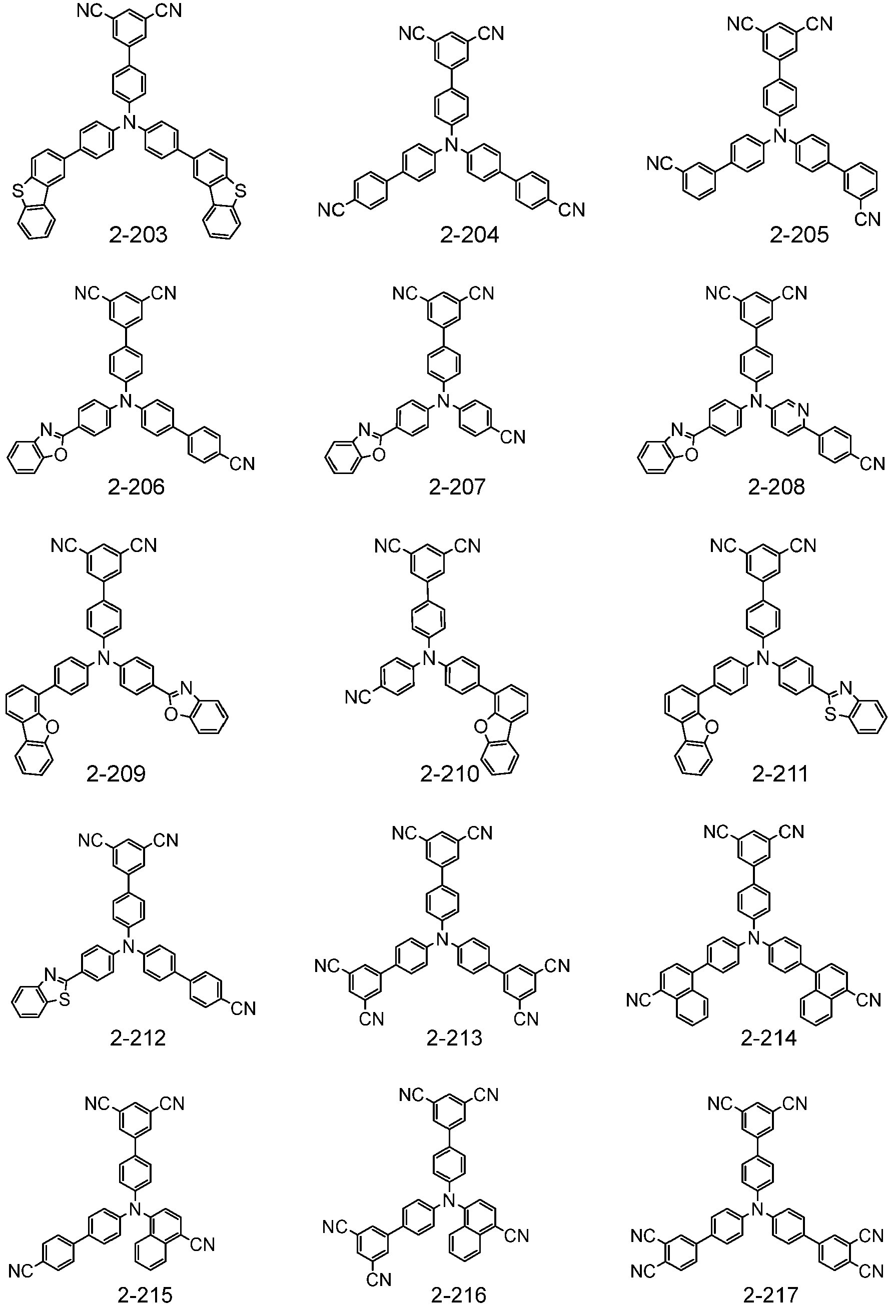

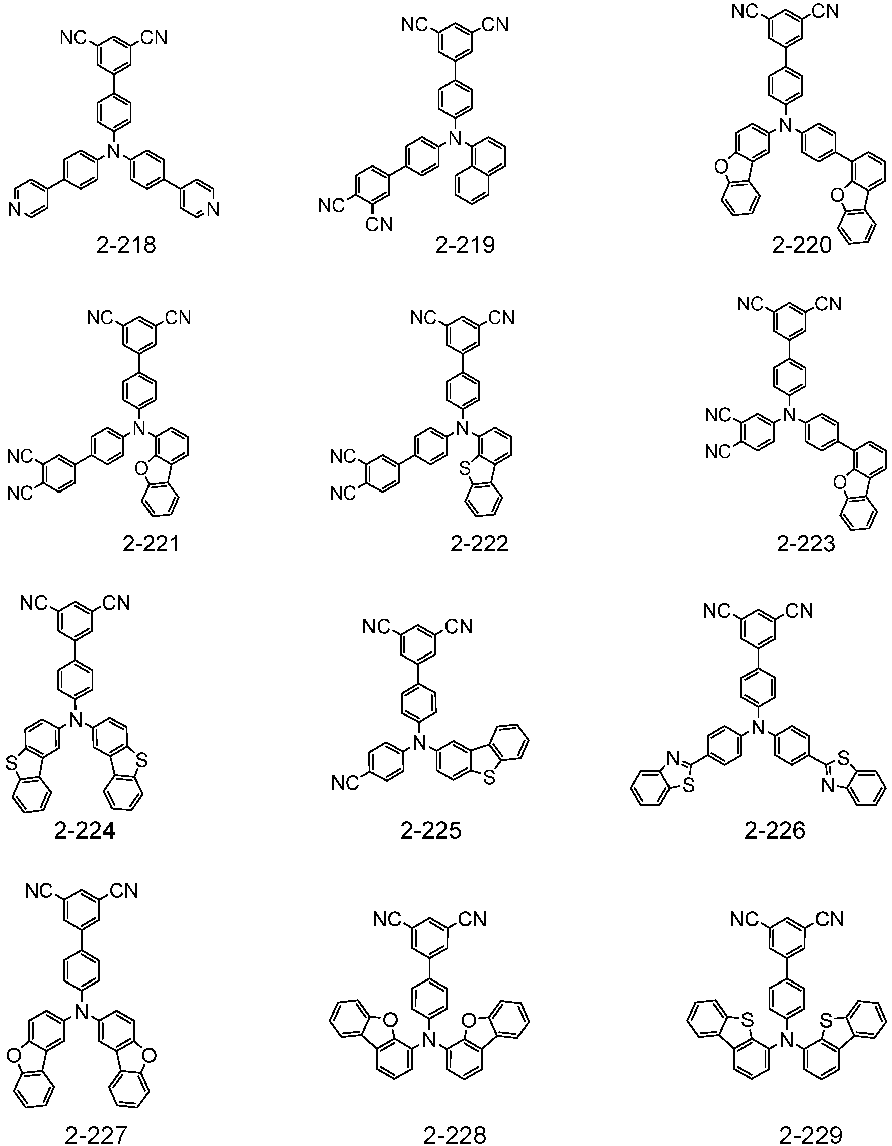

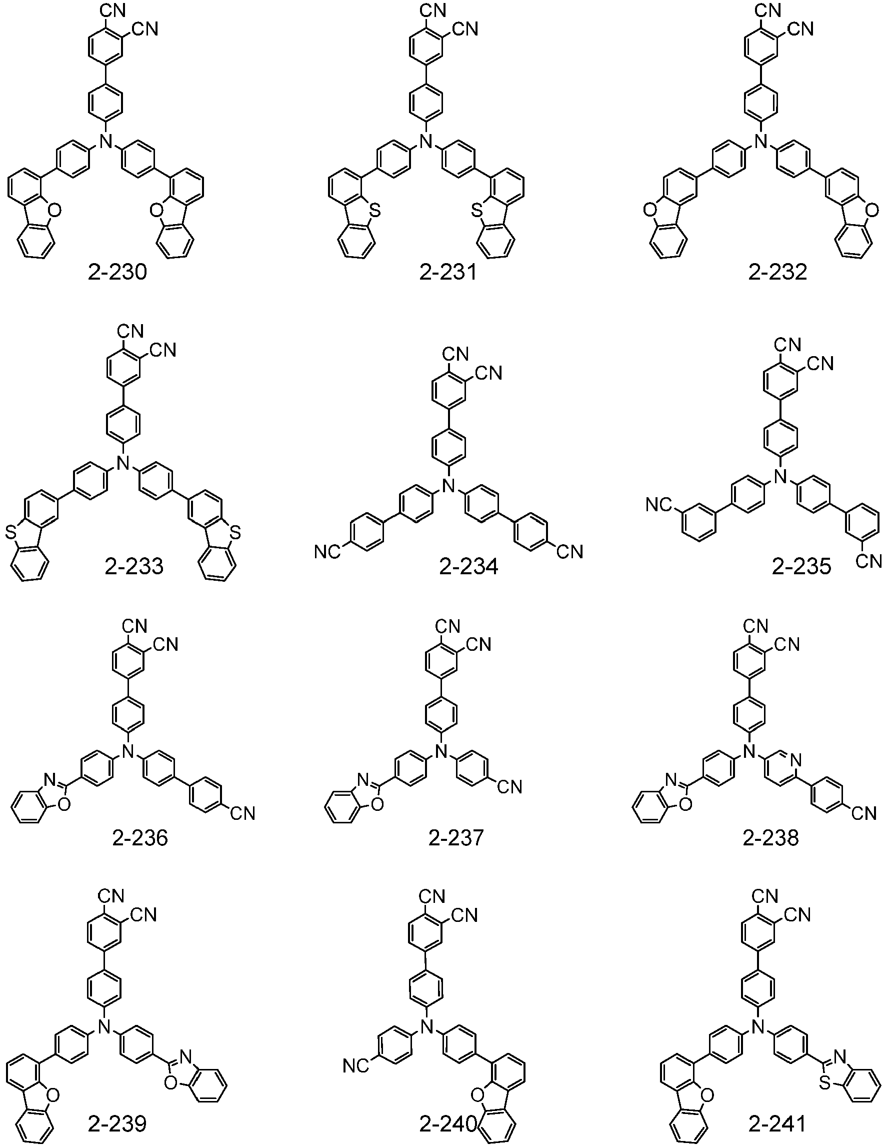

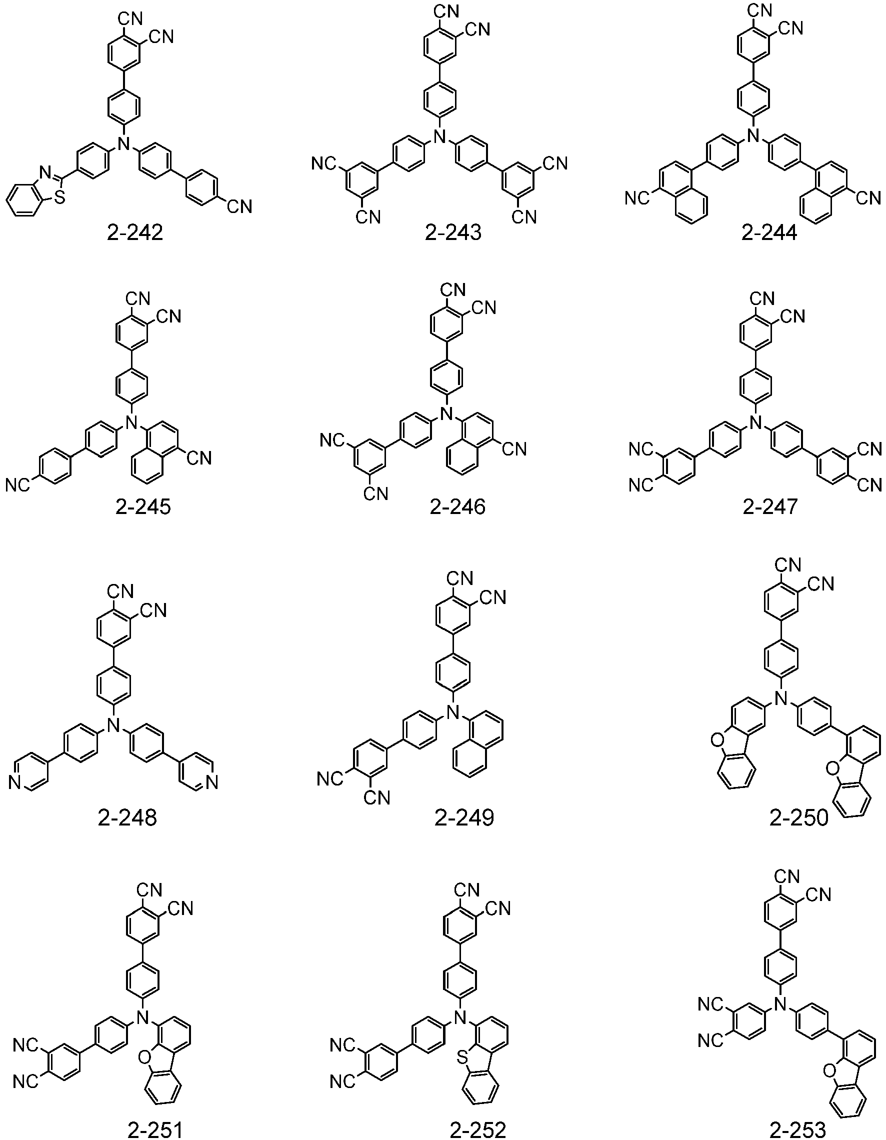

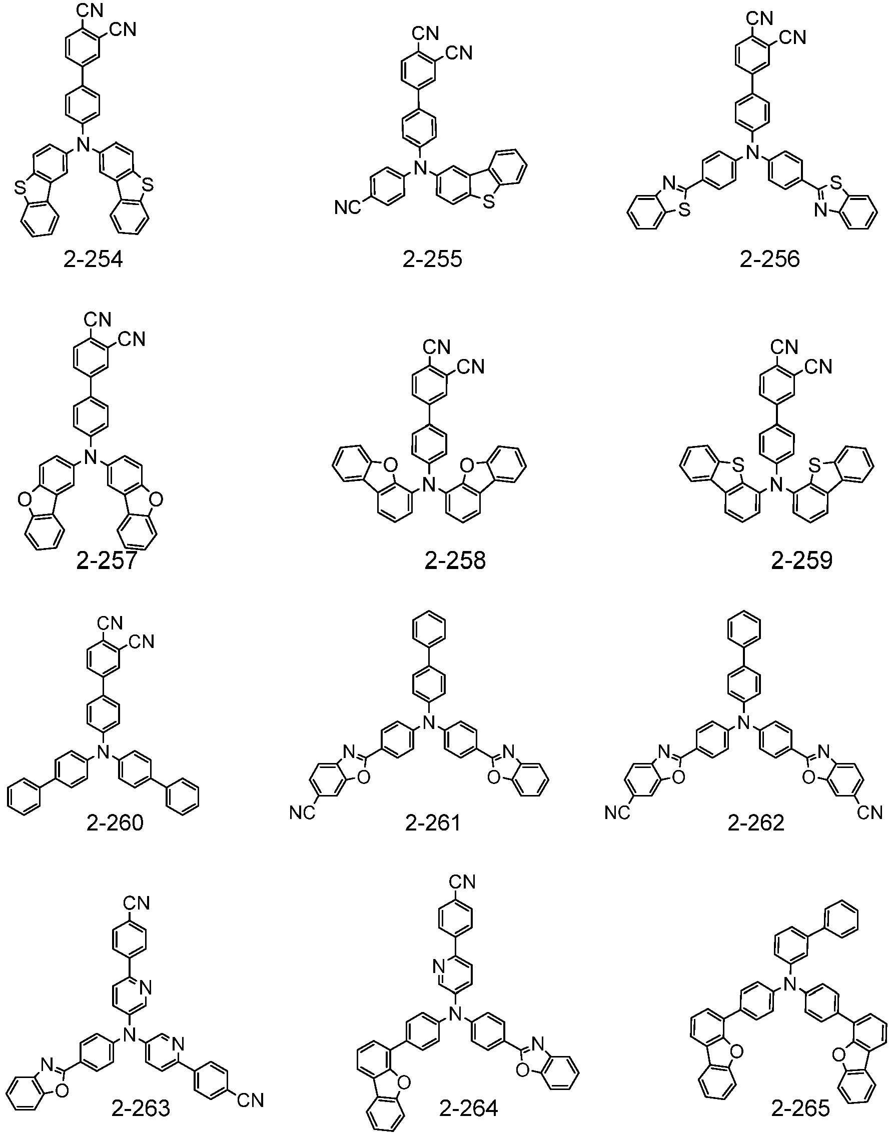

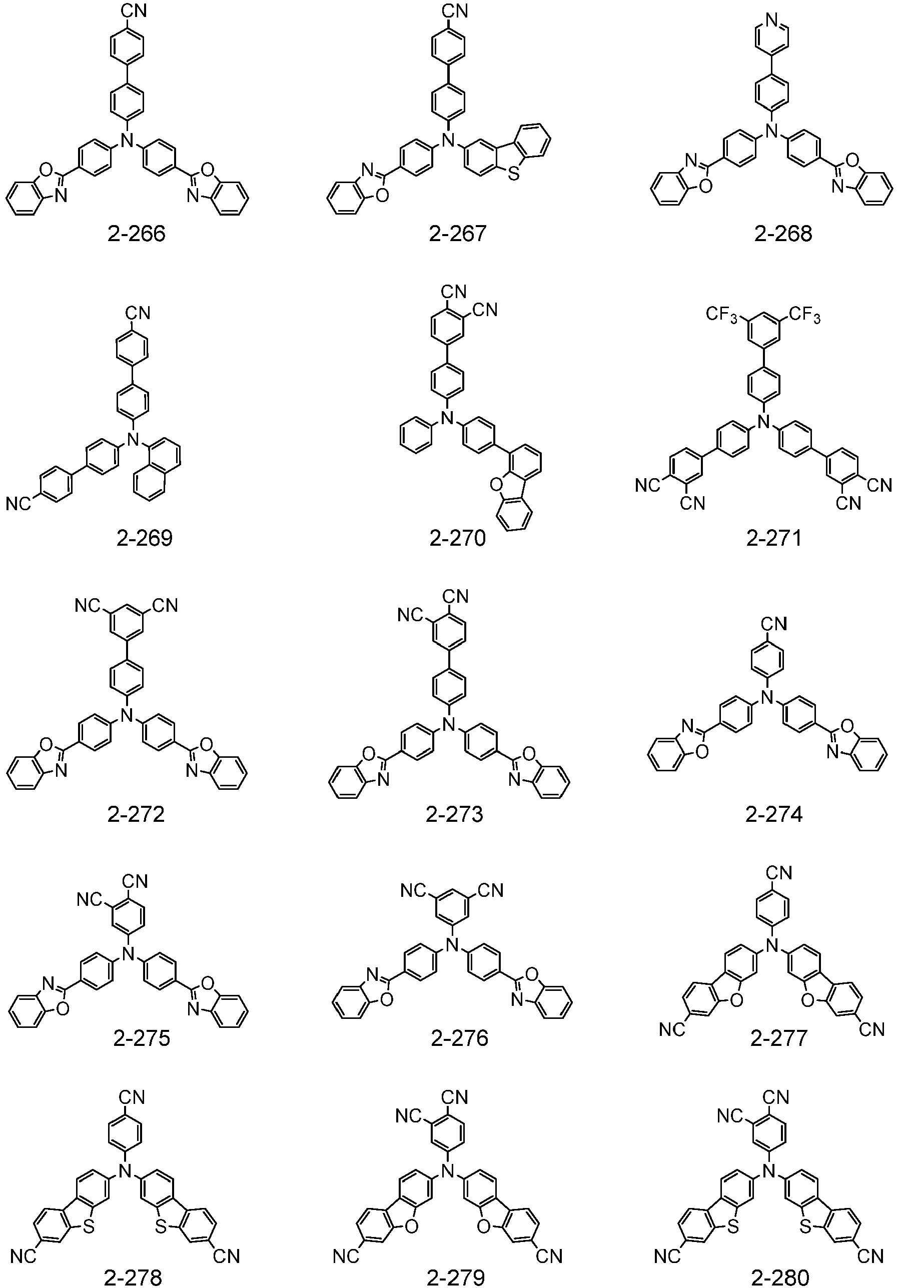

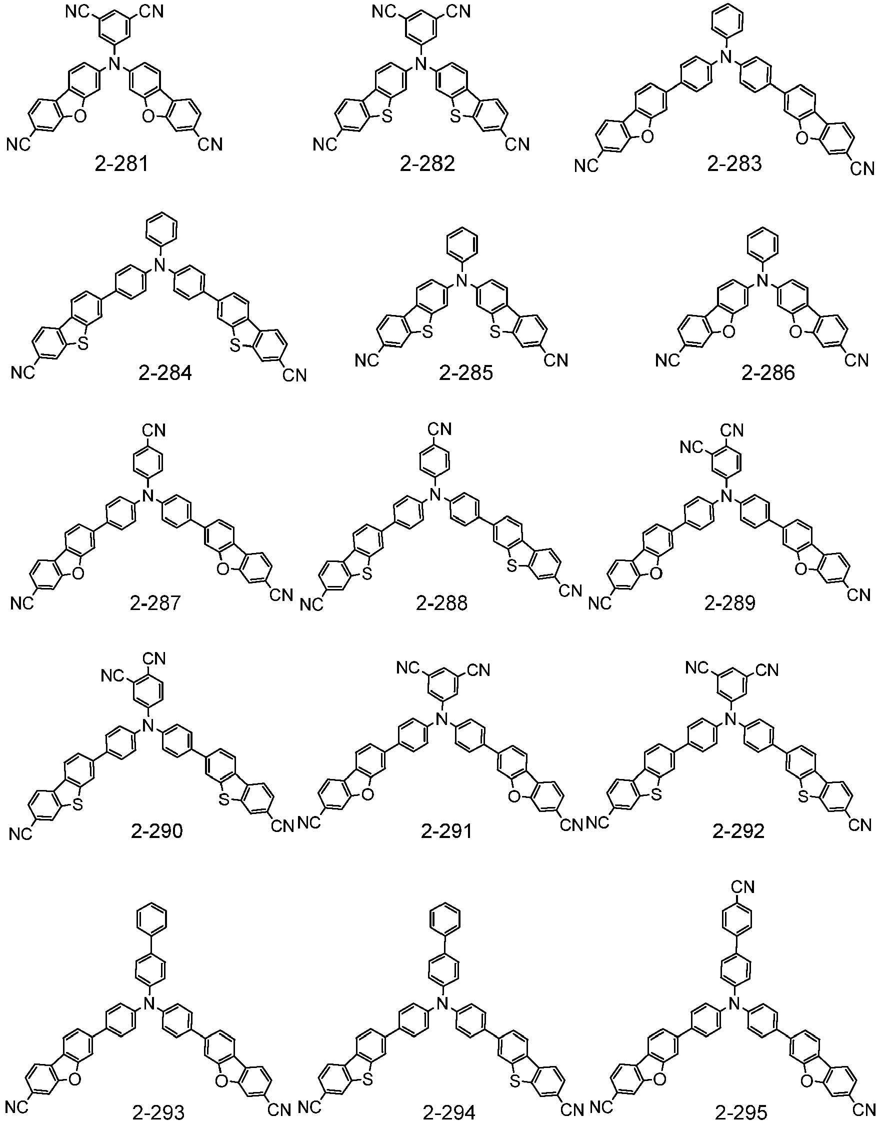

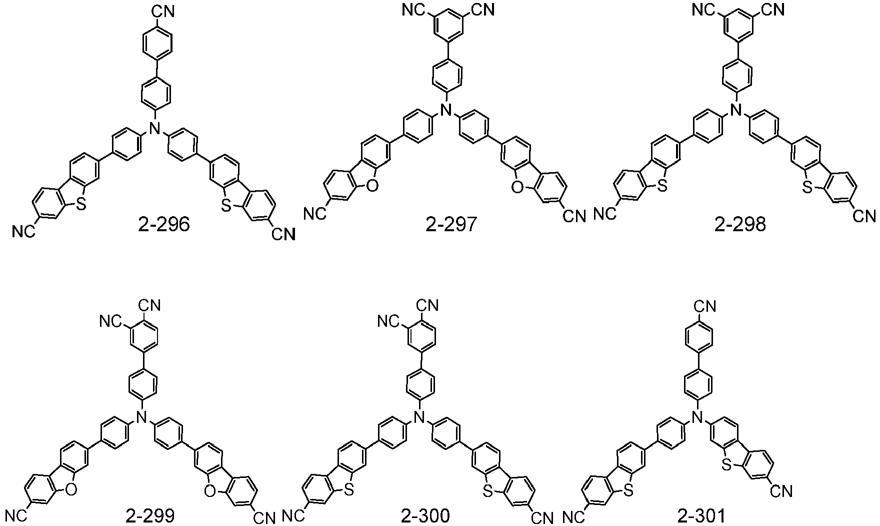

3. An organic electroluminescent device comprising a first electrode, an organic layer consisting of a plurality of layers arranged on the first electrode, a second electrode arranged on the organic layer, and a capping layer arranged on the second electrode wherein the capping layer comprises the tertiary amine derivative according to claim 1 or 2.
